# EUROPEAN PATENT SPECIFICATION

(11) **EP 4 492 406 B1**
(45) Date of publication and mention of the grant of the patent: **19.11.2025**
(21) Application number: 24184583.3
(22) Date of filing: 26.06.2024
(51) Int. Cl.: H01B 1/12, H01B 1/22, H01B 1/24, A61B 5/259, A61B 5/268, A61B 5/28, C08G 77/12, C08G 77/20, C08G 77/38, C08G 77/385, C08G 77/388, C08G 77/392, C08L 83/04, C08L 83/08

(54) **BIO-ELECTRODE COMPOSITION, BIO-ELECTRODE, METHOD FOR MANUFACTURING BIO-ELECTRODE**
BIOELEKTRODENZUSAMMENSETZUNG, BIOELEKTRODE, VERFAHREN ZUR HERSTELLUNG DER BIOELEKTRODE
COMPOSITION DE BIO-ÉLECTRODE, BIO-ÉLECTRODE, PROCÉDÉ DE FABRICATION DE BIO-ÉLECTRODE

(30) Priority: 10.07.2023 JP 2023113345
(43) Date of publication of application: 15.01.2025
(73) Proprietor: SHIN-ETSU CHEMICAL CO., LTD., Tokyo 1000005 (JP)
(72) Inventor: Hatakeyama, Jun, Niigata (JP); Ikeda, Joe, Tokyo, 100-0005 (JP); Nonaka, Shiori, Niigata (JP); Hasegawa, Koji, Tokyo, 100-0005 (JP)
(74) Representative: Sonnenhauser, Thomas Martin

(56) References cited:
- EP-A1- 3 587 473
- EP-A1- 3 881 766
- US-A1- 2022 110 569

## Description

### TECHNICAL FIELD

The present invention relates to a bio-electrode that is used in contact with the skin of a living body and capable of detecting physical conditions such as heart rate by an electric signal transmitted from the skin, a method for manufacturing the bio-electrode, and a bio-electrode composition desirably used for the bio-electrode.

### BACKGROUND ART

A recent growing popularity of Internet of Things (IoT) has accelerated the development of wearable devices, such as watches and eye-glasses that allow for Internet access. Even in the fields of medicine and sports, wearable devices for constantly monitoring the user's physical state are increasingly demanded, and such technological development is expected to be further encouraged.

In the field of medicine, use of wearable devices has been examined for monitoring the state of human organs by sensing extremely weak current, such as an electrocardiogram which detects an electric signal to measure the motion of the heart. The electrocardiogram measurement is conducted by attaching an electrode coated with an electro-conductive paste on a body, but this is a single (not continuous), short-time measurement. On the other hand, development of the above medical wearable device is aimed at device for continuously monitoring the health condition for a few weeks. Accordingly, a bio-electrode used as a medical wearable device is required to make no changes in electric conductivity even in long-time use and cause no skin allergy. In addition to these, it is also required that a bio-electrode is light-weight and can be produced at low cost.

Medical wearable devices are classified into two types: a type which is directly attached to body and a type which is incorporated into clothes. As the type of attaching to a body, it has been proposed a bio-electrode using water-soluble gel containing water and electrolyte, which are materials of the foregoing electro-conductive paste (Patent Document 1). The water-soluble gel contains sodium, potassium, or calcium as the electrolyte in a water-soluble polymer for retaining water, and converts changes of ion concentration from the skin into electricity. On the other hand, as the type of being incorporated into clothes, it has been proposed a means to use cloth in which an electro-conductive polymer such as PEDOT-PSS (poly-3,4-ethylenedioxythiophene-polystyrenesulfonate) or silver paste is incorporated into the fibers for electrodes (Patent Document 2).

However, the use of the above hydrophilic gel containing water and electrolytes brings about a problem of losing electric conductivity due to water loss by drying. Meanwhile, the use of a higher-ionization-tendency metal such as copper has a problem of a risk of causing some users to suffer from skin allergy. The use of an electro-conductive polymer such as PEDOT-PSS has a problem of a risk of causing skin allergy due to the strong acidity of the electro-conductive polymer, and a problem of peeling of the electro-conductive polymer from fibers during washing.

By taking advantage of excellent electric conductivity, the use of metal nanowire, carbon black, carbon nanotube, and the like has been examined as electrode materials (Patent Documents 3, 4, and 5). With higher contact probability among metal nanowires, the nanowires can conduct electricity even when added in small quantities. The metal nanowire, however, can cause skin allergies since it is thin material with a sharp tip. Even if these electrode materials themselves cause no allergic reaction, the biocompatibility may be degraded depending on the shape of a material and its irritant stimulation as in the manners described above, making it difficult to satisfy both electric conductivity and biocompatibility.

Although metal films seem to function as excellent bio-electrodes thanks to extremely high electric conductivity, this is not always the case. As the heart beats, the human skin releases not only extremely weak current, but also sodium ions, potassium ions, and calcium ions. It is thus necessary to convert changes in ion concentration into current. Noble metals, however, are difficult to ionize and are inefficient in converting ions from the skin to current. Therefore, the bio-electrode using a noble metal is characterized by high impedance and high resistance to the skin during electrical conduction.

Meanwhile, the use of a battery containing an ionic liquid has been examined (Patent Document 6). The ionic liquid is thermally and chemically stable, and has excellent electric conductivity, providing wider battery applications. However, an ionic liquid having smaller molecular weight as shown in Patent Document 6 dissolves into water. When a bio-electrode containing such an ionic liquid is used, the ionic liquid is extracted from the bio-electrode by sweating, which not only lowers the electric conductivity but also permeate the skin to cause rough skin.

Batteries using a lithium salt of polymer type sulfonimide have also been examined (Non-Patent Document 1). Lithium has been applied to batteries because of its high ionic mobility, but is not a bio-compatible. Additionally, lithium salts of fluorosulfonate have been also examined in a form of a pendant on silicone (Non-Patent Document 2).

Bio-electrode materials in which an ionic polymer is mixed with a silicone adhesive have been proposed (Patent Documents 7 and 8). These materials have not only high ionic conductivity but also high electronic conductivity when added with conductive powders such as carbon and silver, so they function as an excellent bio-electrode. By combining a silicone adhesive having low allergenic potency to the skin, high water repellency, and an effect of suppressing itching and reddening on the skin after removal, with an ionic polymer that has high ionic conductivity and does not permeate the skin, it is possible not to peel off even when attached for a long period including daily bathing and exercise and to obtain stable biological signals. Patent Document 9 discloses a bio-electrode composition comprising: (A) a polymer compound comprising a repeating unit-a having a structure selected from the group consisting of salts of ammonium, sodium, potassium, and silver formed with any of fluorosulfonic acid, fluorosulfonimide, and N-carbonyl-fluorosulfonamide; and (B) a silicone compound having a polyglycerin structure. However, it is desired to improve further in comfortability during longer-term attachment.

Here, health effects of perfluoroalkyl compounds (PFAS) have been pointed out, and there is a movement to restrict production and sale of PFAS compounds under REACH in Europa. It is an urgent need to develop materials that do not have a PFAS structure.

### CITATION LIST

### PATENT LITERATURE

Patent Document 1: WO 2013-039151 Pamphlet
Patent Document 2: JP 2015-100673 A
Patent Document 3: JP H5-095924 A
Patent Document 4: JP 2003-225217 A
Patent Document 5: JP 2015-019806 A
Patent Document 6: JP 2004-527902 A
Patent Document 7: JP 2018-126496 A
Patent Document 8: JP 2018-130533 A
Patent Document 9: US 2022/110569 A1

### NON PATENT LITERATURE

Non Patent Document 1: J. Mater. Chem. A, 2016, 4, p10038-10069
Non Patent Document 2: J. of the Electrochemical Society, 150(8) A1090-A1094 (2003)

### SUMMARY OF INVENTION

### TECHNICAL PROBLEM

The present invention has been made to solve the above problems. An object of the present invention is to provide: a bio-electrode composition capable of forming a living body contact layer for a bio-electrode which is excellent in electric conductivity and biocompatibility, light-weight, manufacturable at low cost, capable of preventing significant reduction in the electric conductivity even when the bio-electrode is wetted with water or dried, and soft and excellent in stretchability and adhesiveness; a bio-electrode including a living body contact layer formed from the bio-electrode composition; and a method for manufacturing the bio-electrode.

### SOLUTION TO PROBLEM

To solve the above problems, the present invention provides a bio-electrode composition comprising an (A) ionic resin, wherein
the component (A) contains a resin having a structure selected from the group consisting of an ammonium salt, a lithium salt, a sodium salt, a potassium salt, and a silver salt formed with trifluoromethoxybenzenesulfonamide and/or difluoromethoxybenzenesulfonamide.

With using the inventive bio-electrode composition, it is possible to form a living body contact layer for a bio-electrode which is excellent in electric conductivity and biocompatibility, light-weight, manufacturable at low cost, capable of preventing significant reduction in electric conductivity even when the bio-electrode is wetted with water or dried, and soft and excellent in stretchability and adhesiveness.

The resin having a structure selected from the group consisting of an ammonium salt, a lithium salt, a sodium salt, a potassium salt, and silver salt formed with trifluoromethoxybenzenesulfonamide and/or difluoromethoxybenzenesulfonamide preferably has a chemical structure represented by the following general formula (1), 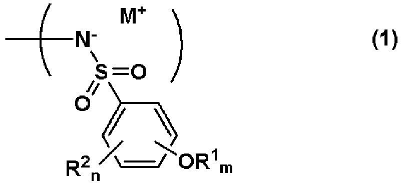 wherein R¹ represents a trifluoromethyl group or a difluoromethyl group; R² represents a hydrogen atom, a linear, branched, or cyclic alkyl group or alkoxy group having 1 to 6 carbon atoms, a hydroxy group, a carboxyl group, or a halogen atom; "m" represents an integer of 1 to 5; "n" represent an integer of 0 to 4; and M⁺ represents an ammonium ion, a lithium ion, a sodium ion, a potassium ion, or a silver ion.

In this event, the chemical structure represented by the general formula (1) preferably bonds to a resin selected from the group consisting of a resin made by polymerizing a monomer having a double bond, a silicone resin, and a polyurethane resin.

Further, in this event, the resin bonded to the chemical structure represented by the general formula (1) is preferably any one of: a resin having a repeating unit represented by the following general formula (2)-1 and being made by polymerizing a monomer having a double bond; a silicone resin selected from the group consisting of a silicone resin having a repeating unit represented by the following general formula (2)-2-1, a silicone resin represented by the following general formula (2)-2-2, a silicone resin represented by the following general formula (2)-2-3, and a silicone resin having a repeating unit represented by the following general formula (2)-2-4; a polyurethane resin having a repeating unit represented by the following general formula (2)-3-1; and a urethane resin having a terminal structure represented by the following general formula (2)-3-2: 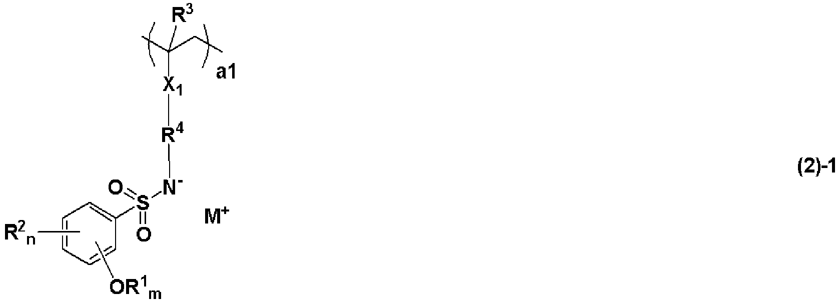 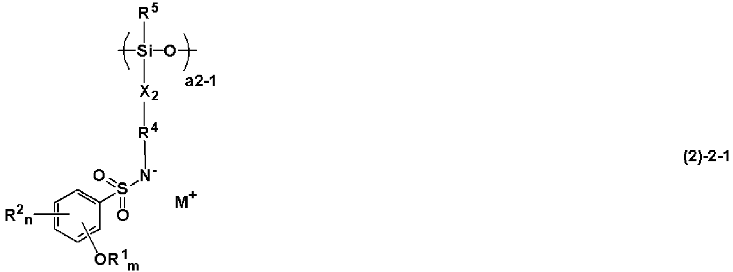 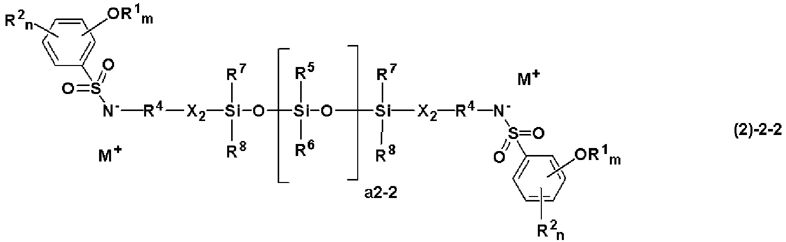 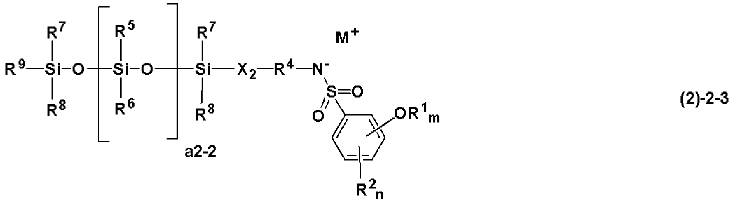 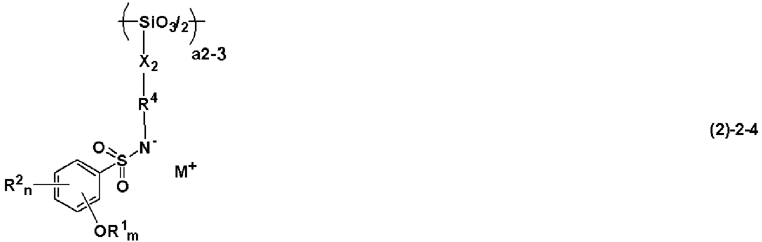 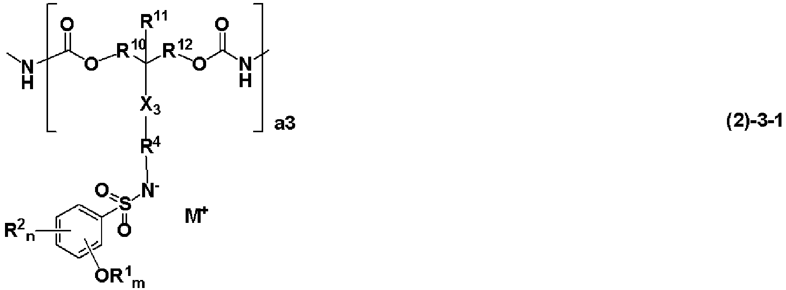 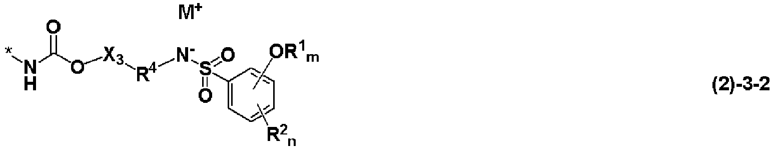 Wherein, R¹, R², "m", "n", and M⁺ are as described above; R³ is a hydrogen atom or a methyl group; R⁴ is a single bond, a linear, branched, or cyclic alkylene group having 1 to 15 carbon atoms, or a divalent hydrocarbon group, and may have an aromatic group, an ester group, an ether group, a carbonyl group, a carbon-carbon double bond, a sulfonyl group, or a sulfonic acid ester group; X₁ is a single bond, a phenylene group, an ester group, a carbonyl group, a sulfonyl group, or a sulfonic acid ester group; X₂ is a linear, branched, or cyclic alkylene group having 1 to 14 carbon atoms, an arylene group having 6 to 12 carbon atoms, or a bonded substance thereof, and may have an ether group, an ester group, a carbonyl group, a nitrogen atom, a sulfide group, a sulfonyl group, or a sulfonic acid ester group; R⁵ to R⁸ are a linear, branched, or cyclic alkyl group having 1 to 66 carbon atoms, or an aryl group having 6 to 10 carbon atoms; R⁹ is a linear or branched alkyl group having 1 to 20 carbon atoms, and may have an ether group or an ester group; R¹⁰ and R¹² are a single bond or a linear or branched alkylene group having 1 to 4 carbon atoms, the alkylene group may have an oxygen atom, and there is no case where both of R¹⁰ and R¹² are single bonds; R¹¹ is a hydrogen atom or a linear or branched alkyl group having 1 to 4 carbon atoms, and may have a hydroxyl group or an ether group; alternatively, R¹⁰ to R¹² and the carbon atom to which they are bonded in the formula may be combined to form a trivalent hydrocarbon group having 6 to 15 carbon atoms, and the trivalent hydrocarbon group may have an aromatic ring and/or a heteroatom; X₃ is a single bond, a carbonyl group, an ester group, or a linear, branched, or cyclic alkylene group having 1 to 10 carbon atoms, may have an aromatic compound, and may be bonded with R¹¹ to form a ring, which may be an aromatic ring; "a1" represents a ratio of repeating units, and satisfies 0<a1≤1.0; "a2-1" and "a2-3" represent a ratio of repeating units, and satisfies 0<a2-1≤1.0 and 0<a2-3≤1.0; "a2-2" represents the number of repeating units and is a number of 0 or more; and "a3" represents the number of repeating units and is a number greater than 0.

As the component (A) of the present invention, specifically, such ionic resins can be used.

The component (A) preferably comprises an ammonium ion represented by the following general formula (3) as the M⁺, 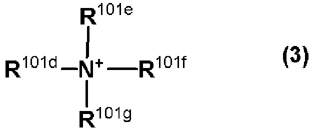 wherein, R^{101d}, R^{101e}, R^{1C1f}, and R^{101g} are each a hydrogen atom, a linear, branched, or cyclic alkyl group having 1 to 12 carbon atoms, a linear, branched, or cyclic alkenyl or alkynyl group having 2 to 12 carbon atoms, or an aromatic group having 4 to 20 carbon atoms, and may have one or more kinds selected from the group consisting of an ether group, a carbonyl group, an ester group, a hydroxy group, an amino group, a nitro group, a sulfonyl group, a sulfinyl group, a halogen atom, and a sulfur atom; R^{101d} and R^{101e}, or R^{101d}, R^{101e}, and R^{101f} may form a ring together with the nitrogen atom to which they are bonded, and when they form a ring, R^{101d} and R^{101e}, and R^{101d}, R^{101e}, and R^{101f} are alkylene groups having 3 to 10 carbon atoms, or form a heteroaromatic ring having the nitrogen atom described in the formula in the ring.

When the component (A) contains a resin having a structure of an ammonium salt, the ammonium ion may be as such.

Additionally, the inventive bio-electrode composition preferably contains further a resin as a component (B), which is different from the ionic resin as the component (A).

By containing such a component (B), it is possible to prevent elution of the salt of the component (A) and improve adhesiveness further.

Further, the component (B) is preferably one or more kinds selected from the group consisting of a silicone resin, a (meta)acrylate resin, and a urethane resin.

Furthermore, the resin as the component (B) has adhesiveness.

The bio-electrode composition may comprise as the component (B) further a silicone resin having RₓSiO_{(4-x)/2} unit and SiO₂ unit, wherein R is a substituted or unsubstituted monovalent hydrocarbon group having 1 to 10 carbon atoms and x is in a range of 2.5 to 3.5.

As the component (B), such resins can be used specifically.

The inventive bio-electrode composition preferably further comprises carbon powder and/or metal powder as a component (C).

By including such a component (C), the electric conductivity is further improved.

The carbon powder is preferably either or both of carbon black and carbon nanotube.

The metal powder is preferably selected from the group consisting of gold, silver, platinum, copper, tin, titanium, nickel, aluminum, tungsten, molybdenum, ruthenium, chromium, and indium.

In this event, the metal powder is preferably silver powder.

As the component (C), such an electric conductivity improver can be used specifically.

The bio-electrode composition preferably comprises an organic solvent further as a component (D).

By containing such a component (D), coatability can be improved further.

The present invention provides a bio-electrode comprising a conductive substrate and a living body contact layer formed on the conductive substrate, wherein the living body contact layer is a cured product of the bio-electrode composition.

The inventive bio-electrode has the living body contact layer formed using the bio-electrode composition that can form the living body contact layer for a bio-electrode that is excellent in electric conductivity and biocompatibility, light-weight, manufacturable at low cost, capable of preventing significant reduction in the electric conductivity even when the bio-electrode is wetted with water or dried, and soft and excellent in stretchability and adhesiveness.

In this event, the conductive substrate preferably comprises one or more kinds selected from the group consisting of gold, silver, silver chloride, platinum, aluminum, magnesium, tin, tungsten, iron, copper, nickel, stainless steel, chromium, titanium, carbon, and conductive polymers.

The inventive bio-electrode can use such a conductive substrate.

Further, the present invention provides a method for manufacturing a bio-electrode having a conductive substrate and a living body contact layer formed on the conductive substrate, comprising:
applying the bio-electrode composition onto the conductive substrate; and
curing the bio-electrode composition to form the living body contact layer.

The inventive bio-electrode can be manufactured in such a way.

In this event, it is preferable to use the conductive substrate that comprises one or more kinds selected from the group consisting of gold, silver, silver chloride, platinum, aluminum, magnesium, tin, tungsten, iron, copper, nickel, stainless steel, chromium, titanium, carbon, and conductive polymers.

In the inventive method for manufacturing the bio-electrode, such a conductive substrate can be used.

### ADVANTAGEOUS EFFECTS OF INVENTION

As described above, the inventive bio-electrode composition can provide a bio-electrode composition that can form a living body contact layer for a bio-electrode which is excellent in electric conductivity and biocompatibility, light-weight, manufacturable at low cost, capable of preventing significant reduction in the electric conductivity even when the bio-electrode is wetted with water or dried, and soft and excellent in stretchability and adhesiveness; a bio-electrode including a living body contact layer formed from the bio-electrode composition; and a method for manufacturing the bio-electrode.

### BRIEF DESCRIPTION OF DRAWINGS

FIG. 1 is a schematic sectional view showing an example of the inventive bio-electrode;
FIG. 2 is a schematic sectional view showing an example of the inventive bio-electrode attached on a living body;
FIG. 3 is a schematic view of printed bio-electrodes prepared in Examples of the present invention;
FIG. 4 is a schematic view of one of the bio-electrodes prepared in Examples of the present invention, the bio-electrode being cut out and provided with an adhesive layer and an electro-conductive wire thereon;
FIG. 5 is a view showing locations where electrodes and earth are attached on a human body in measuring biological signals in Examples of the present invention; and
FIG. 6 shows one of electrocardiogram waveforms obtained using the bio-electrodes in Examples of the present invention.

### DESCRIPTION OF EMBODIMENTS

As noted above, it has been desired to develop: a bio-electrode composition that can form a living body contact layer for a bio-electrode that is excellent in electric conductivity and biocompatibility, light-weight, manufacturable at low cost, soft, stretchable, adhesive, capable of obtaining electric signals from the skin stably even when attached to the skin for a long time and wetted with water in bathing etc. or when dried, and leaving no residue on the skin after removing; a bio-electrode including a living body contact layer formed from the bio-electrode composition; and a method for manufacturing the bio-electrode.

The surface of the skin releases ions of sodium, potassium, and calcium in accordance with heartbeat. A bio-electrode has to convert the increase and decrease of these ions released from the skin to electric signals. Accordingly, the bio-electrode requires a material that is excellent in ionic conductivity to transmit the increase and decrease of ions.

For attaching the bio-electrode on the skin to obtain biological signals stably, softness, stretchability, and adhesiveness as a bio-electrode film are necessary. The corneocyte of the outermost layer of the skin is renewed daily, and old corneocyte (dirt) accumulates between the attached bio-electrode film and the skin. Old corneocyte easily peels from the outermost layer of the skin, so that the bio-electrode becomes detached, making it impossible to obtain biological signals. Therefore, it is necessary for the adhesiveness of the bio-electrode not to decrease even when the bio-electrode is attached for a long time. Meanwhile, if a residue is left on the skin when the bio-electrode is detached after keeping attached for a long time, the residue may cause a rash or rough skin.

In neutralized salts formed from highly acidic acids, ions are strongly polarized to improve the ionic conductivity. This is why lithium salts of bis(trifluoromethanesulfonyl)imidic acid and tris(trifluoromethanesulfonyl)methide acid show high ionic conductivity as a lithium ion battery. On the other hand, the higher acidity of the acid before the neutral salt formation results in a problem that the salt has stronger irritation to a body. That is, the relation between ionic conductivity and irritation to the body is a trade-off relation. However, a salt applied to a bio-electrode has to achieve both higher ionic conductivity and lower irritation to a body.

An ionic compound having higher molecular weight has such nature that the permeability into the skin becomes lower to make less irritation to the skin. Accordingly, the ionic compound is preferably a polymer type with higher molecular weight. Thus, the present inventors have solved the problem of the irritation to the skin by making the ionic compound have a polymerizable double bond to polymerize them, or by making the ionic compound bonded with silicone, polyurethane, polyether, polyester, etc.

Further, the inventers found that by mixing such a salt with a silicone-based, acrylic-based, or urethane-based adhesive (resin) for example, it is possible to keep close adhesion on the skin all of the time to obtain electric signals stably for a long time.

That is, the present invention is a bio-electrode composition comprising an (A) ionic resin, wherein the component (A) contains a resin having a structure selected from the group consisting of an ammonium salt, a lithium salt, a sodium salt, a potassium salt, and a silver salt formed with trifluoromethoxybenzenesulfonamide and/or difluoromethoxybenzenesulfonamide.

Hereinafter, the present invention will be described in detail, but the present invention is not limited thereto.

### <Bio-Electrode Composition>

The inventive bio-electrode composition is a bio-electrode composition comprising an (A) ionic resin, wherein the component (A) contains a resin having a structure selected from the group consisting of an ammonium salt, a lithium salt, a sodium salt, a potassium salt, and a silver salt formed with trifluoromethoxybenzenesulfonamide and/or difluoromethoxybenzenesulfonamide. Hereinafter, each component will be described in further detail.

### [(A) Ionic Resin]

The (A) ionic resin contains a resin having a structure selected from the group consisting of an ammonium salt, a lithium salt, a sodium salt, a potassium salt, and a silver salt formed with trifluoromethoxybenzenesulfonamide and/or difluoromethoxybenzenesulfonamide.

The resin (ionic resin) having a structure selected from the group consisting of an ammonium salt, a lithium salt, a sodium salt, a potassium salt, and a silver salt formed with trifluoromethoxybenzenesulfonamide and/or difluoromethoxybenzenesulfonamide preferably has a chemical structure (partial structure) represented by the following general formula (1). 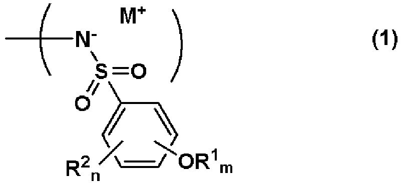 In the above formula, R¹ represents a trifluoromethyl group or a difluoromethyl group; R² represents a hydrogen atom, a linear, branched, or cyclic alkyl group or alkoxy group having 1 to 6 carbon atoms, a hydroxy group, a carboxyl group, or a halogen atom; "m" represents an integer of 1 to 5; "n" represent an integer of 0 to 4; and M⁺ represents an ammonium ion, a lithium ion, a sodium ion, a potassium ion, or a silver ion.

In this event, the chemical structure represented by the general formula (1) preferably bonds to a resin selected from the group consisting of a resin made by polymerizing a monomer having a double bond, a silicone resin, and a polyurethane resin.

The resin bonded to the chemical structure represented by the general formula (1) is any one of: a resin having a repeating unit represented by the following general formula (2)-1 and being made by polymerizing a monomer having a double bond; a silicone resin selected from the group consisting of a silicone resin having a repeating unit represented by the following general formula (2)-2-1, a silicone resin represented by the following general formula (2)-2-2, a silicone resin represented by the following general formula (2)-2-3, and a silicone resin having a repeating unit represented by the following general formula (2)-2-4; a polyurethane resin having a repeating unit represented by the following general formula (2)-3-1; and a urethane resin having a terminal structure represented by the following general formula (2)-3-2. 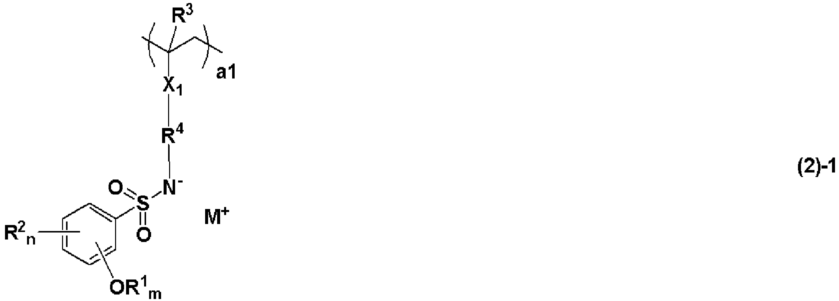 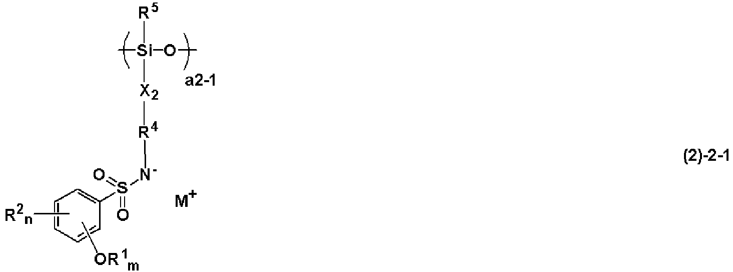 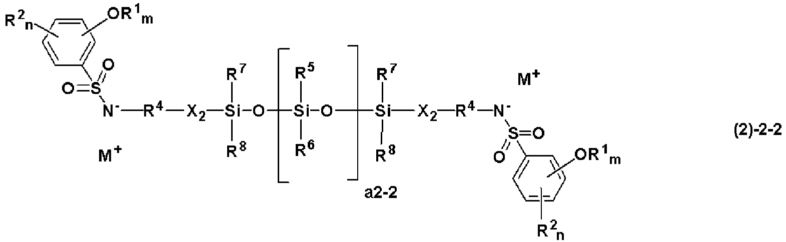 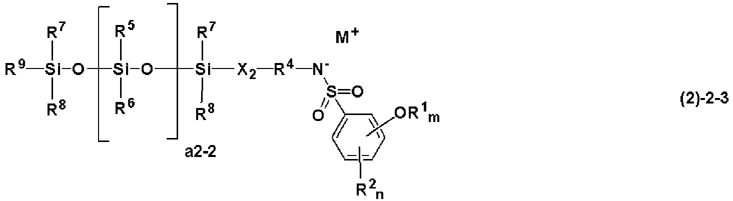 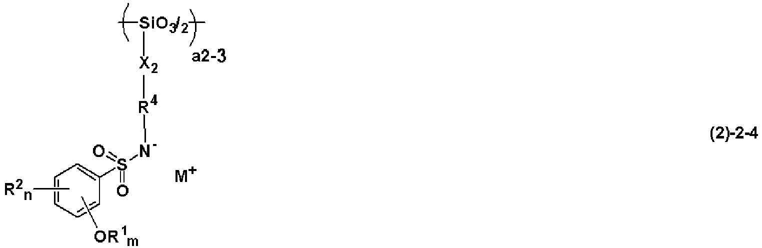 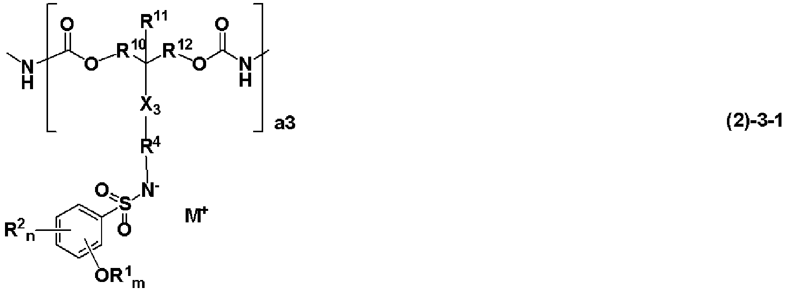 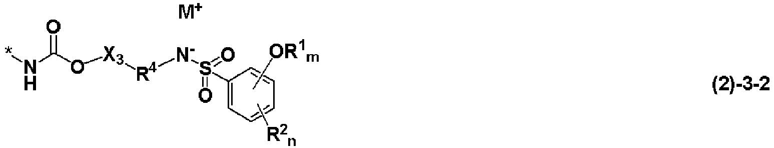 In the above formulae, R¹, R², "mm", "n", and M⁺ are as described above; R³ is a hydrogen atom or a methyl group; R⁴ is a single bond, a linear, branched, or cyclic alkylene group having 1 to 15 carbon atoms, or a divalent hydrocarbon group, and may have an aromatic group, an ester group, an ether group, a carbonyl group, a carbon-carbon double bond, a sulfonyl group, or a sulfonic acid ester group; X_ is a single bond, a phenylene group, an ester group, a carbonyl group, a sulfonyl group, or a sulfonic acid ester group; X₂ is a linear, branched, or cyclic alkylene group having 1 to 14 carbon atoms, an arylene group having 6 to 12 carbon atoms, or a bonded substance thereof, and may have an ether group, an ester group, a carbonyl group, a nitrogen atom, a sulfide group, a sulfonyl group, or a sulfonic acid ester group; R³ to R⁸ are a linear, branched, or cyclic alkyl group having 1 to 66 carbon atoms, or an aryl group having 6 to 10 carbon atoms; R⁹ is a linear or branched alkyl group having 1 to 20 carbon atoms, and may have an ether group or an ester group; R¹⁰ and R¹² are a single bond or a linear or branched alkylene group having 1 to 4 carbon atoms, the alkylene group may have an oxygen atom, and there is no case where both of R¹⁰ and R¹² are single bonds; R¹¹ is a hydrogen atom or a linear or branched alkyl group having 1 to 4 carbon atoms, and may have a hydroxyl group or an ether group; alternatively, R¹⁰ to R¹² and the carbon atom to which they are bonded in the formula may be combined to form a trivalent hydrocarbon group having 6 to 15 carbon atoms, and the trivalent hydrocarbon group may have an aromatic ring and/or a heteroatom; X₃ is a single bond, a carbonyl group, an ester group, or a linear, branched, or cyclic alkylene group having 1 to 10 carbon atoms, may have an aromatic compound, and may be bonded with R¹¹ to form a ring, which may be an aromatic ring; "a1" represents a ratio of repeating units, and satisfies 0<a1≤1.0; "a2-1" and "a2-3" represent a ratio of repeating units, and satisfies 0<a2-1≤1.0 and 0<a2-3≤1.0; "a2-2" represents the number of repeating units and is a number of 0 or more; and "a3" represents the number of repeating units and is a number greater than 0.

### [Resin made by Polymerizing Monomer having Double Bond]

The monomer to obtain the repeating unit-a1 represented by the above general formula (2)-1 is represented by the following general formula (2)-1-1. 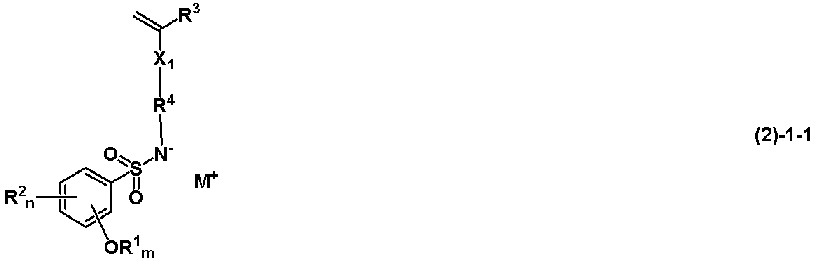 In the general formula, R¹ to R⁴, X₁, M⁺, "m", and "n" are as defined above.

Specific examples of the monomer represented by the above general formula (2)-1-1 can include the following. 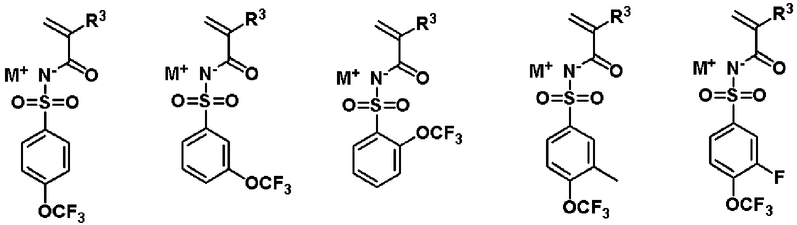 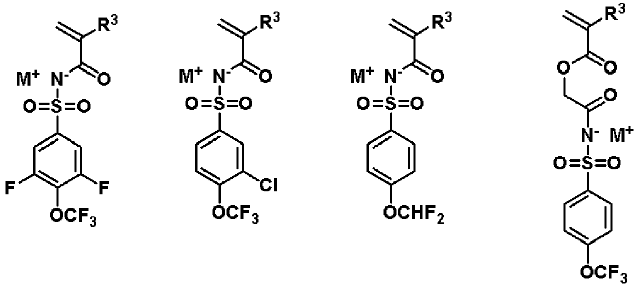 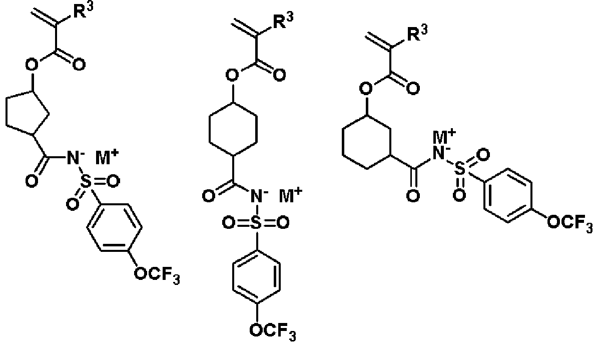 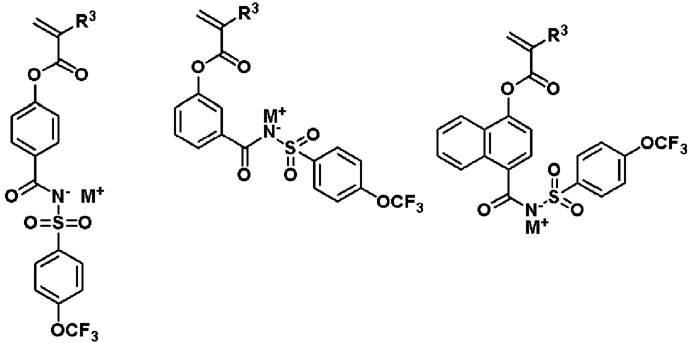 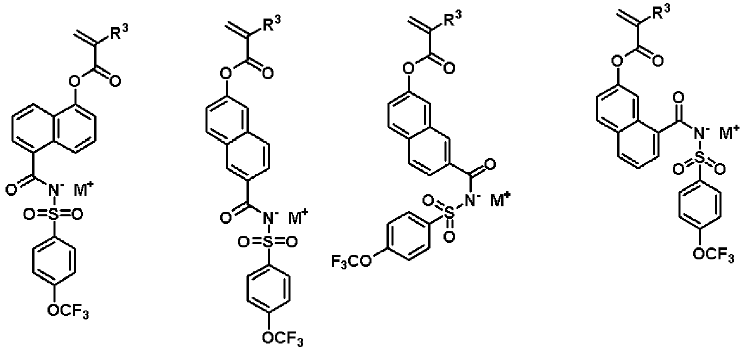 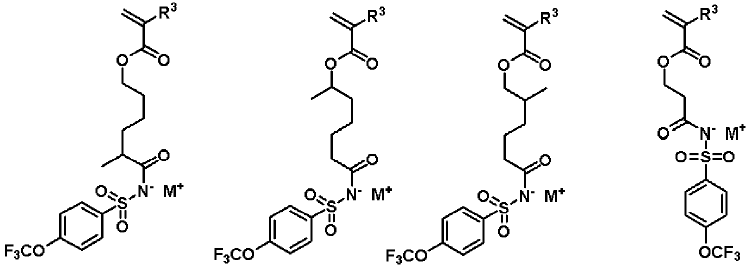 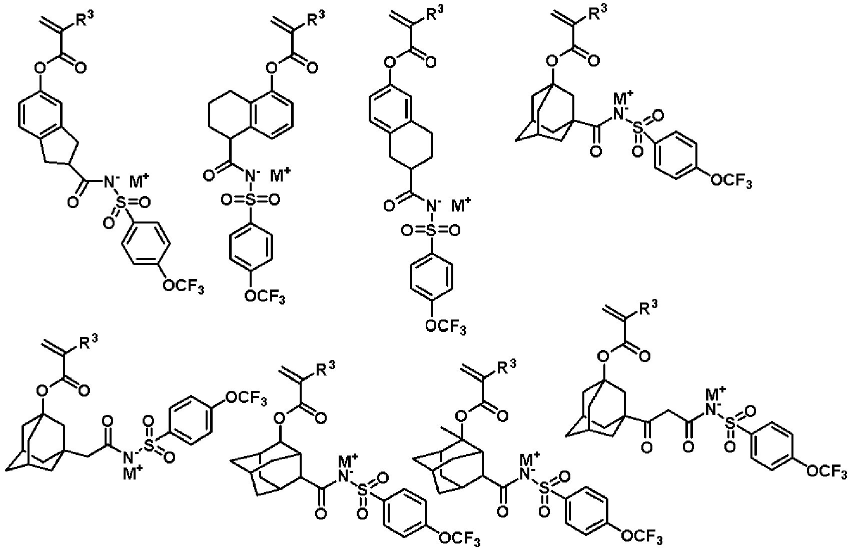 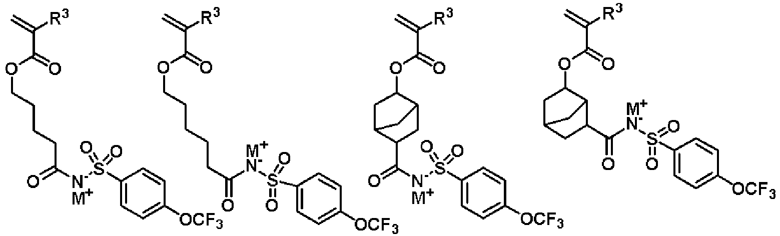 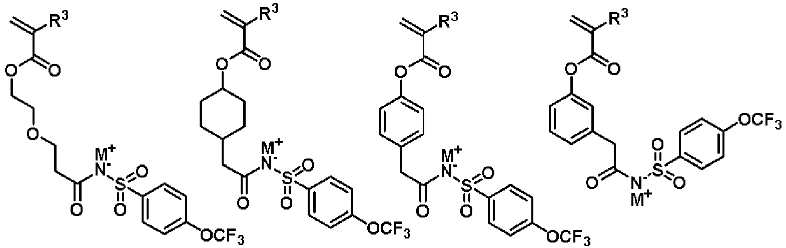 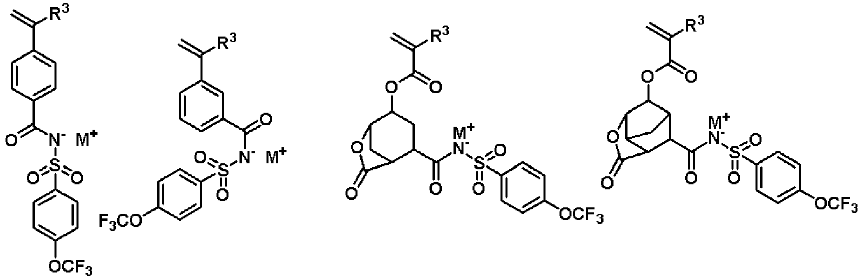 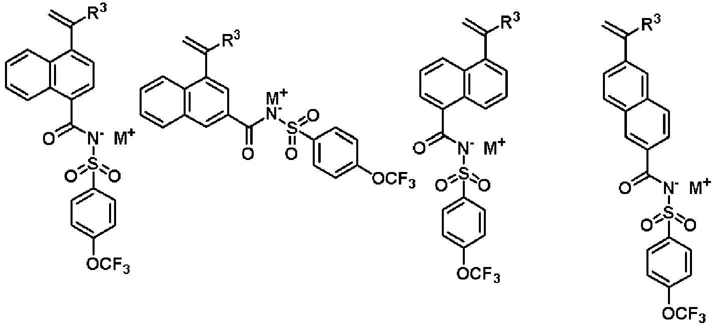 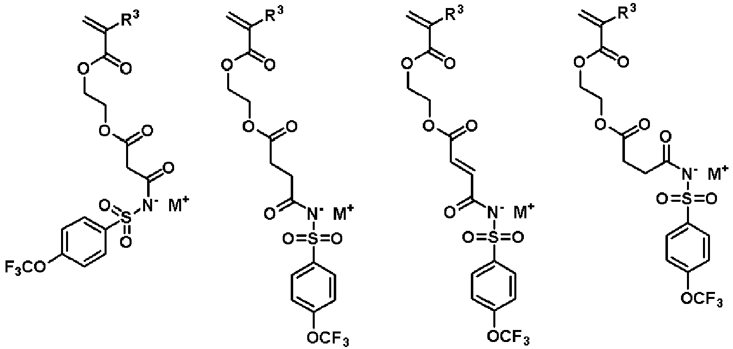 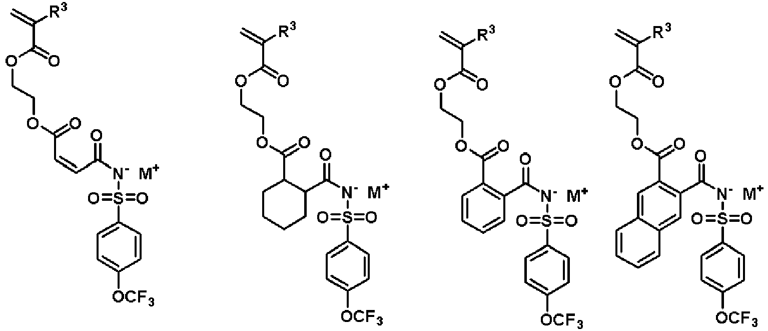 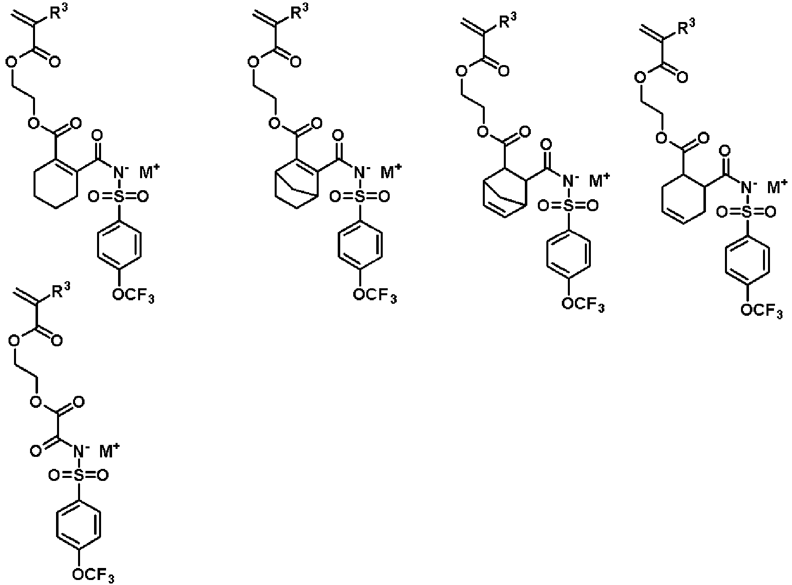 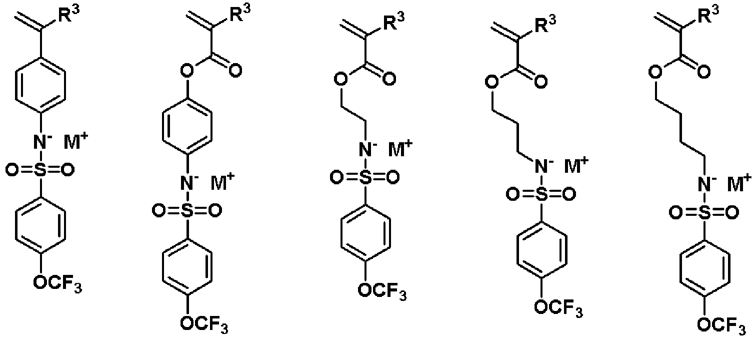 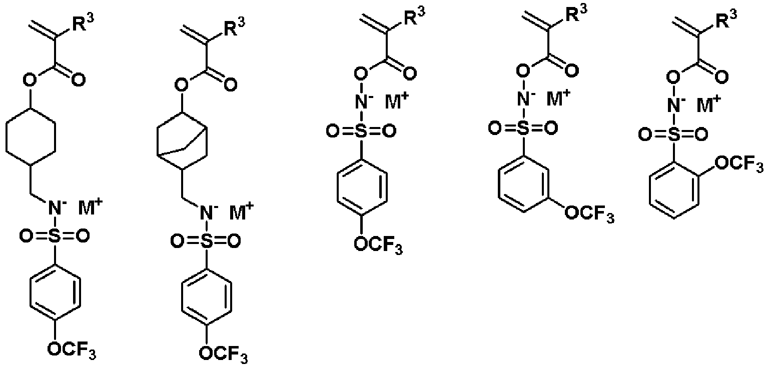 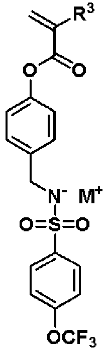 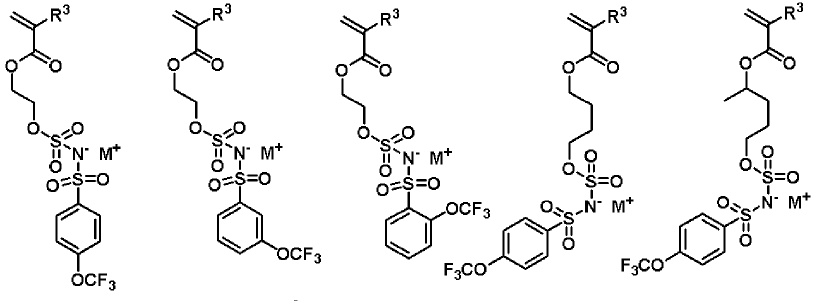 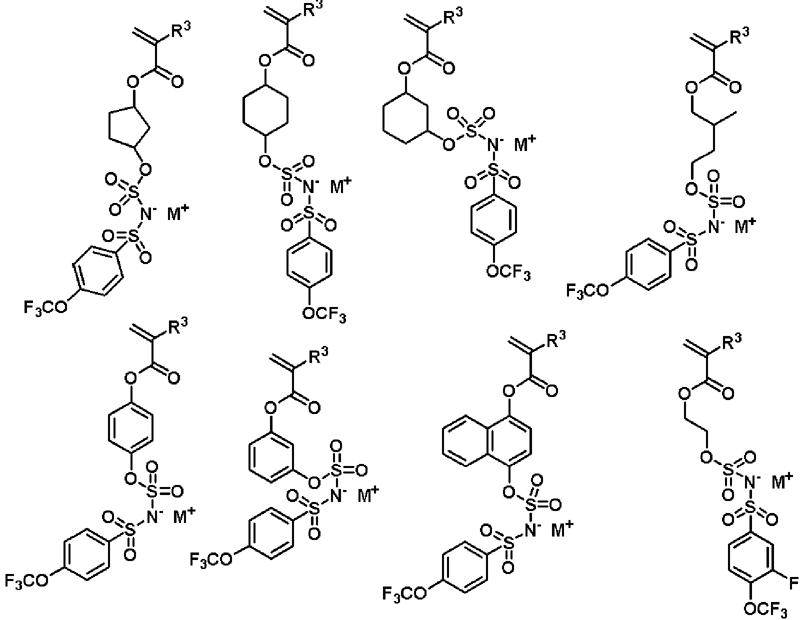 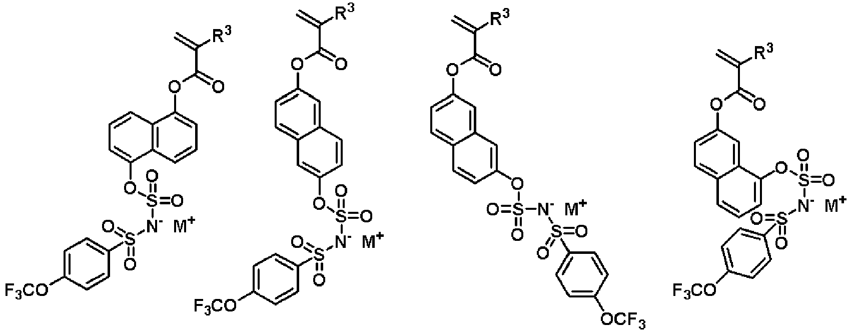 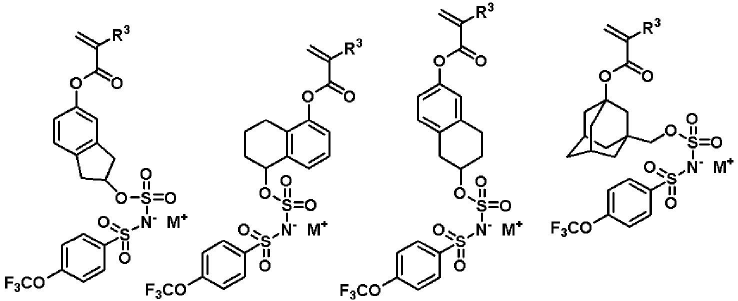 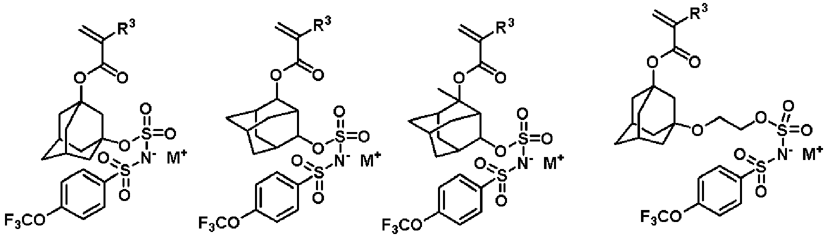 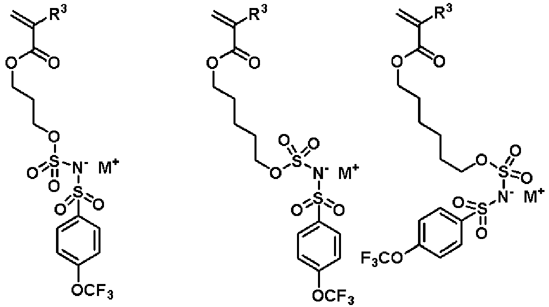 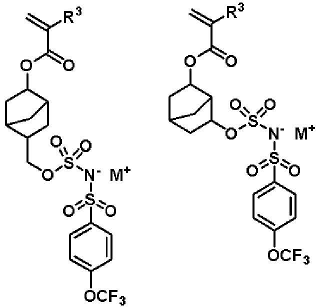 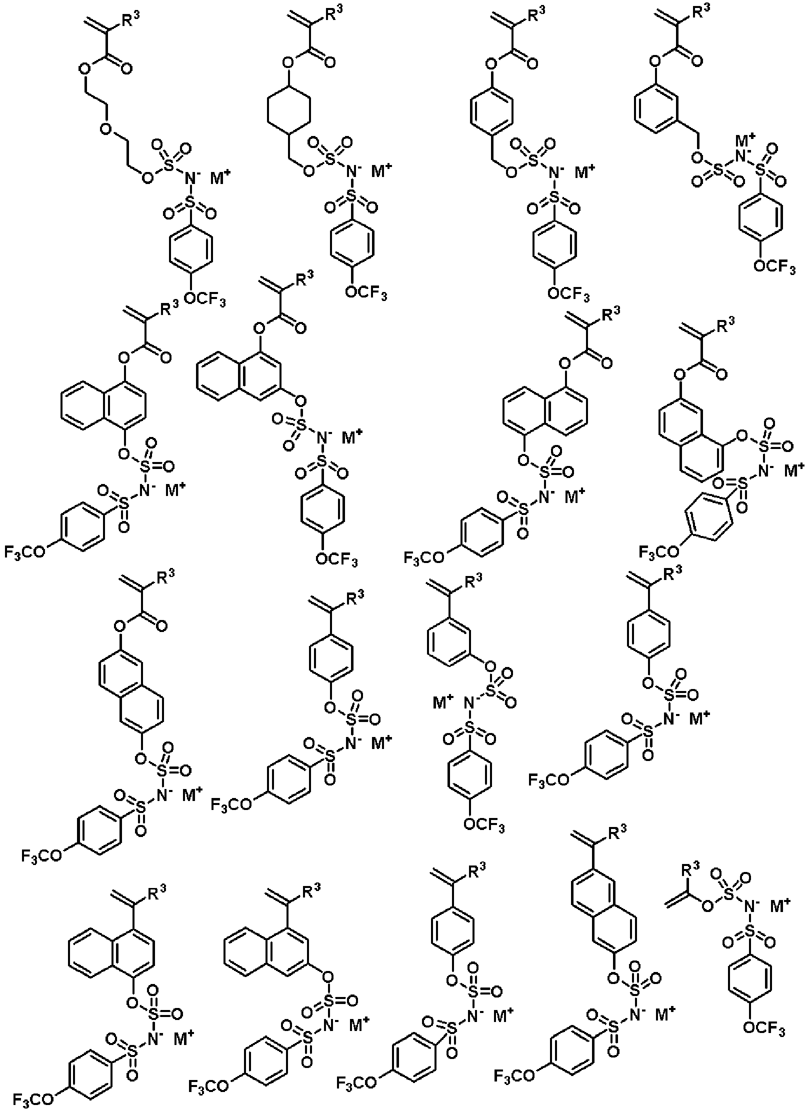 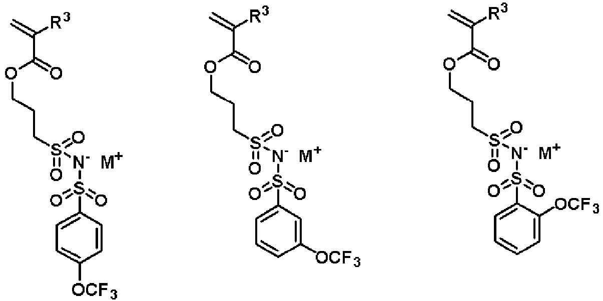 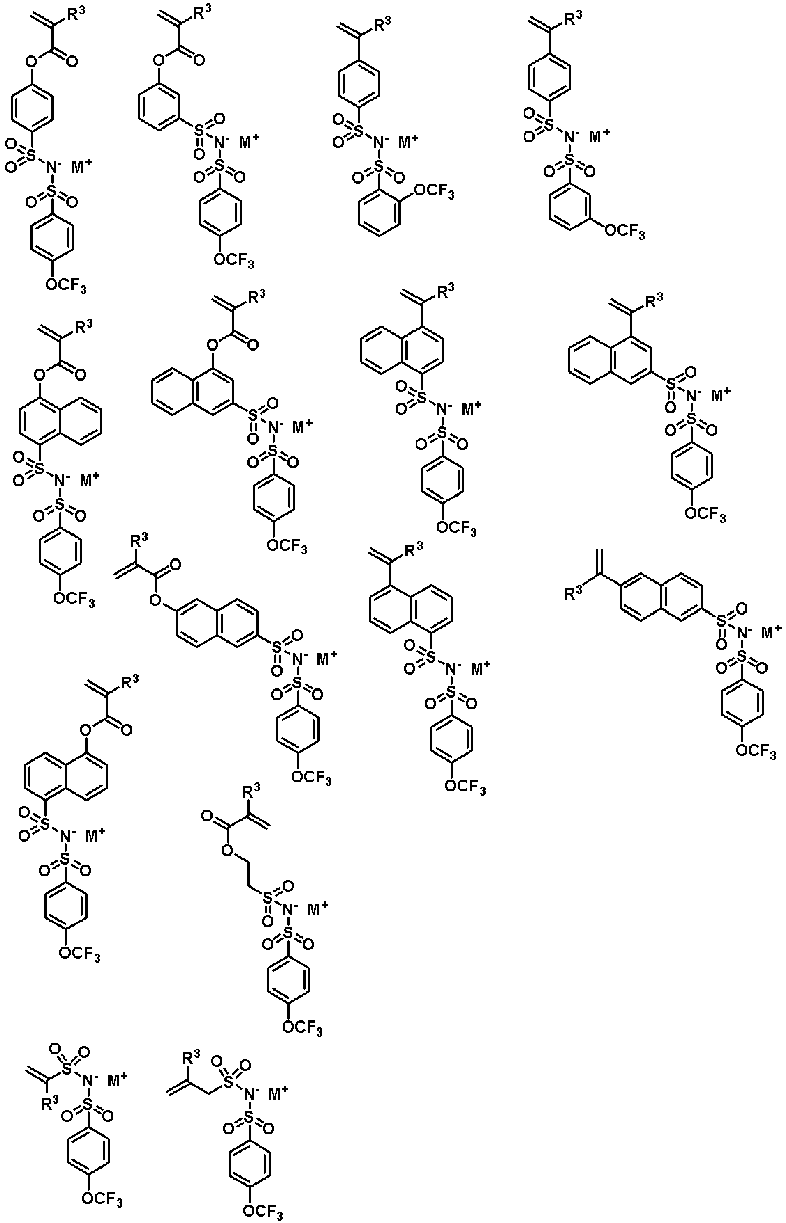 In the formulae, R³ and M are as defined above.

Furthermore, the component (A) preferably contains an ammonium ion (ammonium cation) represented by the following general formula (3) as M⁺ in the repeating unit-a (repeating unit-a1).

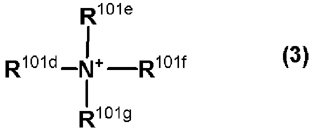

In the general formula (3), R^{101d}, R^{101e}, R^{101f}, and R^{101g} are each a hydrogen atom, a linear, branched, or cyclic alkyl group having 1 to 12 carbon atoms, a linear, branched, or cyclic alkenyl or alkynyl group having 2 to 12 carbon atoms, or an aromatic group having 4 to 20 carbon atoms, and may have one or more kinds selected from the group consisting of an ether group, a carbonyl group, an ester group, a hydroxy group, an amino group, a nitro group, a sulfonyl group, a sulfinyl group, a halogen atom, and a sulfur atom; R^{101d} and R^{101e}, or R^{10ld}, R^{101e}, and R^{101f} may form a ring together with the nitrogen atom to which they are bonded, and when they form a ring, R^{101d} and R^{101e}, and R^{101d}, R^{101e}, and R^{101f} are alkylene groups having 3 to 10 carbon atoms, or form a heteroaromatic ring having the nitrogen atom described in the formula in the ring.

Specific examples of the ammonium ion shown by the general formula (3) include the following.

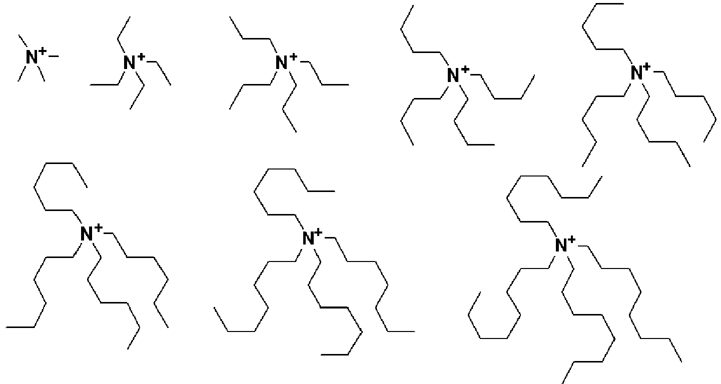

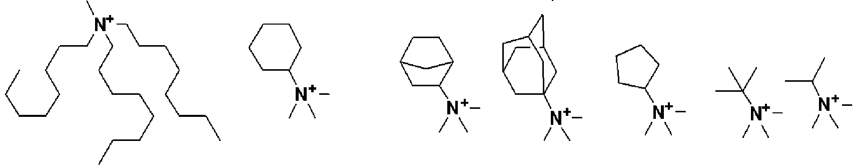

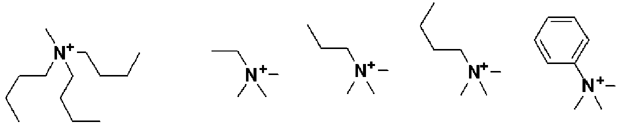

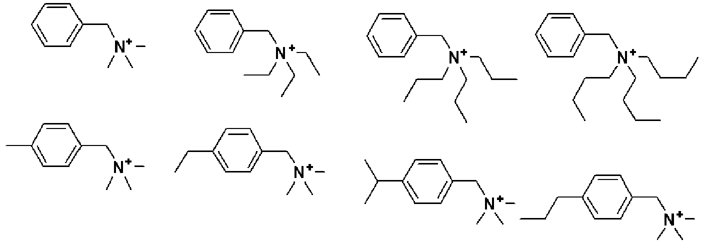

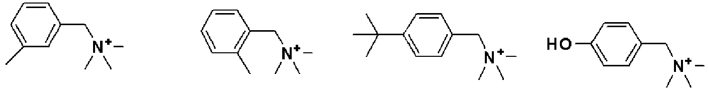

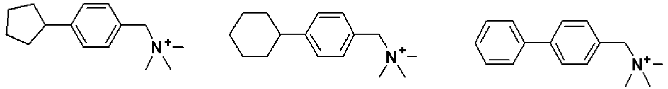

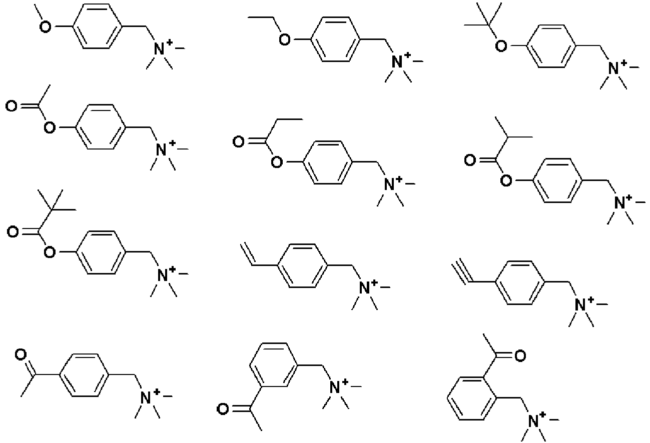

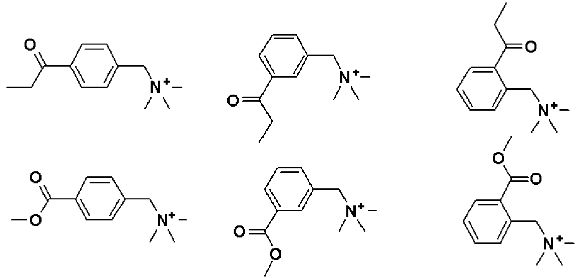

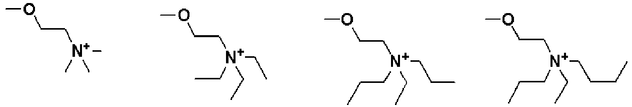

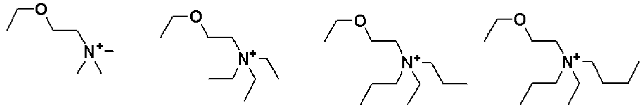

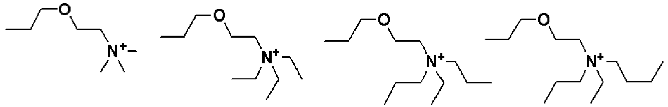

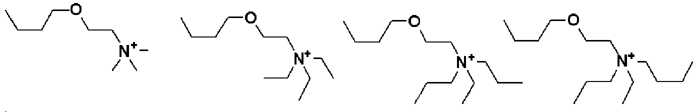

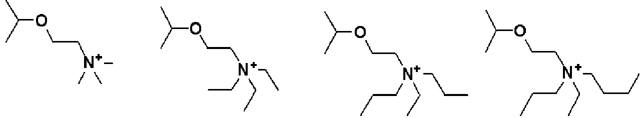

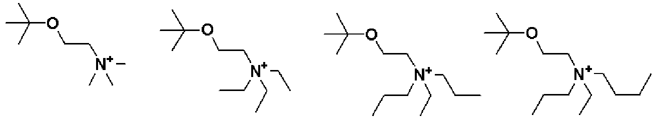

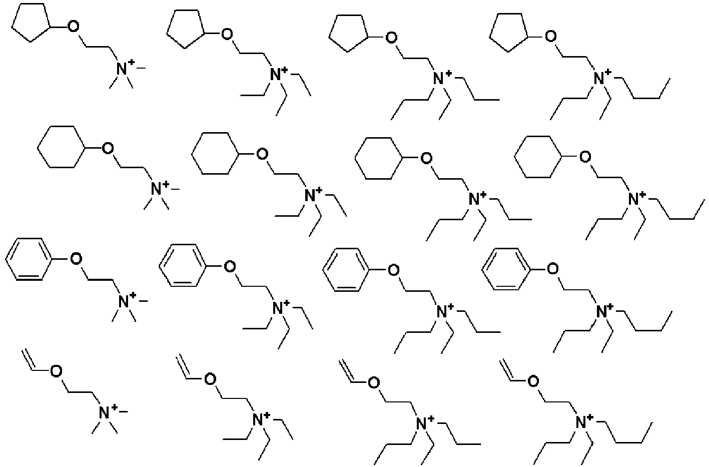

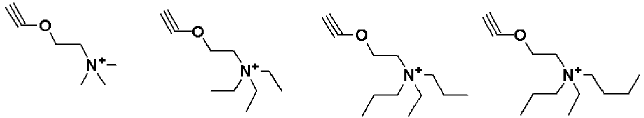

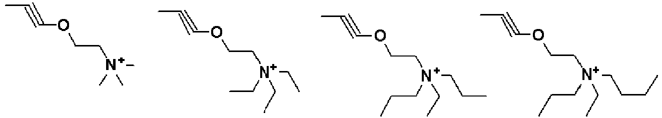

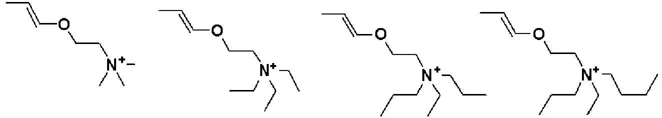

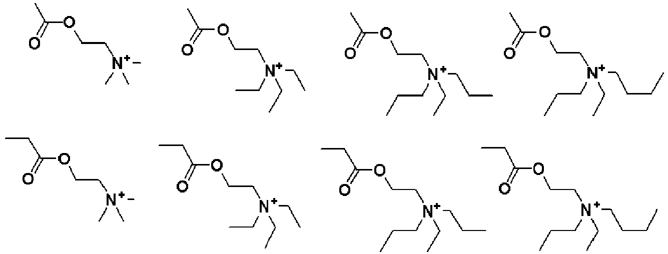

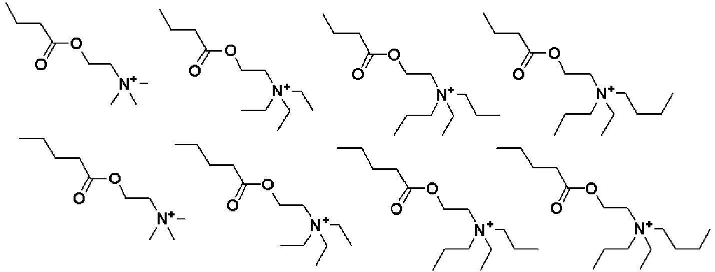

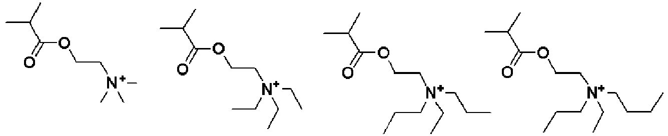

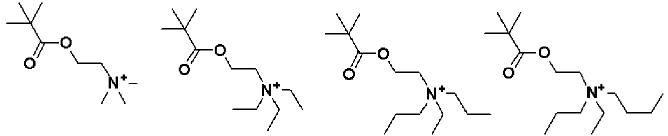

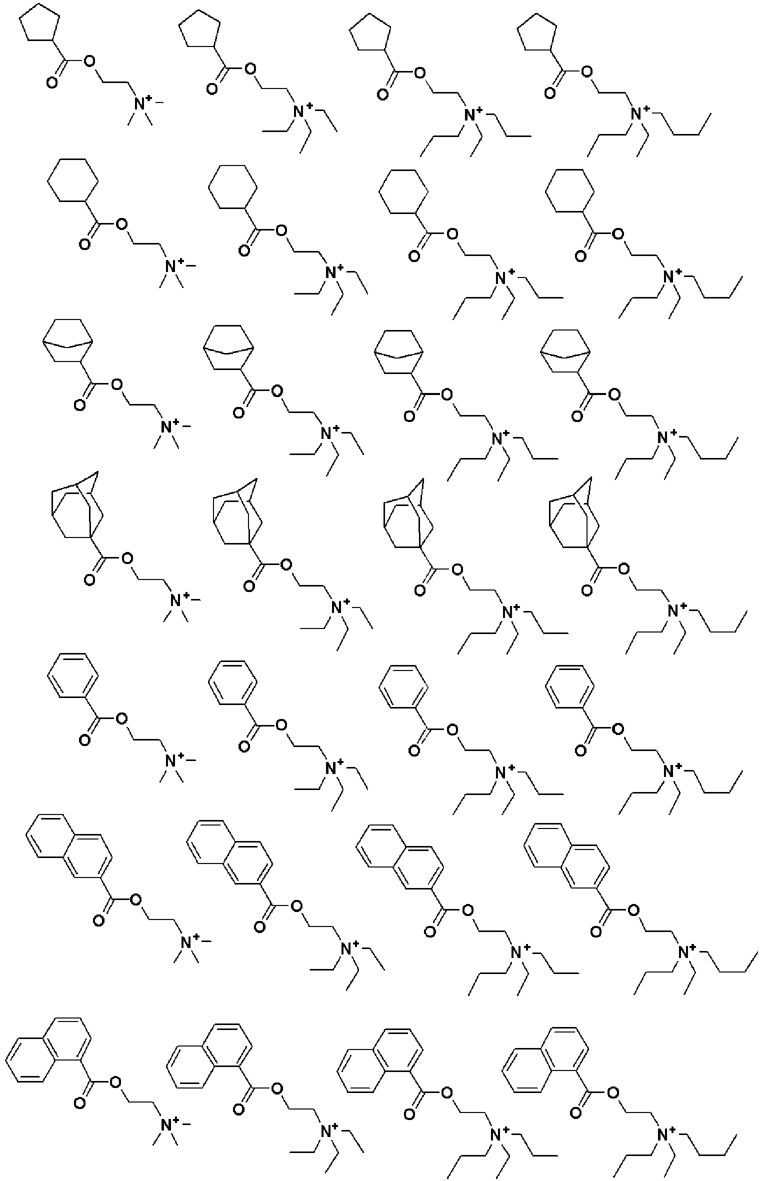

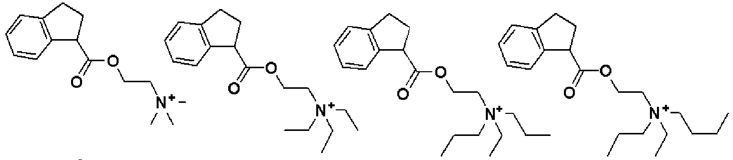

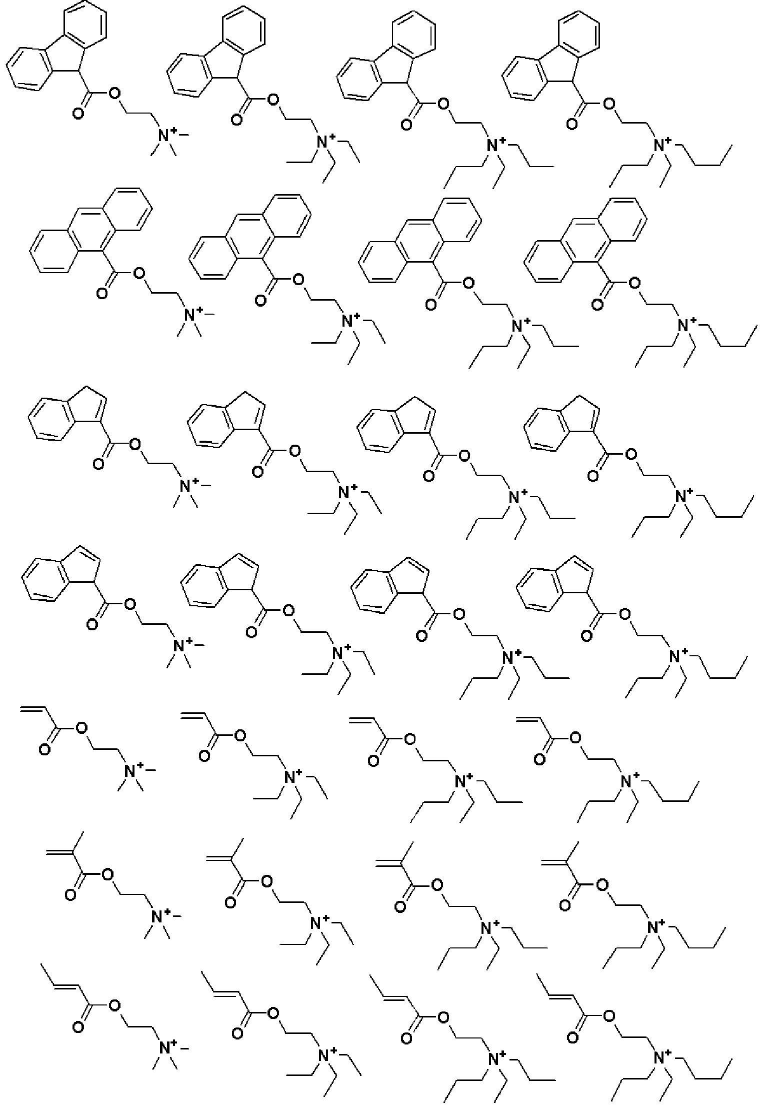

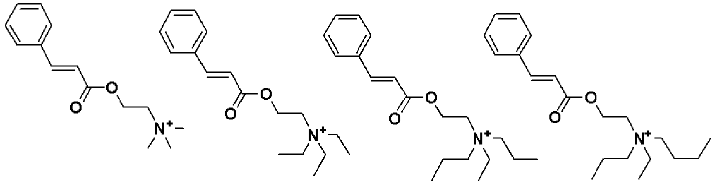

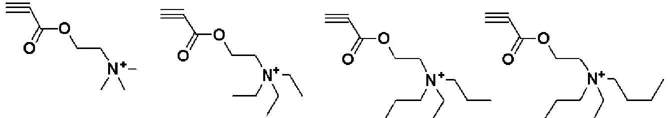

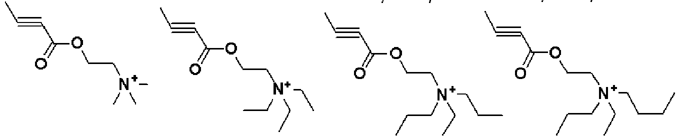

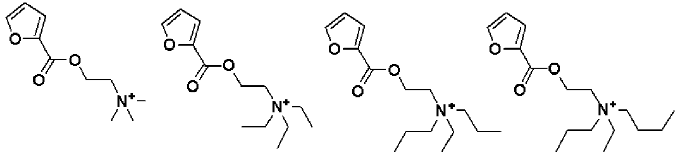

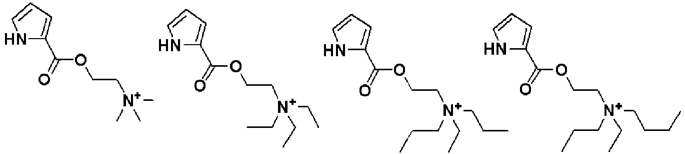

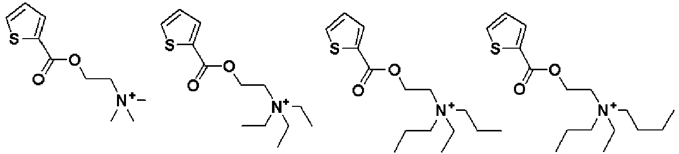

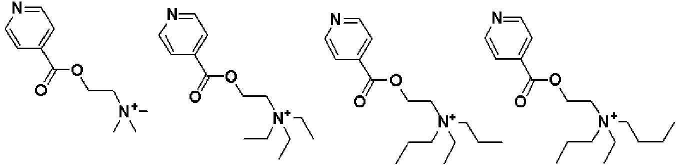

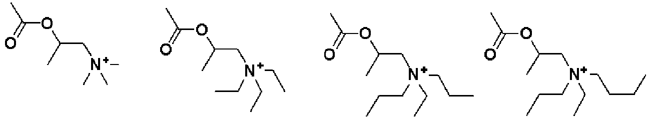

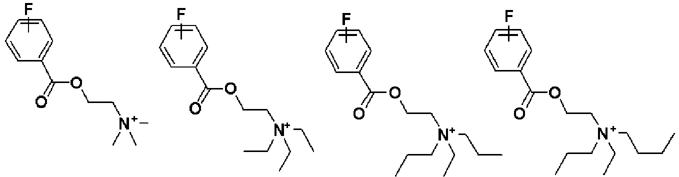

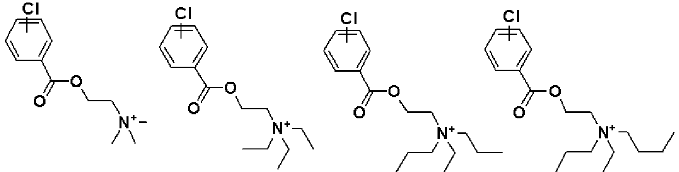

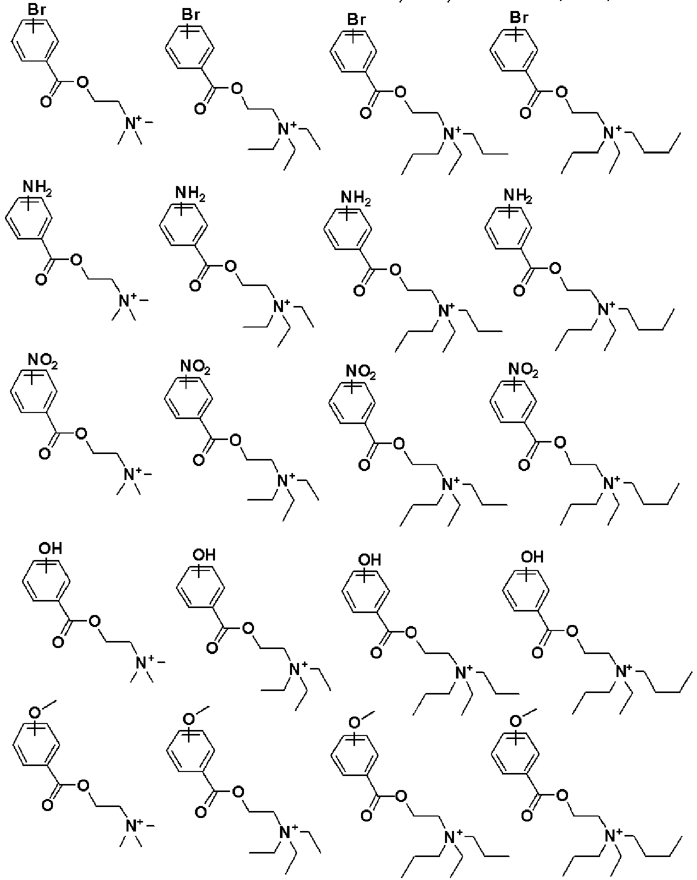

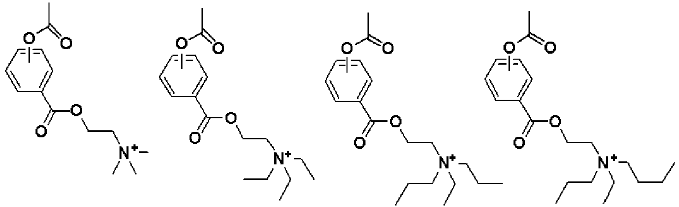

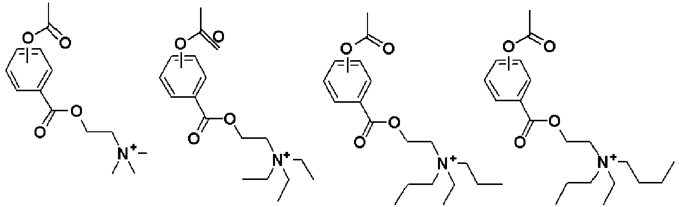

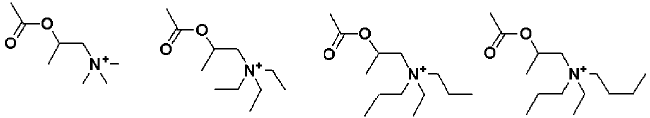

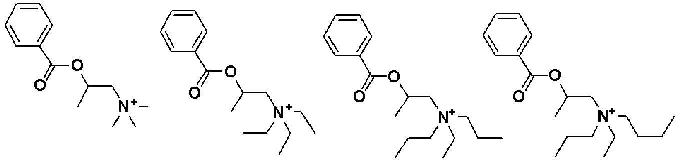

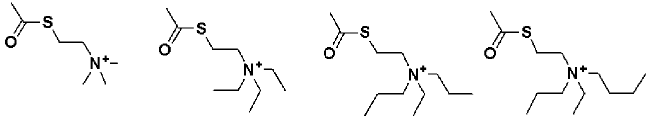

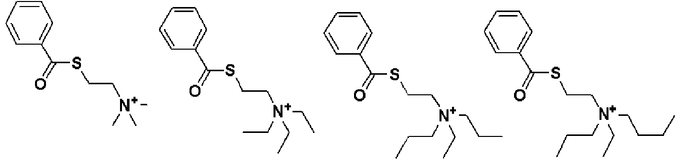

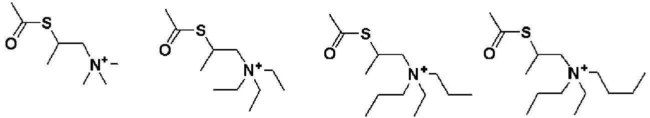

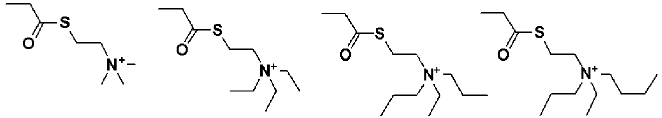

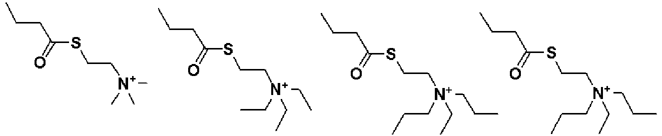

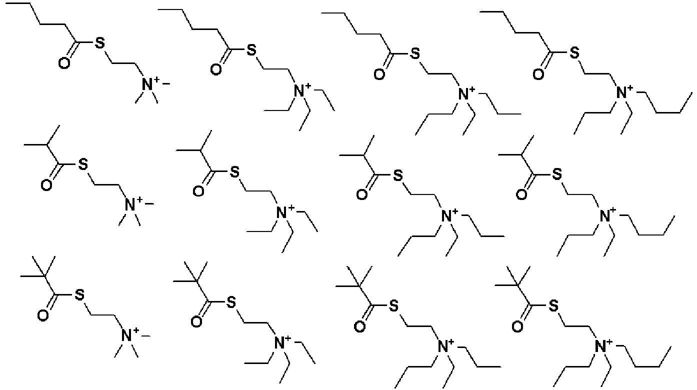

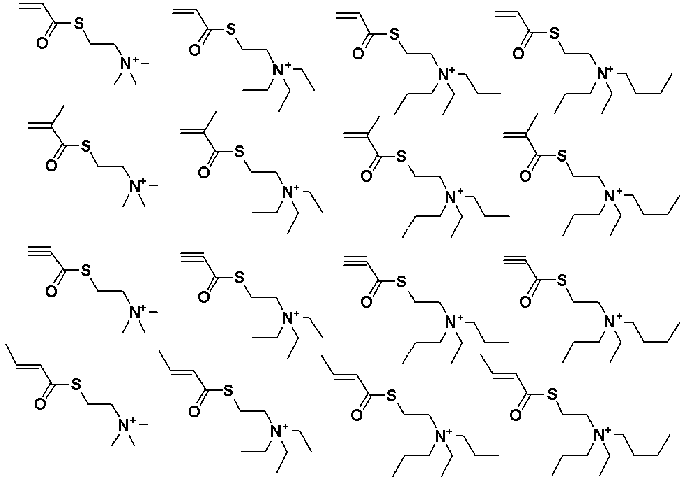

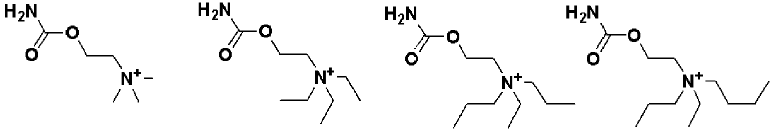

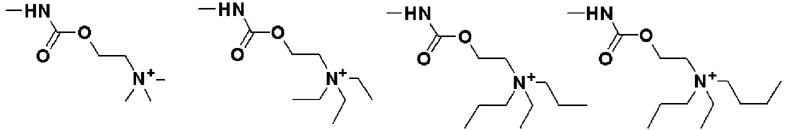

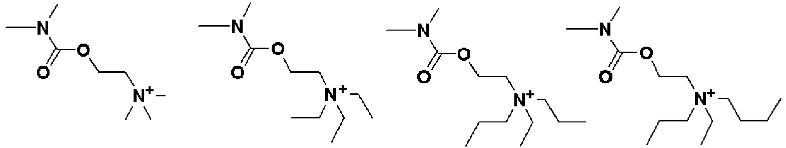

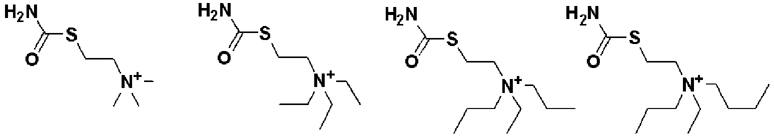

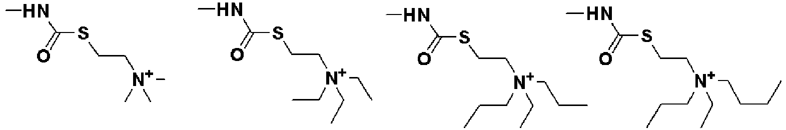

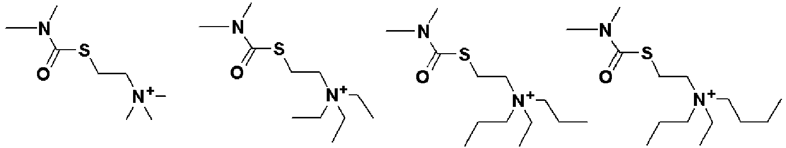

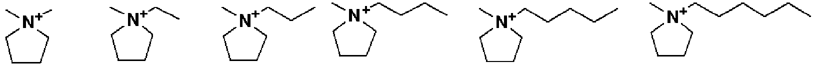

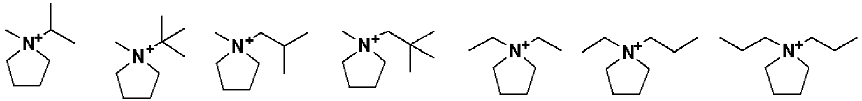

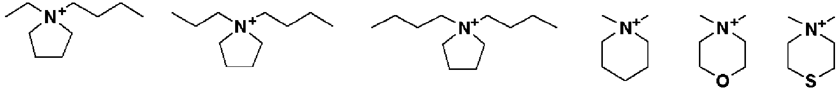

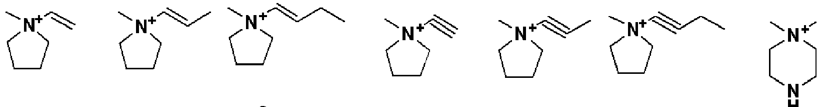

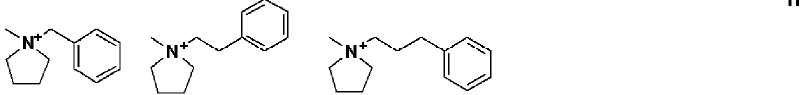

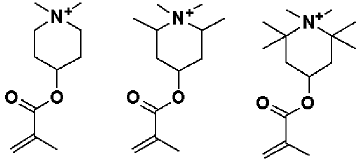

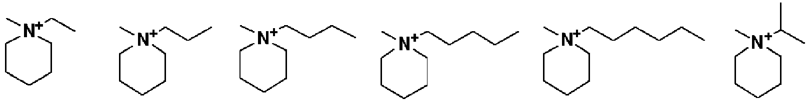

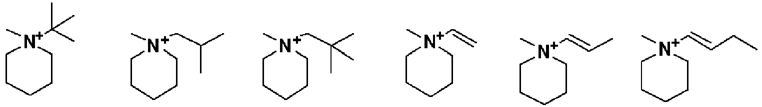

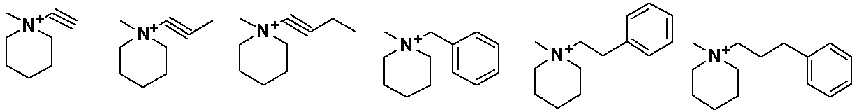

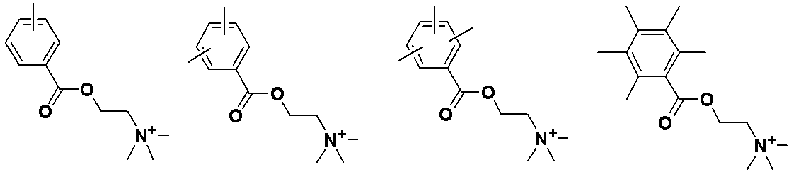

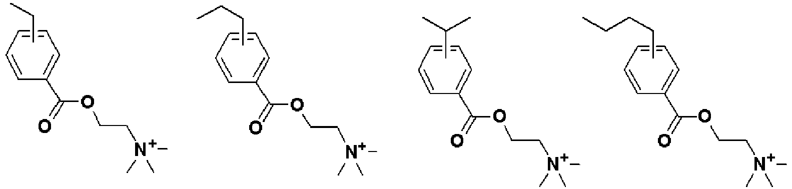

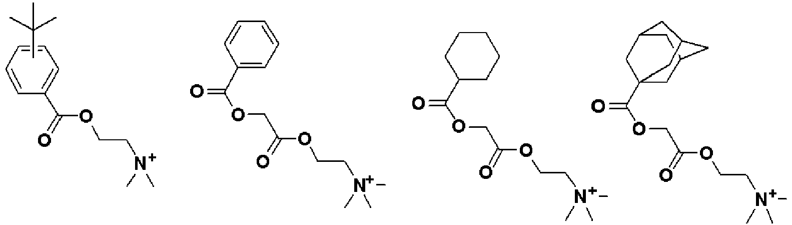

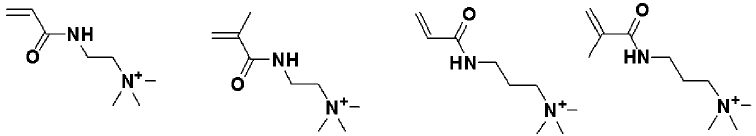

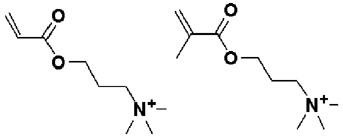

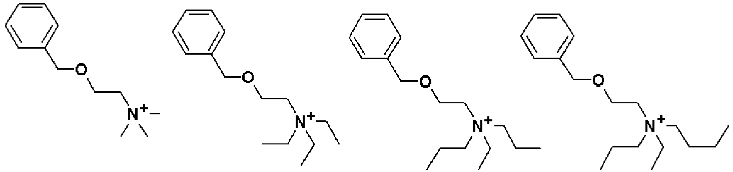

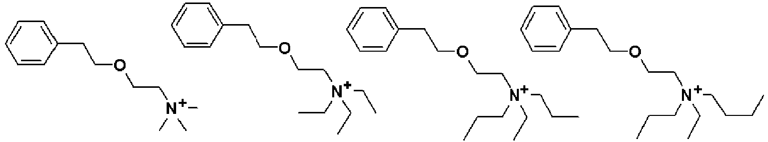

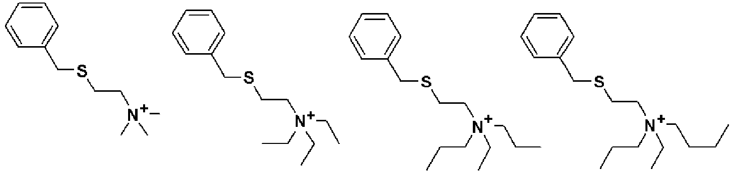

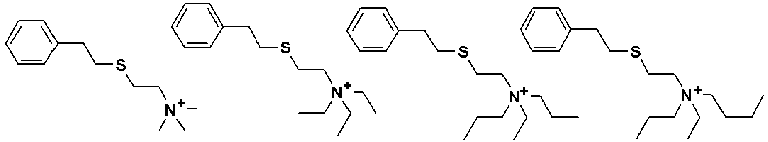

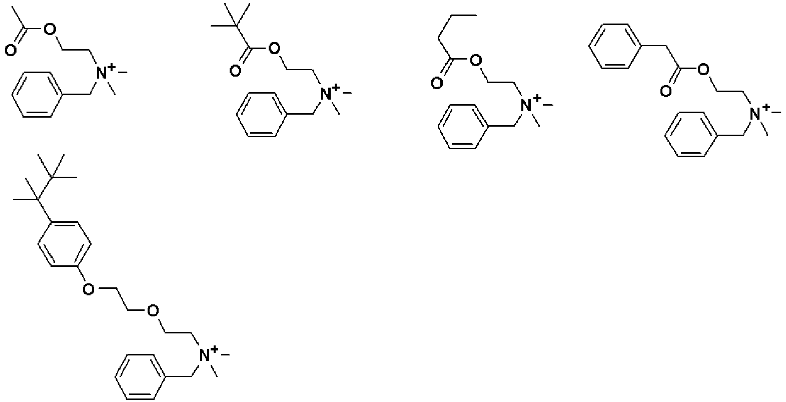

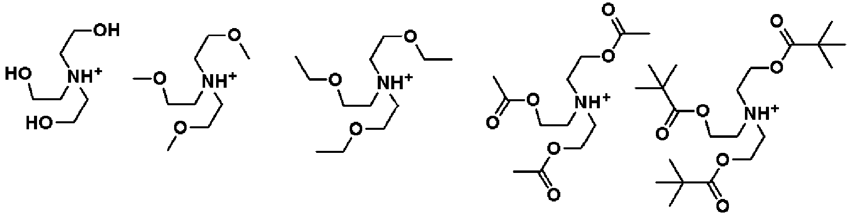

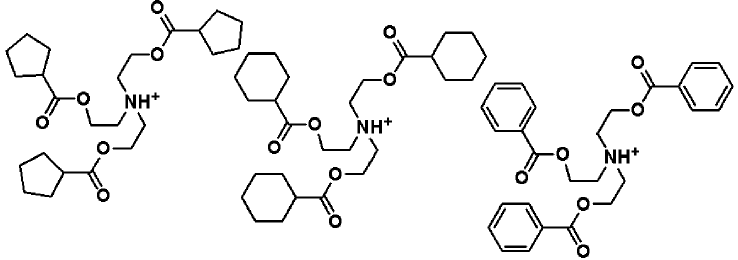

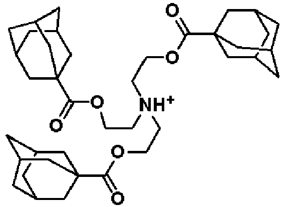

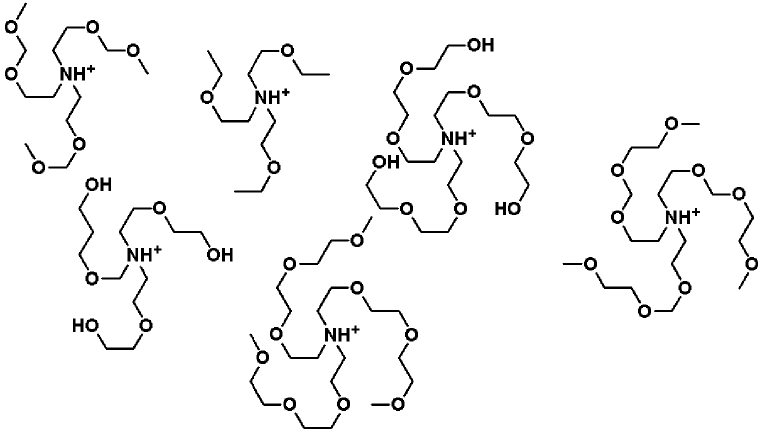

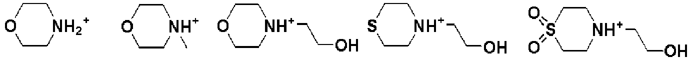

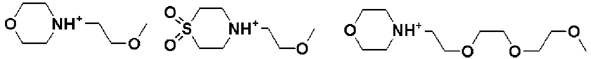

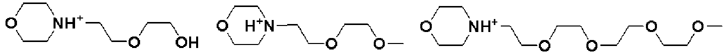

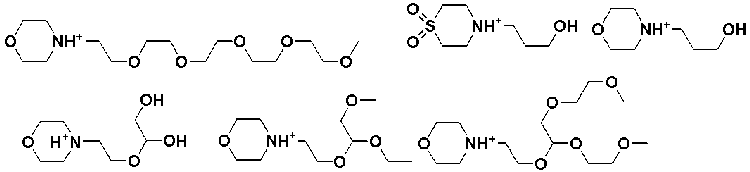

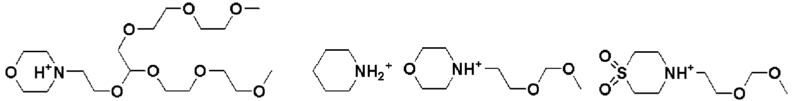

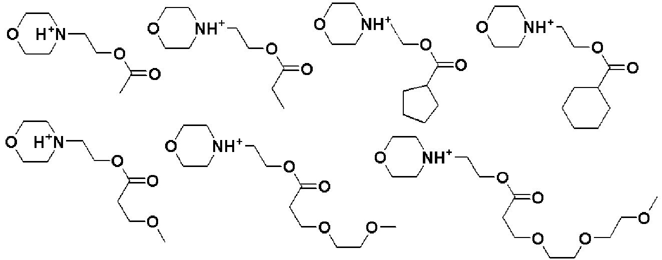

The ammonium ion represented by the above general formula (3) is particularly preferably a tertiary or quaternary ammonium ion.

### (Repeating Unit-b1)

In the component (A) of the inventive bio-electrode composition, in addition to the repeating unit-a1 that is made by polymerizing a monomer having a polymerizable double bond, a repeating unit-b1 having a glyme chain may also be copolymerized in order to enhance the electric conductivity. Specific examples of a monomer to obtain the repeating unit-b1 having a glyme chain can include the following. The copolymerization with the repeating unit having a glyme chain facilitates the movement of ions released from the skin in a dry electrode film, and thus can increase the sensitivity of the dry electrode. 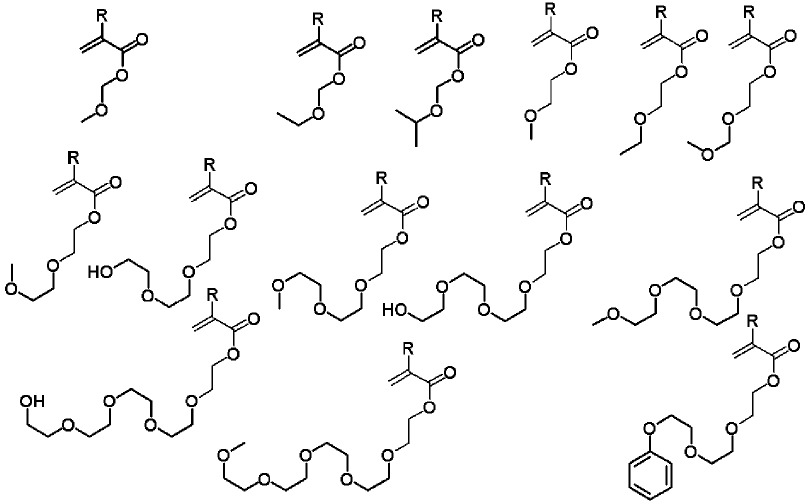 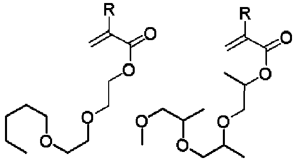 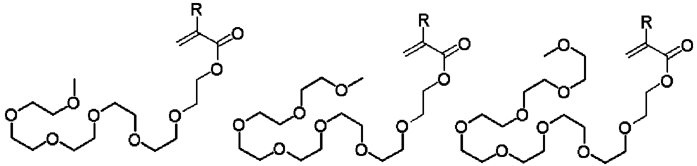 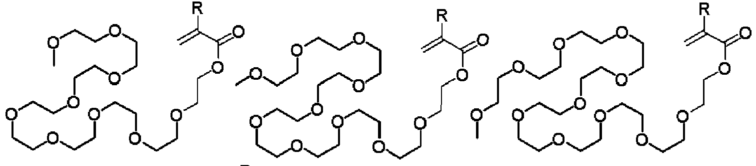 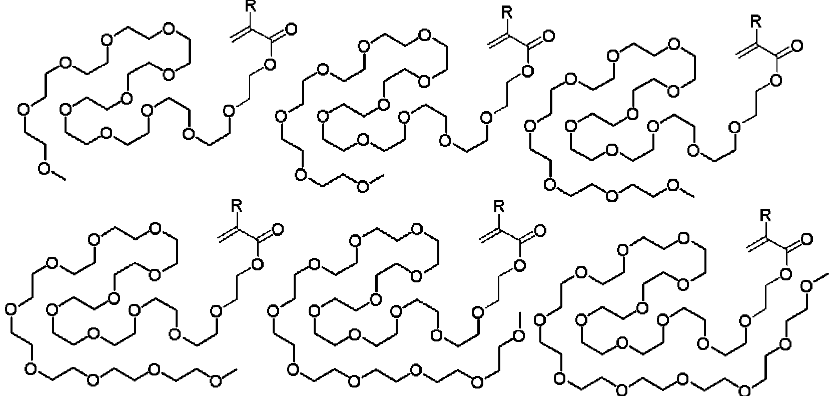 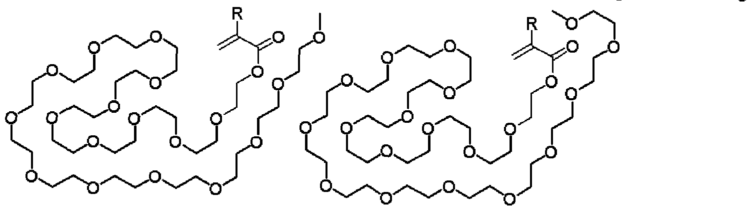 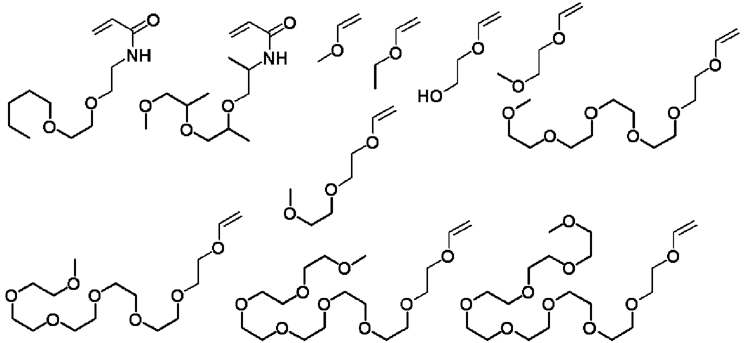 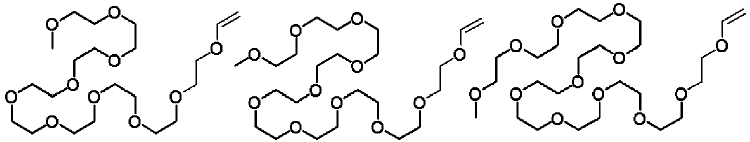 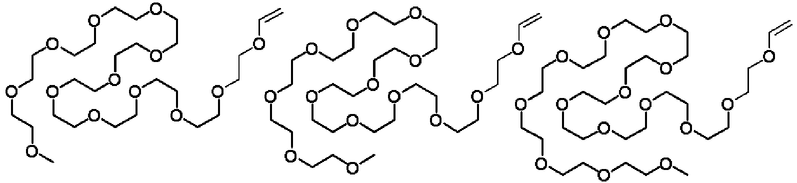 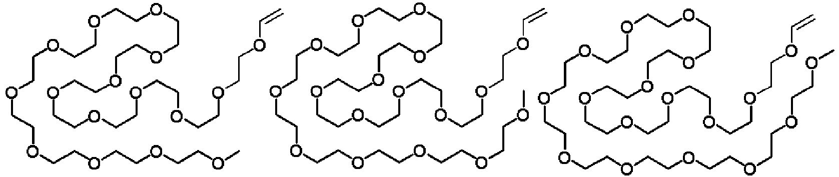 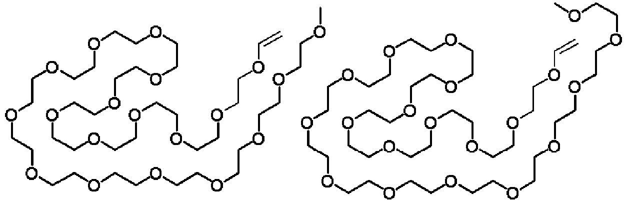 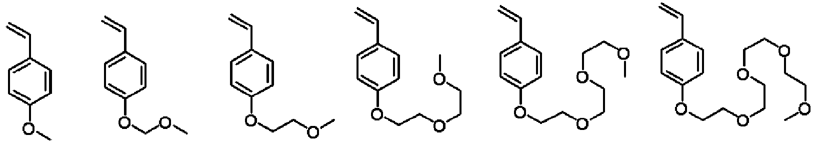 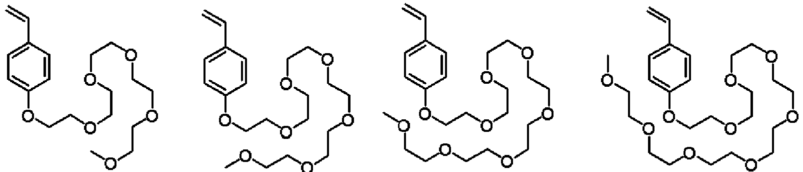 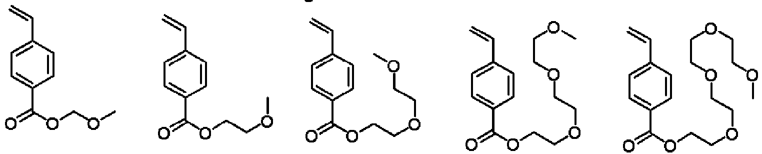 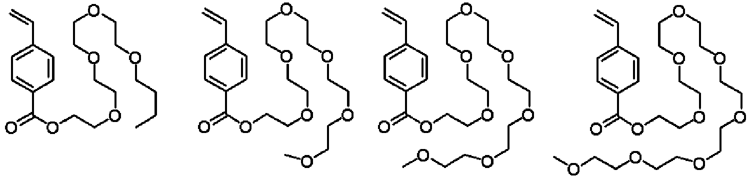 R represents a hydrogen atom or a methyl group.

### (Repeating Unit-c1)

In the component (A) of the inventive bio-electrode composition, in addition to the repeating units-a1 and - b1, it is also possible to copolymerize a hydrophilic repeating unit-c1 having a hydroxy group, a carboxy group, an ammonium salt, betaine, an amide group, pyrrolidone, a lactone ring, a lactam ring, a sultone ring, a sodium salt of sulfonic acid, or a potassium salt of sulfonic acid in order to enhance electric conductivity. Specific examples of a monomer to obtain the hydrophilic repeating unit-c1 can include the following. The copolymerization with the repeating unit containing such a hydrophilic group can increase the sensitivity of the dry electrode by increasing the sensitivity to ions released from the skin. 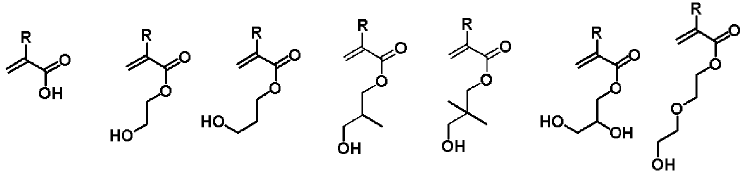 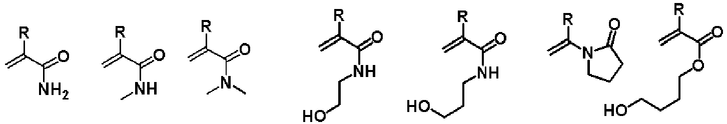 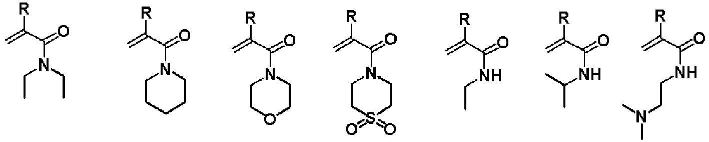 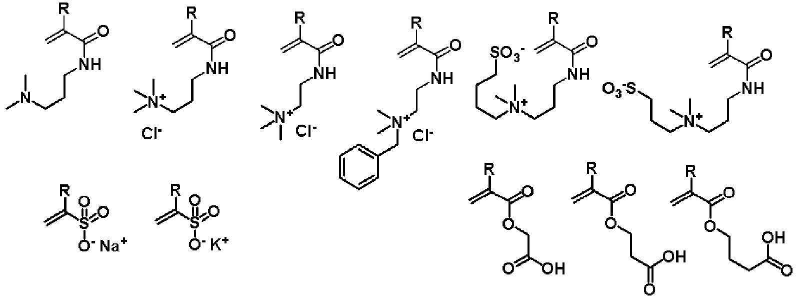 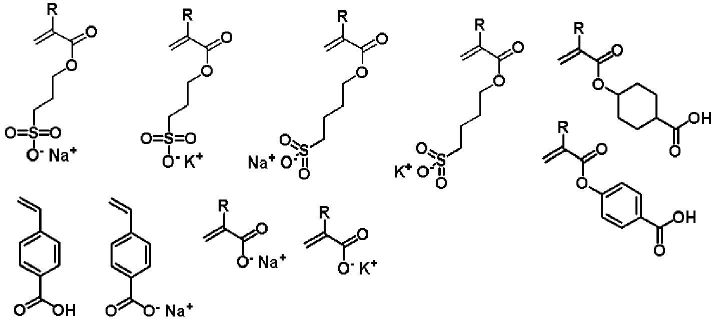 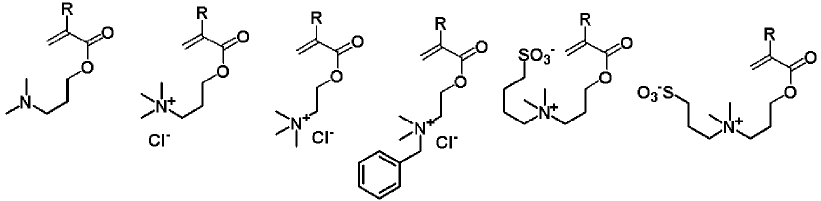 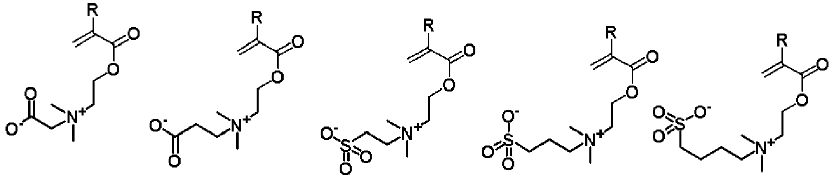 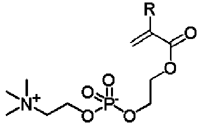 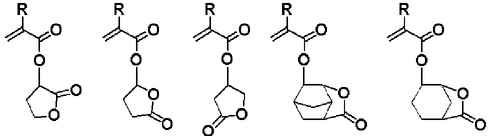 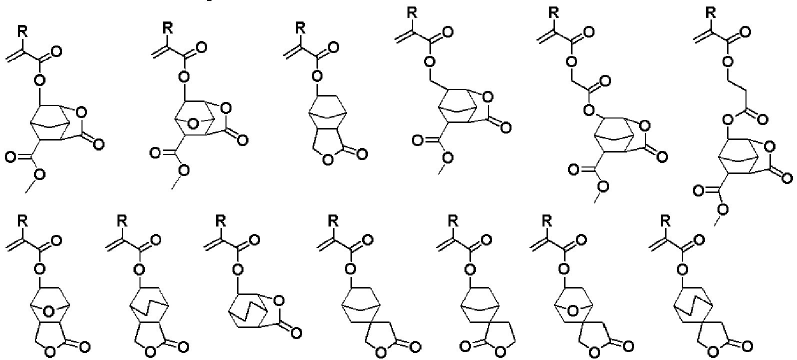 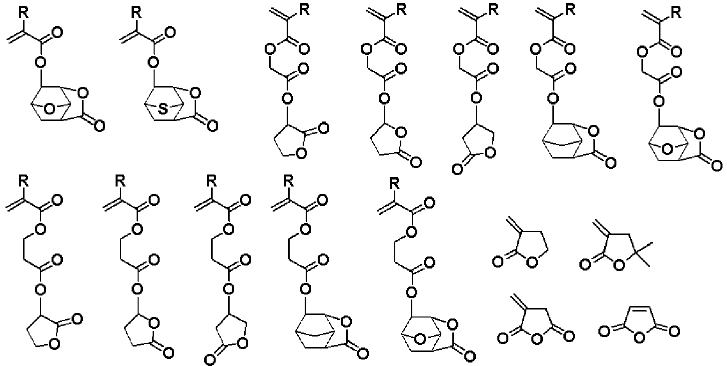 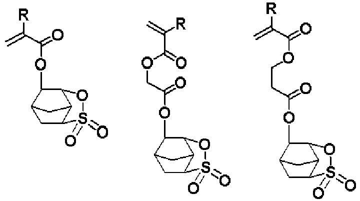 R represents a hydrogen atom or a methyl group.

The component (A) of the inventive bio-electrode composition can have a repeating unit-d1 to impart adhesive properties.

Specific examples of a monomer to obtain the repeating unit-d1 include the following.

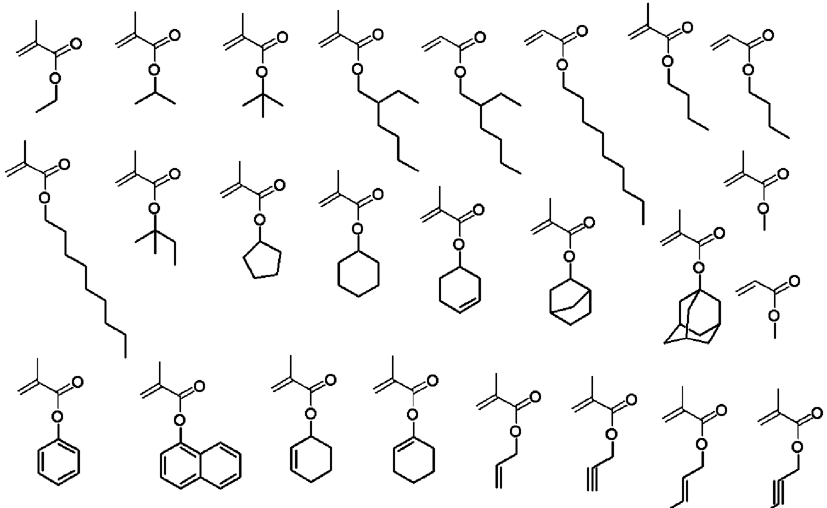

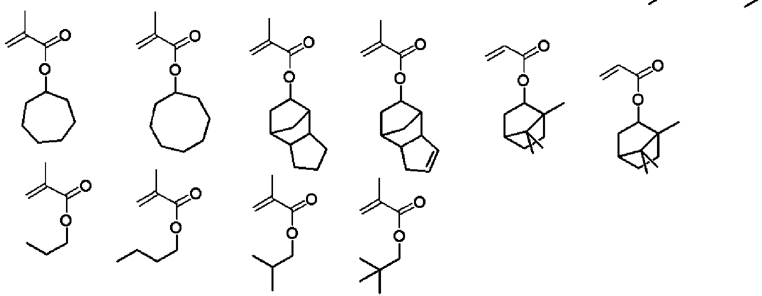

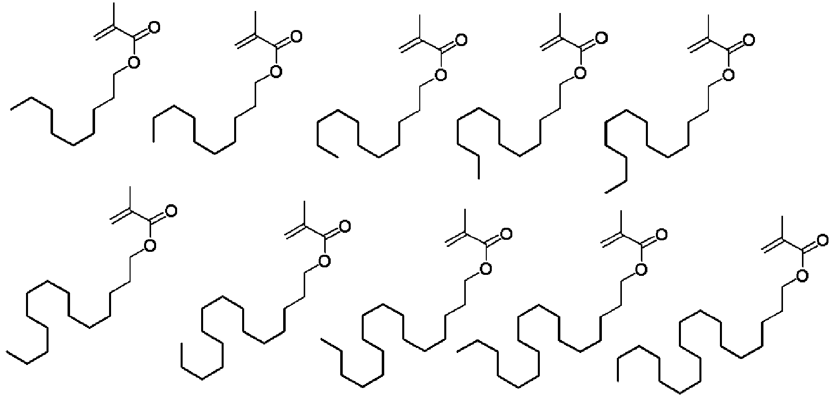

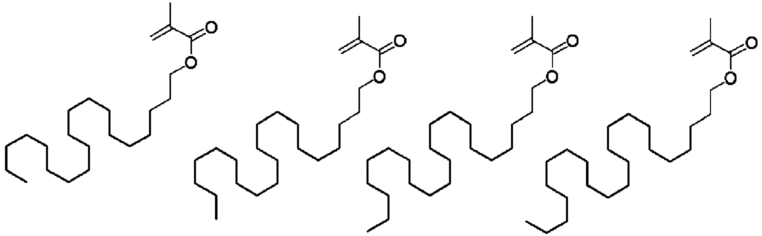

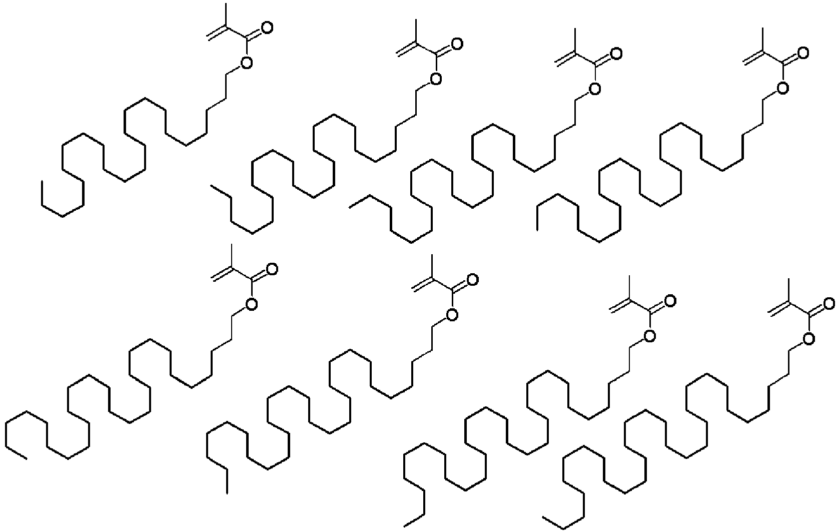

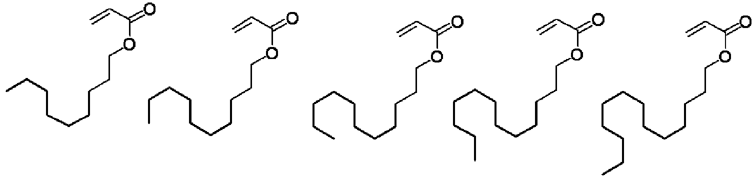

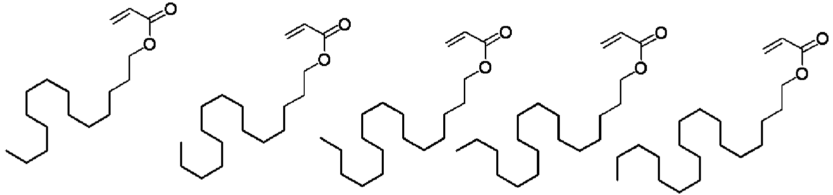

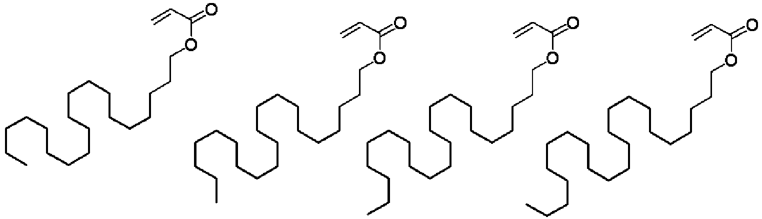

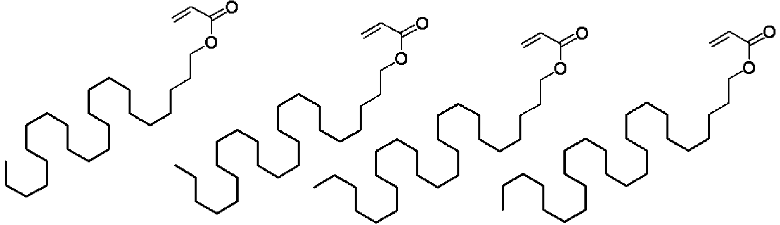

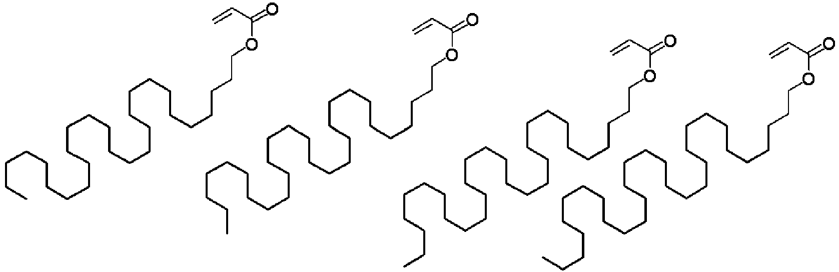

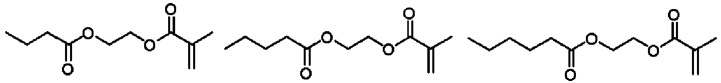

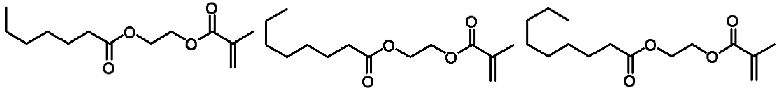

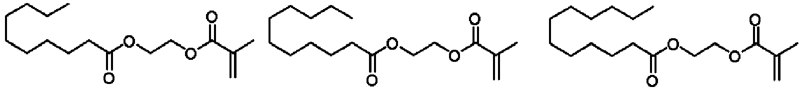

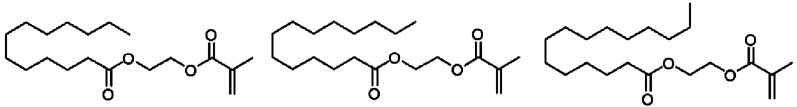

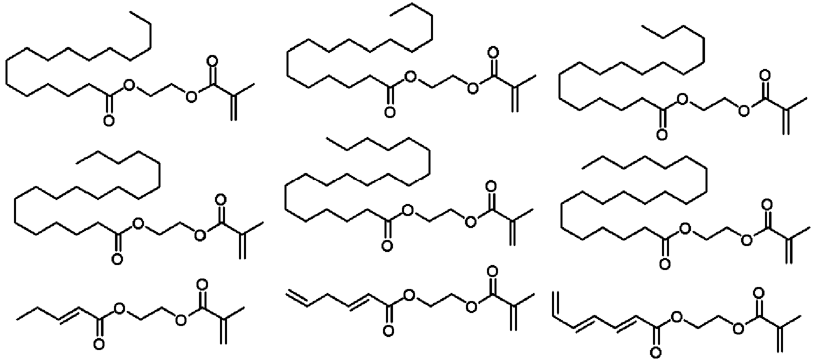

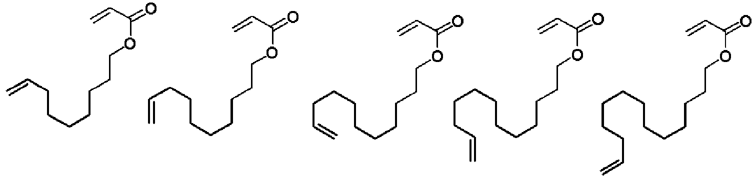

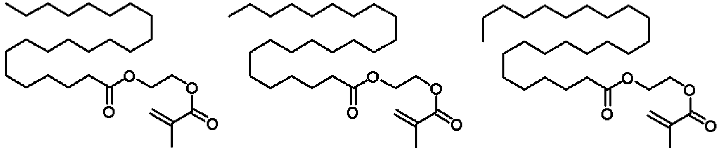

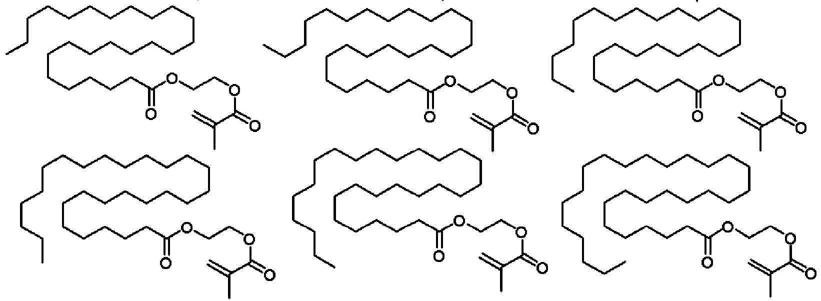

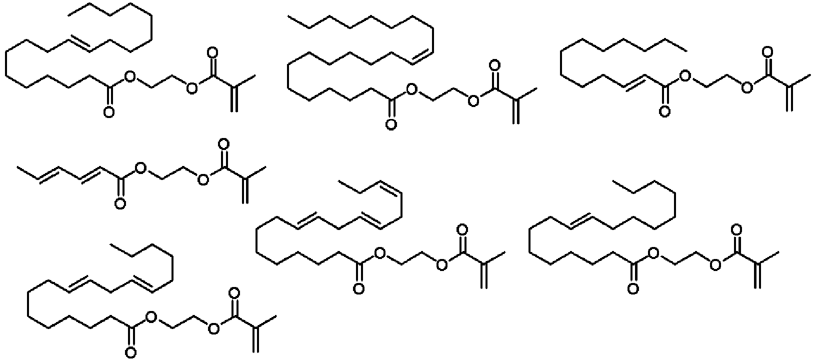

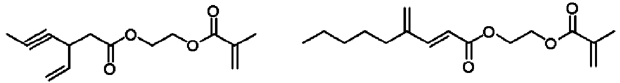

Further, it is also possible to copolymerize a crosslinkable repeating unit-e1. Examples of the crosslinkable repeating unit can include repeating units having an oxirane ring or an oxetane ring.

Specific examples of monomers to obtain the repeating unit-e1 having an oxirane ring or an oxetane ring can include the following. 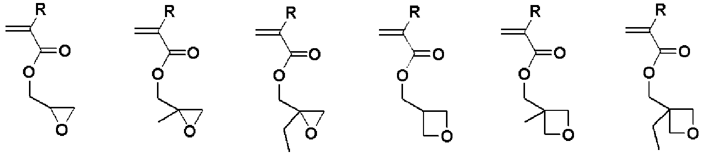 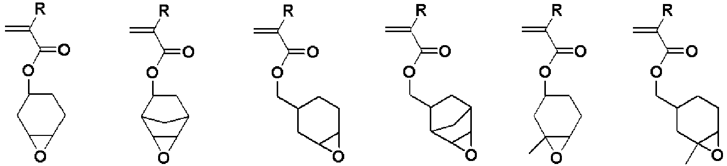 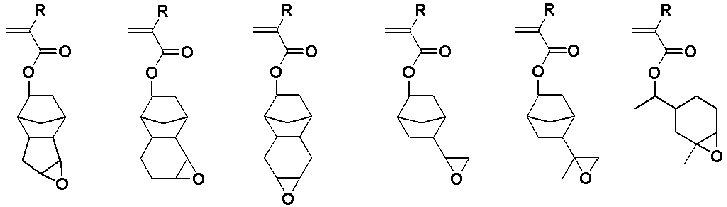 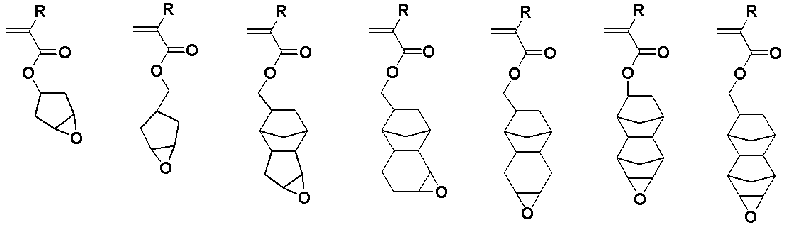 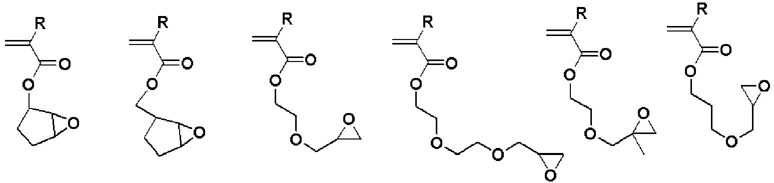 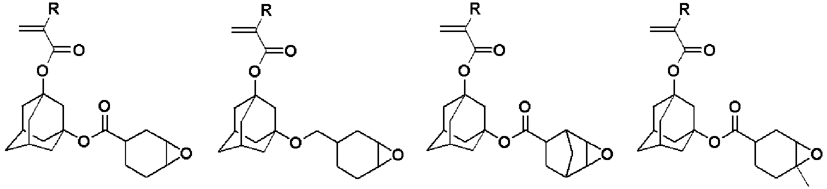 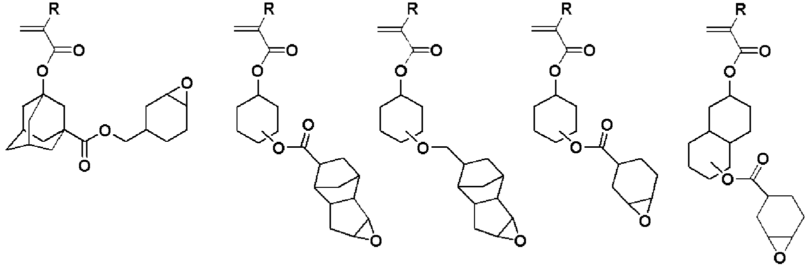 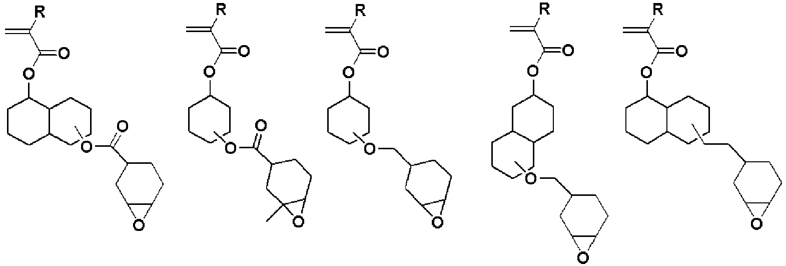 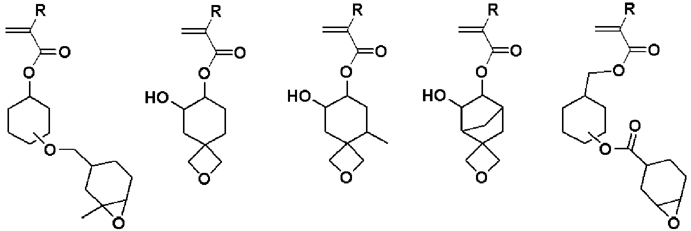 Here, R represents a methyl group or a hydrogen atom.

In addition to the repeating unit(s) selected from the above-described -a1, -b1, -c1, -d1, and -e1, the component (A) of the inventive bio-electrode composition can also have a repeating unit-f1 having silicon. Specific examples can include the following.

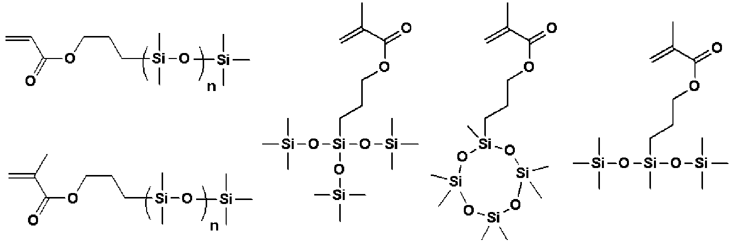

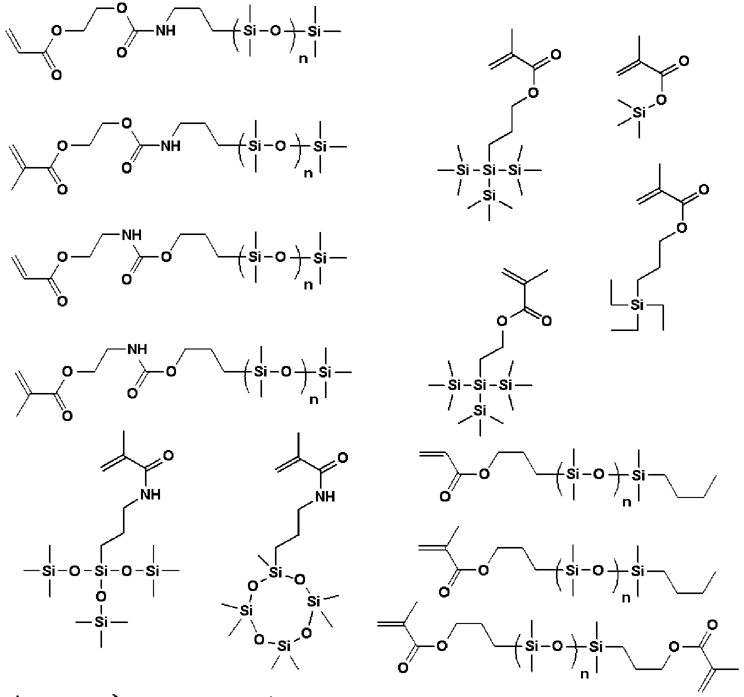

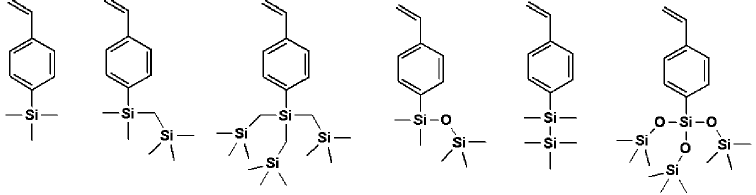

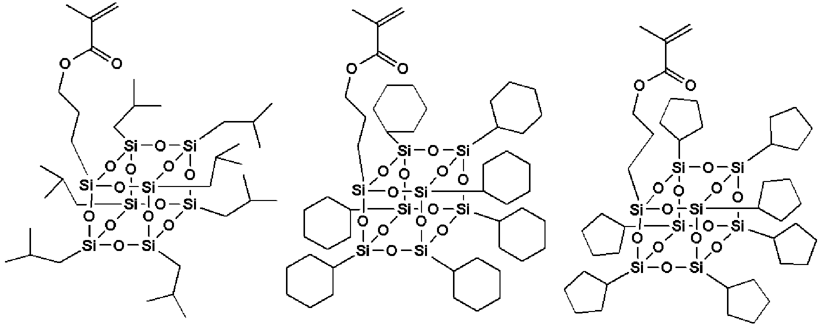

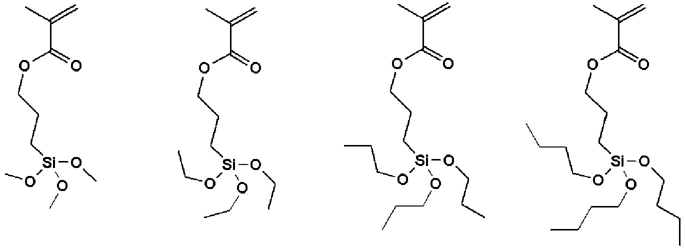

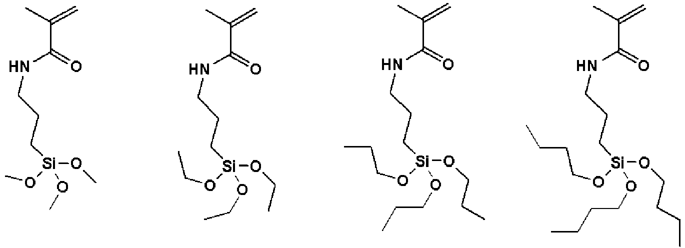

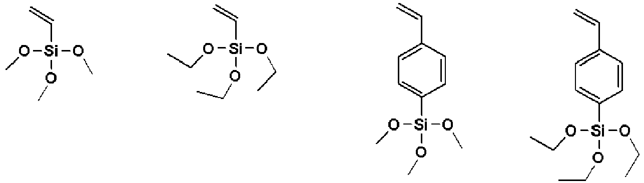

A silsesquioxane structure may be formed by first copolymerizing the above repeating units having an alkoxysilyl group and then hydrolyzing the alkoxysilyl group as disclosed in JP 2022-164579 A, or a complex with silica may be formed by reacting with silanol on the surface of silica as disclosed in JP 2022-64291 A.

In addition to the repeating unit(s) selected from the group consisting of the above-described -a1, -b1 to -f1, the component (A) of the inventive bio-electrode composition can also have a repeating unit-g1 having fluorine.

Specific examples of monomers to obtain the repeating unit-g1 having fluorine can include the following. 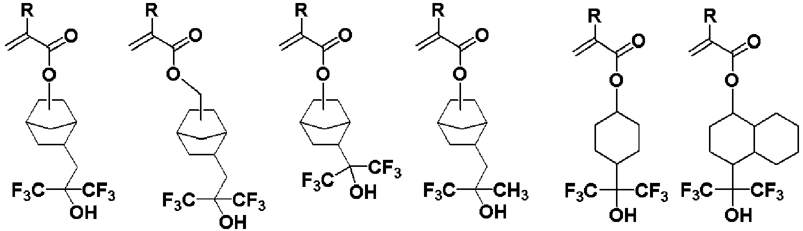 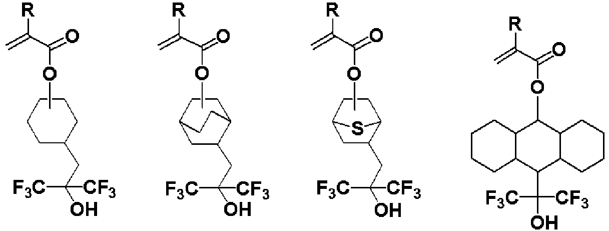 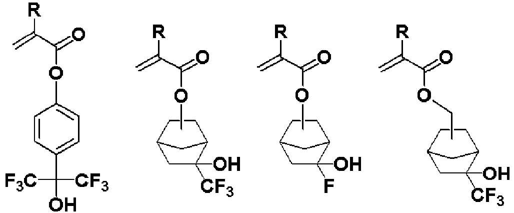 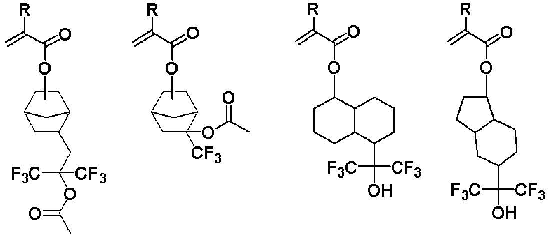 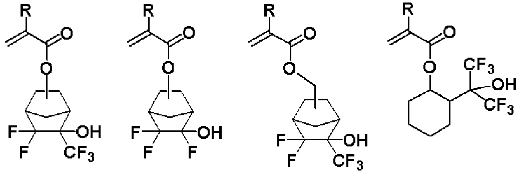 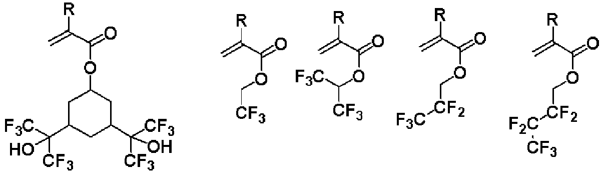 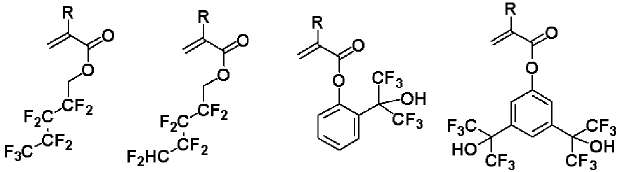 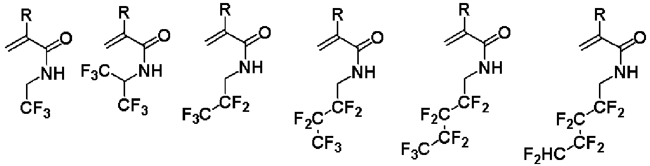 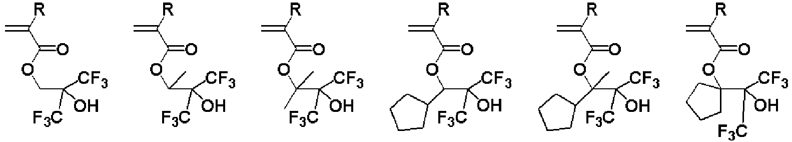 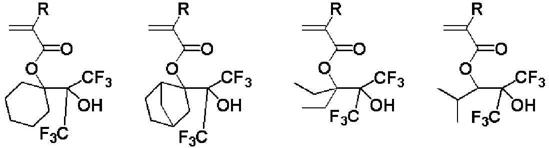 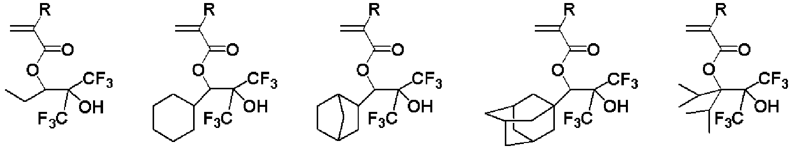 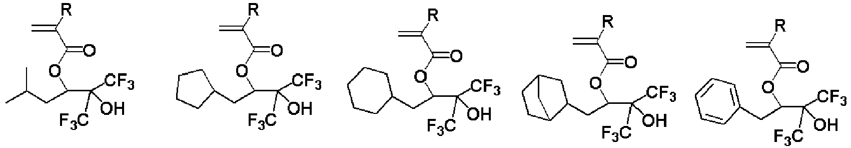 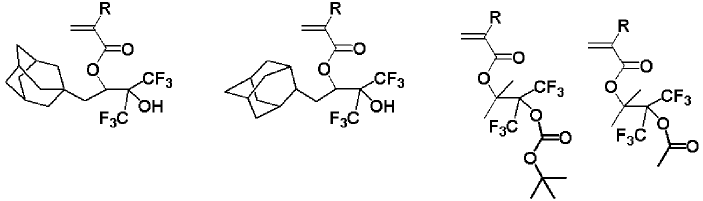 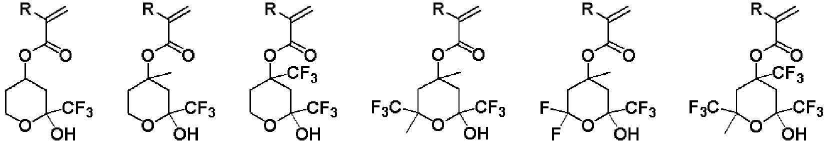 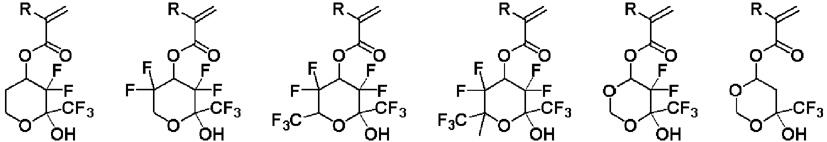 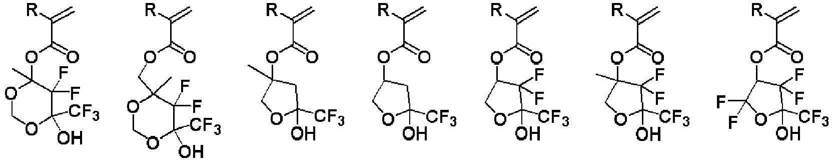 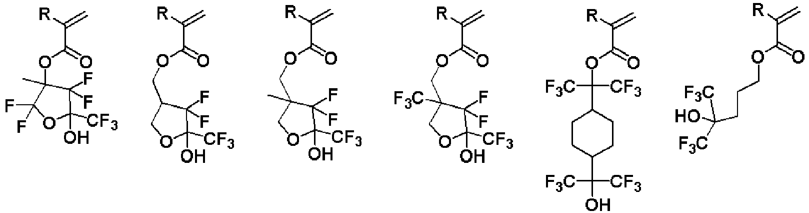 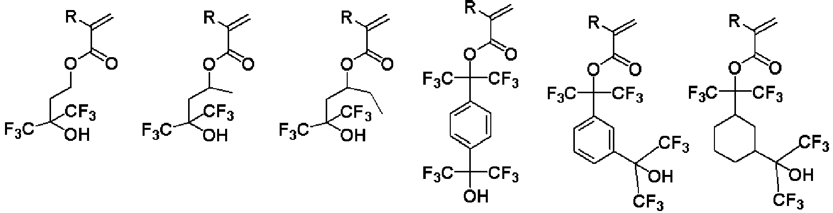 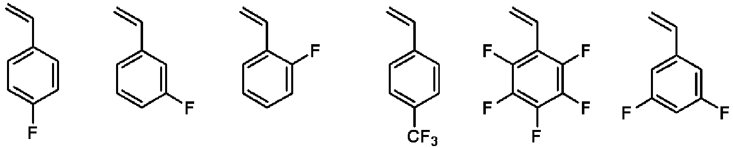 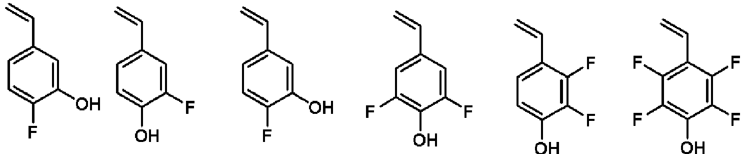 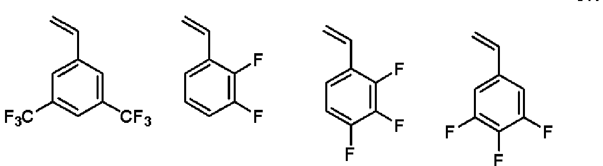 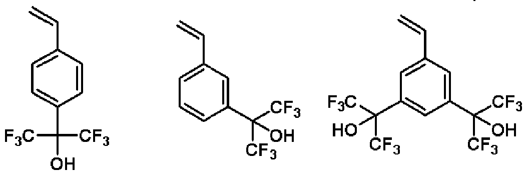 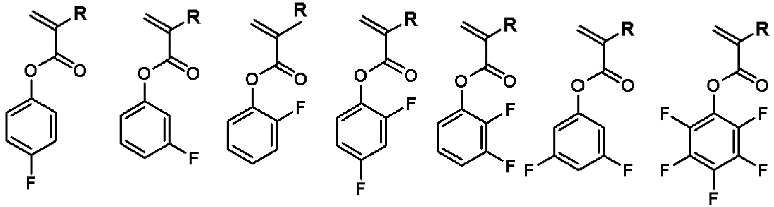 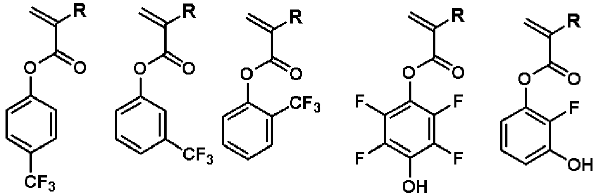 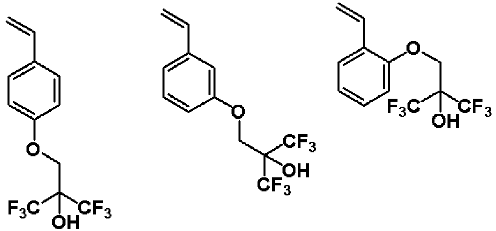 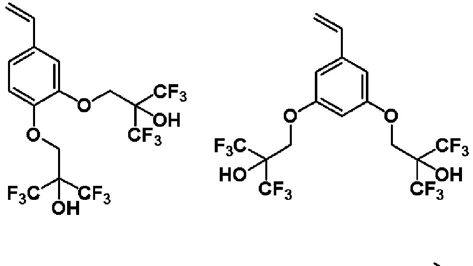 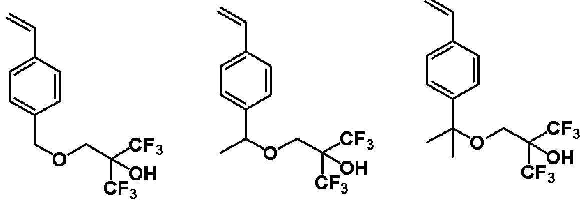 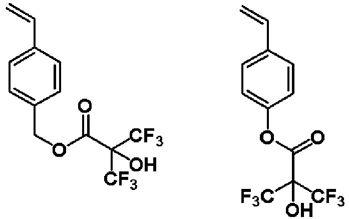 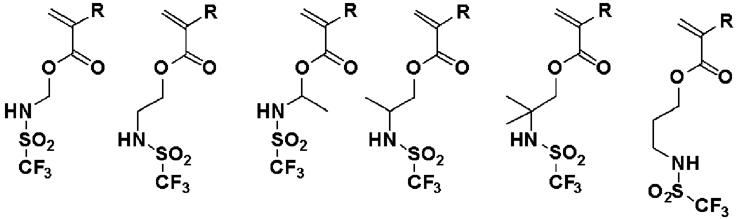 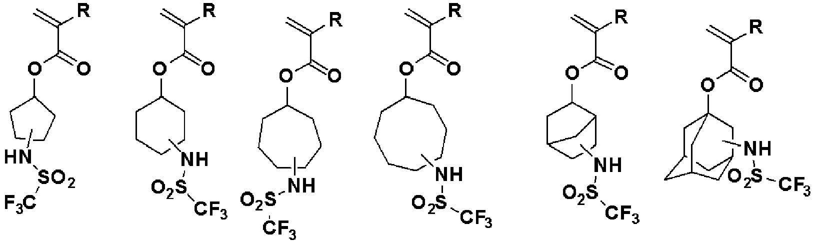 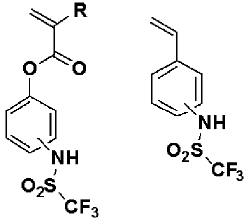 Here, R represents a hydrogen atom or a methyl group.

In addition to the repeating unit(s) selected from the group consisting of the above-described -a1, -b1 to -g1, the component (A) of the inventive bio-electrode composition can also have a repeating unit-h1 having a cyano group.

Specific examples of monomers to obtain the repeating unit-h1 having a cyano group can include the following. 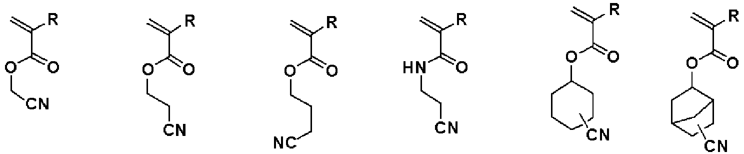 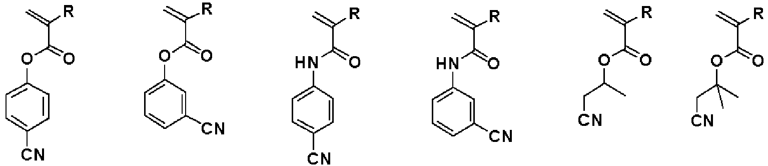 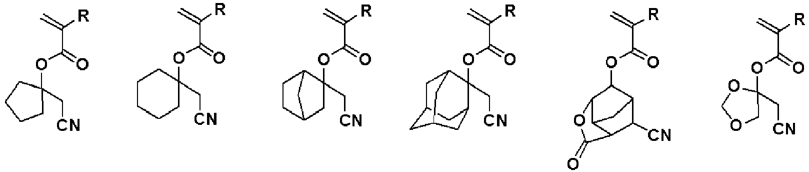 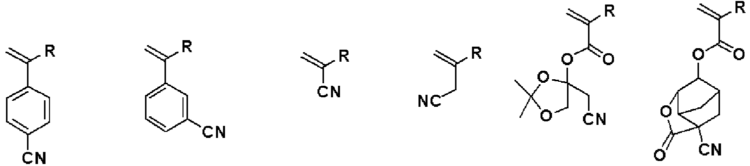 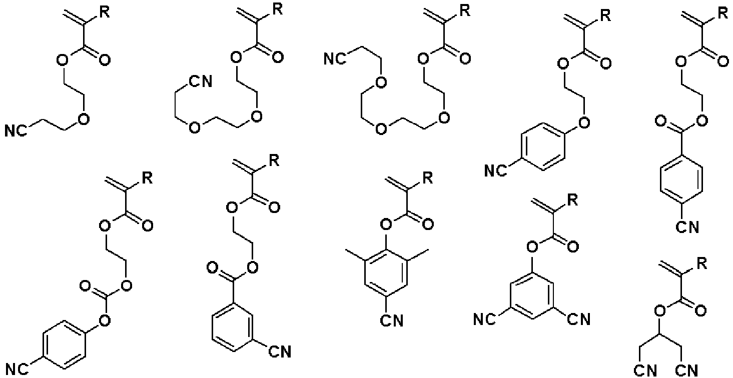 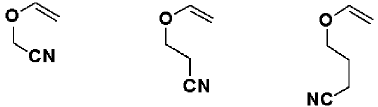 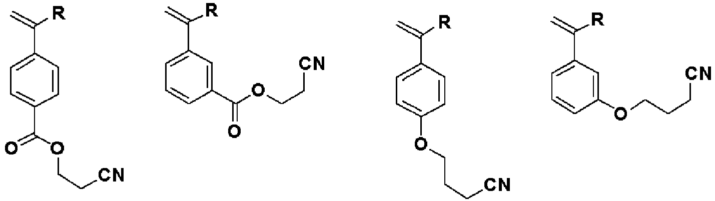 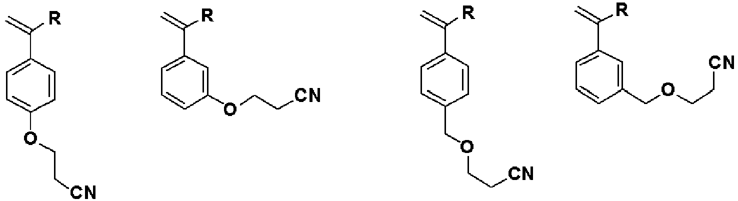 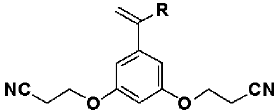 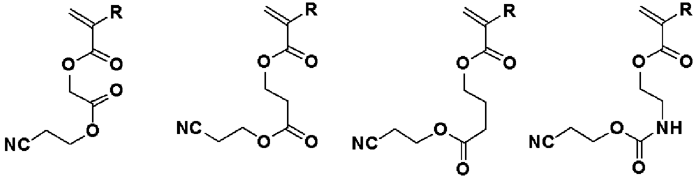 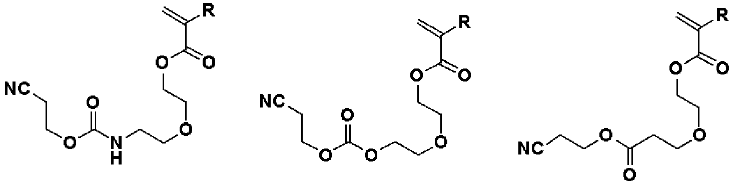 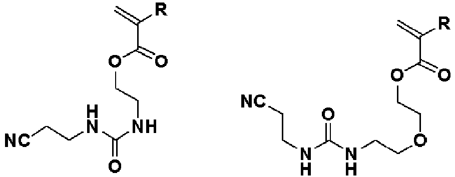 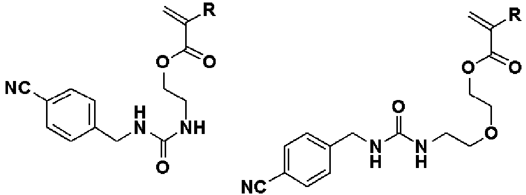 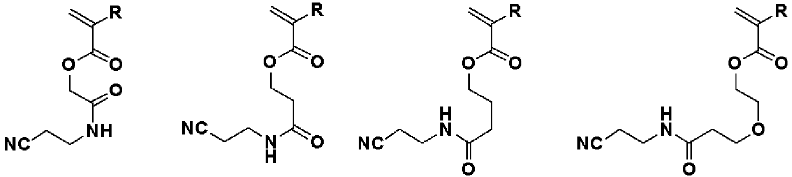 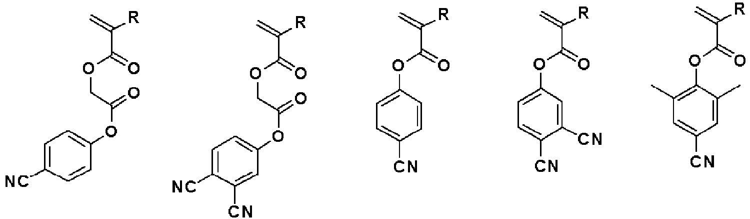 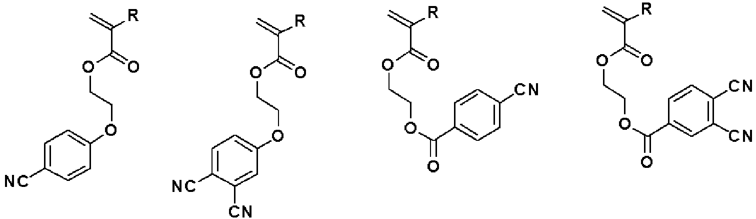 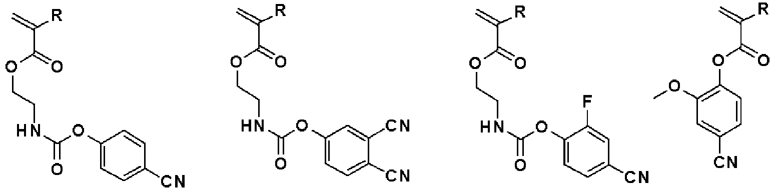 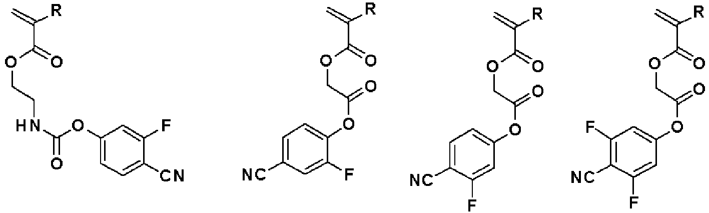 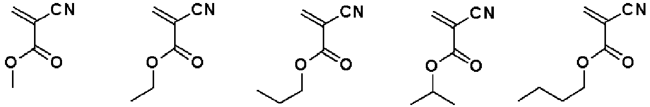 Here, R represents a hydrogen atom or a methyl group.

In addition to the repeating unit(s) selected from the group consisting of the above-described -a1, -b1 to -h1, the component (A) of the inventive bio-electrode composition can also have a repeating unit-i1 having a nitro group.

Specific examples of monomers to obtain the repeating unit-i1 having a nitro group can include the following. 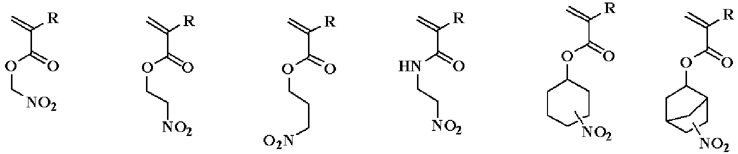 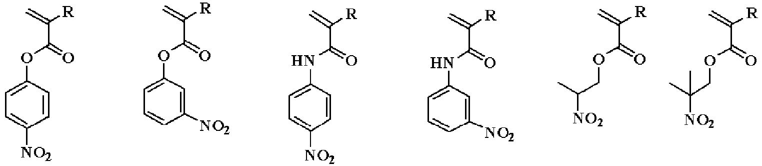 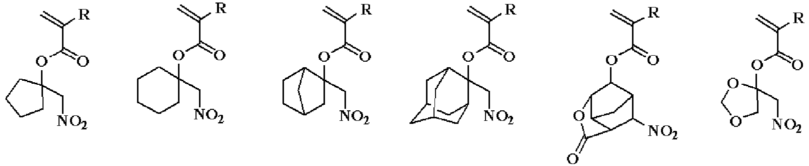 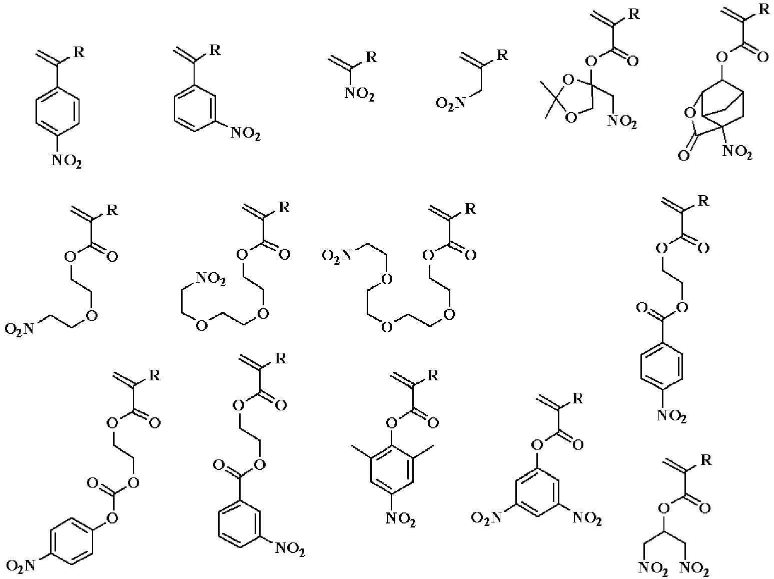 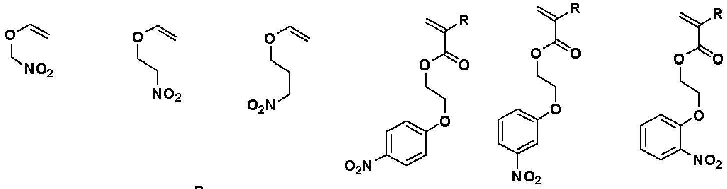 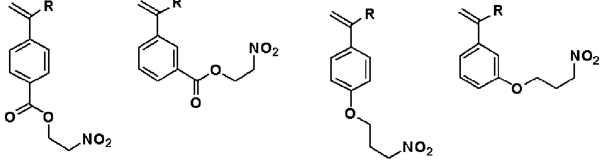 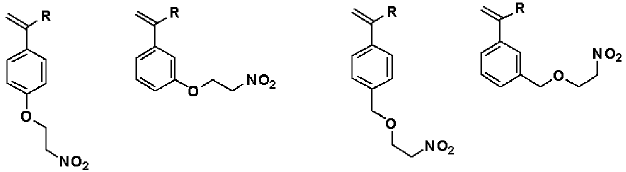 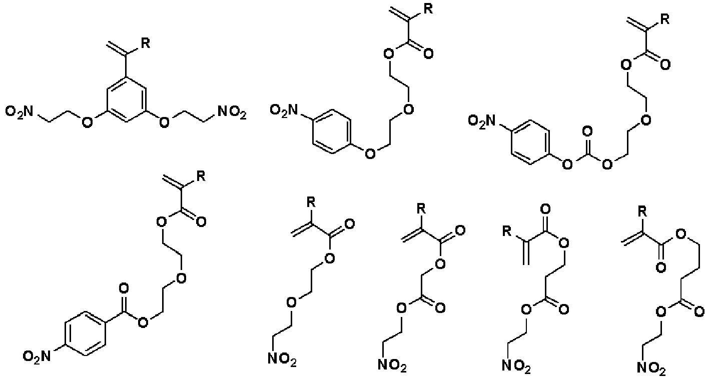 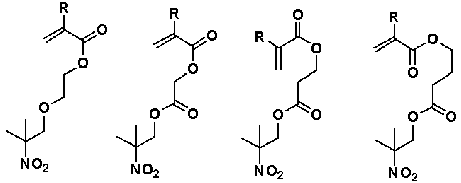 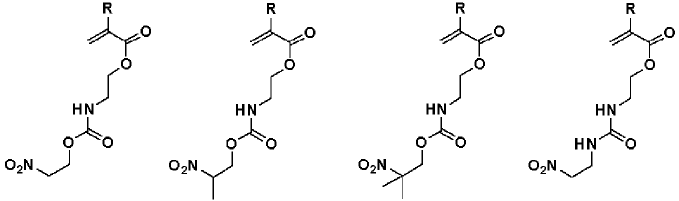 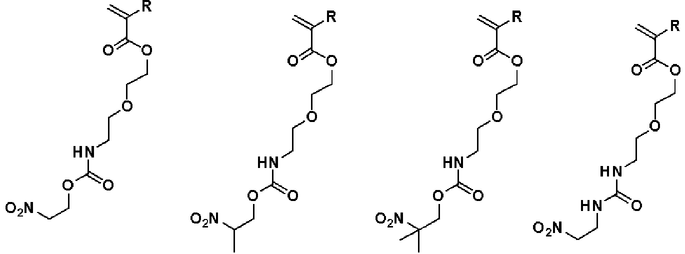 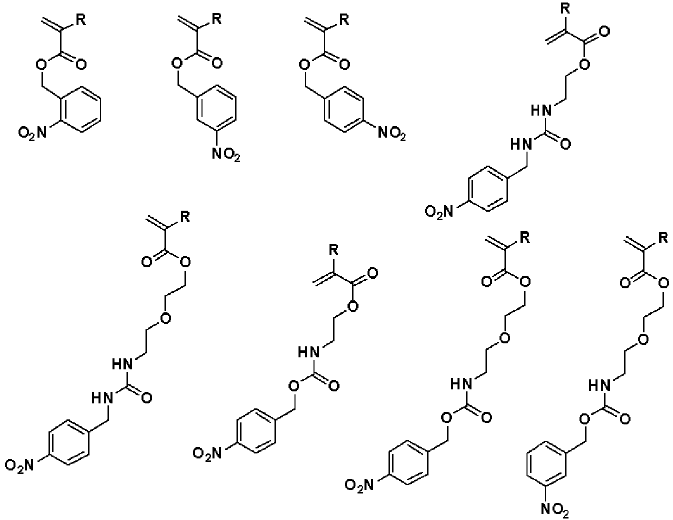 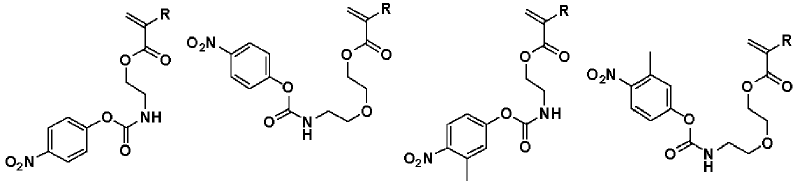 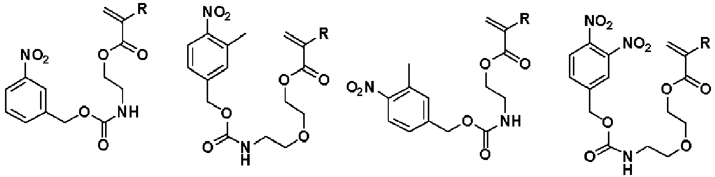 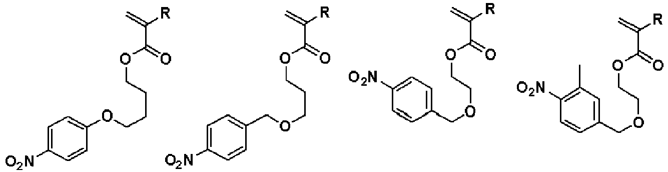 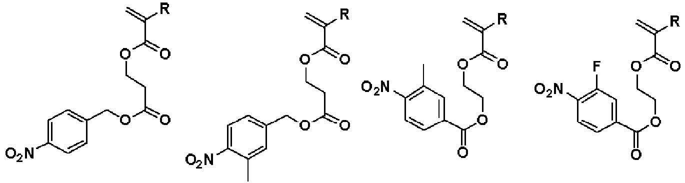 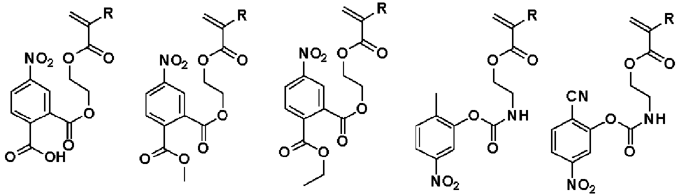 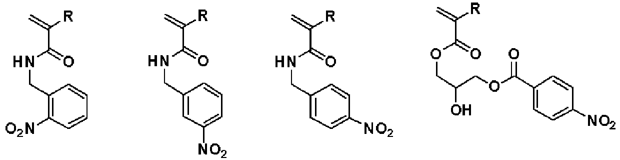 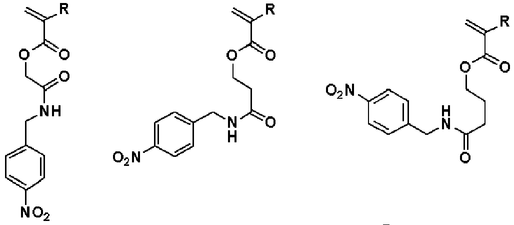 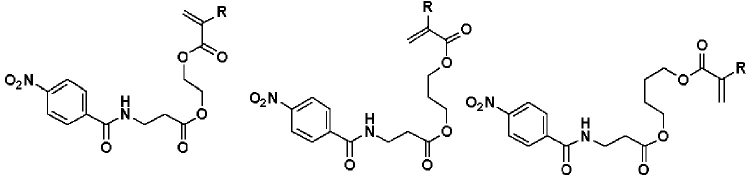 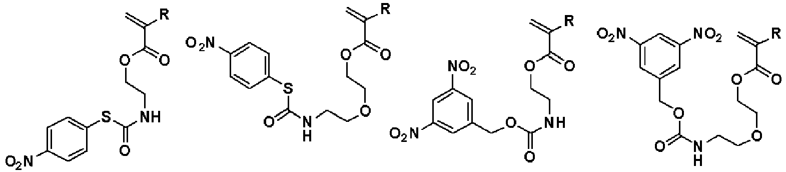 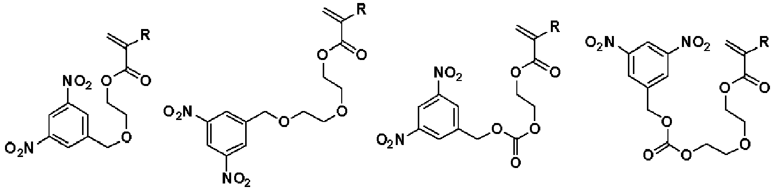 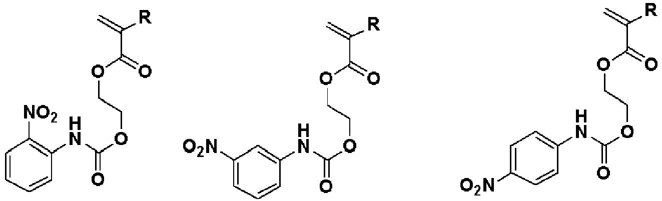 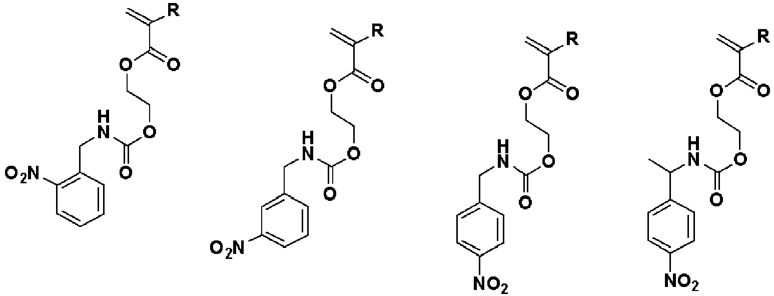 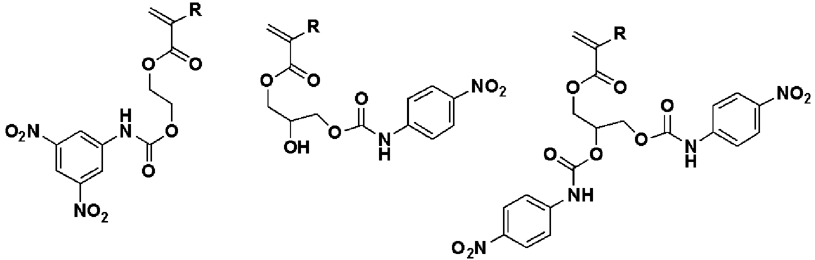 Here, R represents a hydrogen atom or a methyl group.

As one method for synthesizing an ionic resin (a polymer compound) containing -a1 having a polymerizable double bond of component (A), a polymer compound of a copolymer can be obtained by a method in which desired monomer(s) among the monomers to obtain the repeating units-al, -b1, -c1, -d1, -e1, -f1, -g1, -h1, and -i1 undergo heat polymerization in an organic solvent to which a radical polymerization initiator is added.

Examples of the organic solvent used in the polymerization include toluene, benzene, tetrahydrofuran, diethyl ether, dioxane, etc. Examples of the polymerization initiator include 2,2'-azobisisobutyronitrile (AIBN), 2,2'-azobis(2,4-dimethylvaleronitrile), dimethyl 2,2-azobis(2-methylpropionate), benzoyl peroxide, lauroyl peroxide, etc. The heating temperature is preferably 50 to 80°C, and the reaction time is preferably 2 to 100 hours, more preferably 5 to 20 hours.

Here, the proportions of the repeating units-al, - b1, -c1, -d1, -e1, -f1, -g1, -h1, and -i1 in the component (A) ionic resin can be 0<a1≤1.0, 0≤b1<1.0, 0≤c1<1.0, 0≤d1<1.0, 0≤e1<0.9, 0≤f1<0.9, 0≤g1<0.9, 0≤h1<0.9, 0≤i1<0.9; preferably 0.1≤a1≤0.9, 0.01≤b1≤0.9, 0≤c1≤0.8, 0≤d1≤0.8, 0≤e1<0.8, 0≤f1<0.8, 0≤g1<0.8, 0≤h1<0.8, 0≤i1<0.8; more preferably 0.15≤a1≤0.8, 0.05≤b1≤0.8, 0≤c1≤0.7, 0≤d1≤0.5, 0≤e1<0.3, 0≤f1<0.7, 0≤g1<0.7, 0≤h1<0.7, 0≤i1<0.7.

Note that, a1+b1+c1+d1+e1+f1+g1+h1+i1=1 means, for example, that the total amount of the repeating units-a1, -b1, -c1, -d1, -e1, -f1, -g1, -h1, and -i1 is 100 mol% based on the total amount of all the repeating units, in the polymer compound containing the repeating units-al, -b1, -c1, -d1, -e1, -f1, -g1, -h1, and -i1; and a1+b1+c1+d1+e1+f1+g1+h1+i1<1 means that the total amount of the repeating units-al, -b1, -c1, -d1, -e1, - f1, -g1, -h1, and -i1 is less than 100 mol% based on the total amount of all the repeating units, indicating that the polymer compound has other repeating unit(s) than the repeating units-al, -b1, -c1, -d1, -e1, -f1, -g1, - h1, and -i1.

The molecular weight of the component (A) ionic resin having -a1 having a polymerizable double bond, as the weight-average molecular weight, is preferably 500 or more for both, more preferably 1,000 or more and 1,000,000 or less, and further preferably 2,000 or more and 500,000 or less. When the amount of ionic monomer (residual monomer) that is not incorporated into the component (A) ionic resin after polymerization is small, there is no risk that the residual monomer permeates into the skin and causes allergy in biocompatibility test. Accordingly, it is preferable to decrease the amount of residual monomers. The amount of residual monomers is preferably 10 parts by mass or less based on 100 parts by mass of the entire component (A) ionic resin before condensation reaction of the component (A). In addition, one kind of the component (A) may be used, or two or more kinds which differ in molecular weight, dispersity, and constitutive polymerizable monomer may be used in mixture.

### [Silicone Resin]

Specific examples of the repeating unit a2-1 represented by the above general formula (2)-2-1 can include the following. 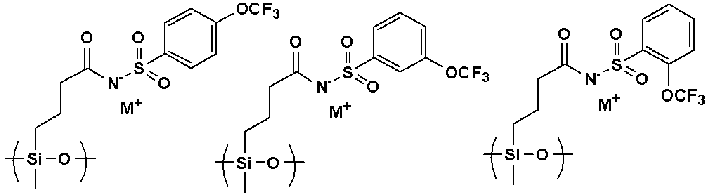 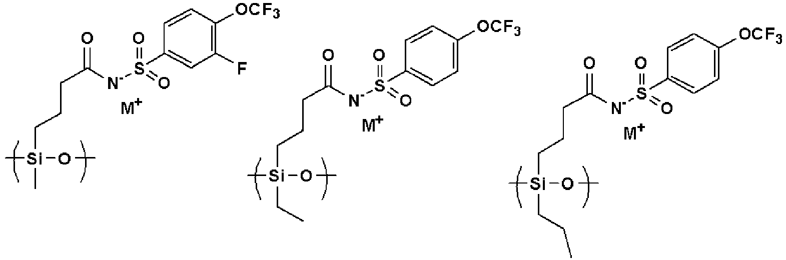 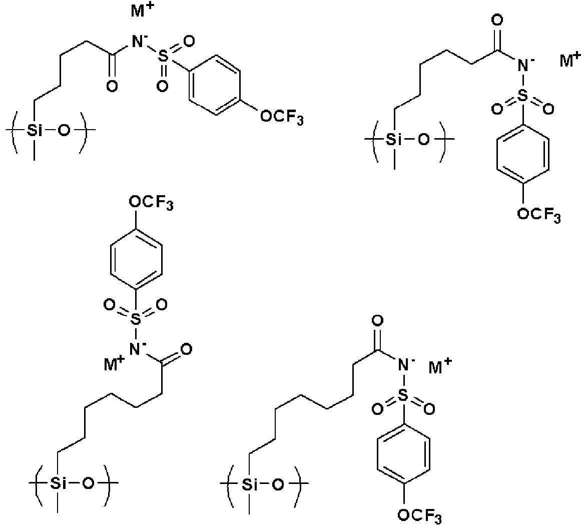 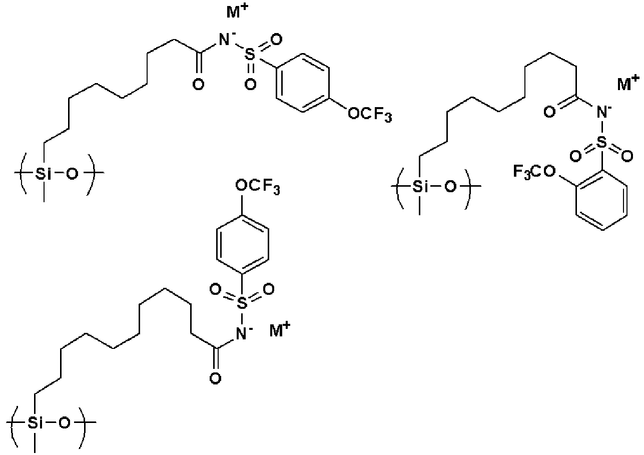 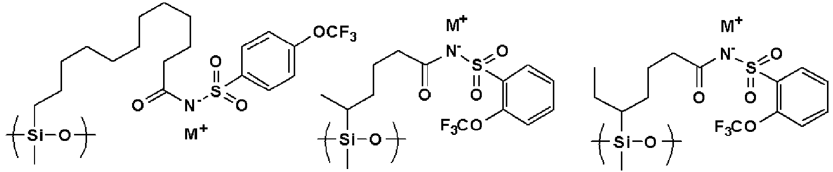 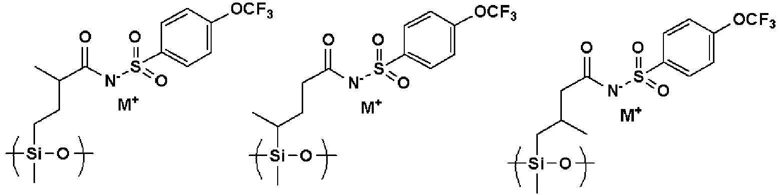 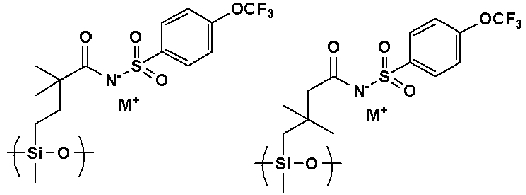 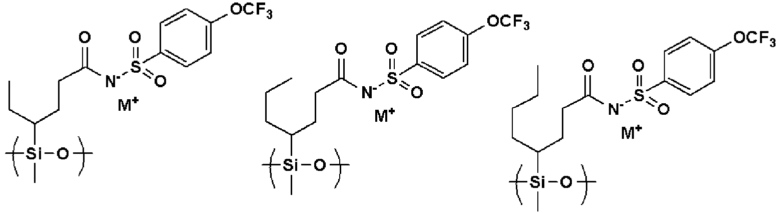 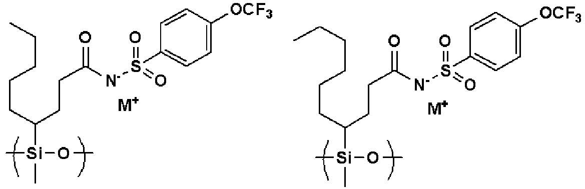 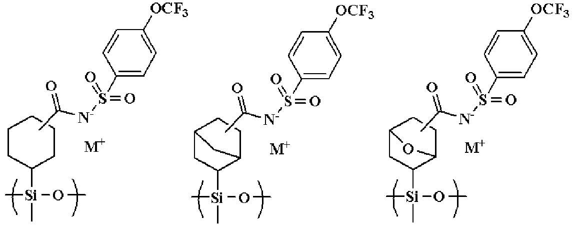 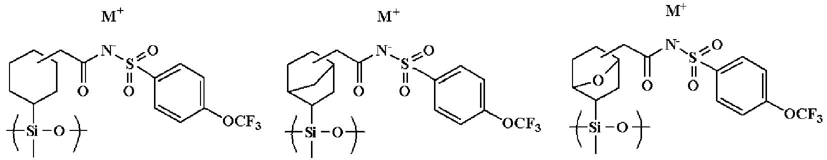 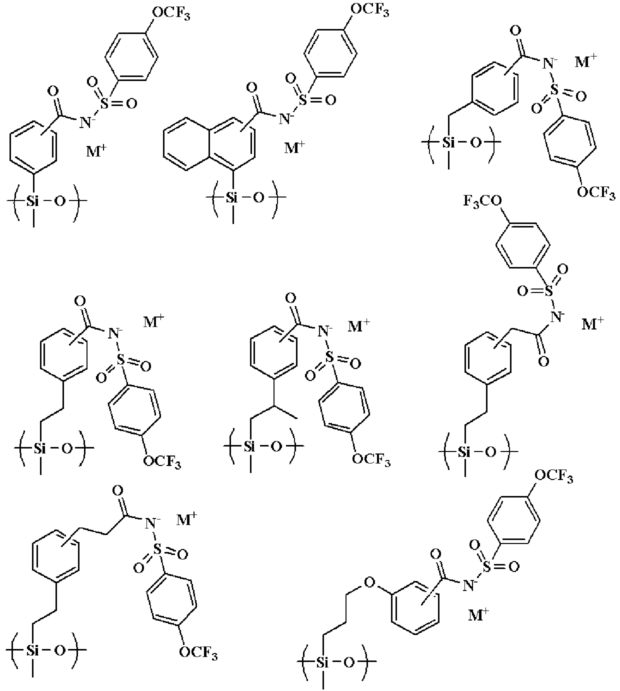 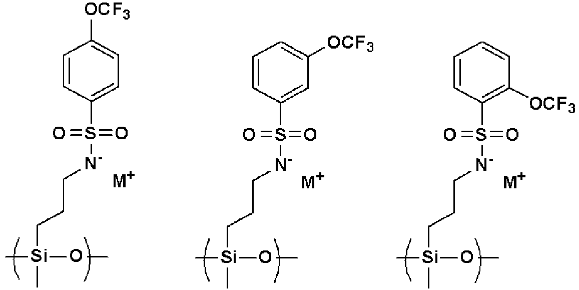 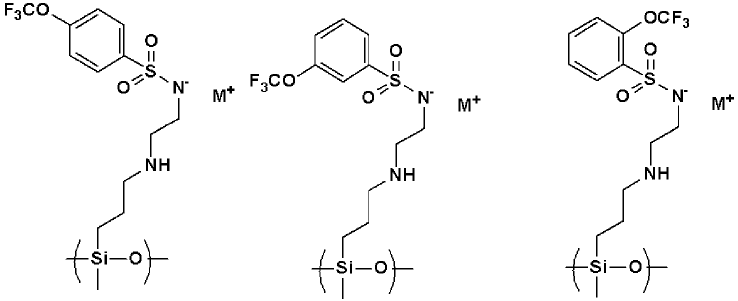 Here, M⁺ is the same are as defined above.

### (Repeating Unit-b2)

In addition to the above repeating unit a2-1, the component (A) of the inventive bio-electrode composition can have a repeating unit-b2 having a glyme chain in order to enhance the electric conductivity. Specific examples of the repeating unit-b2 can include those disclosed in paragraphs [0068] and [0069] of JP 2020-851 A.

### (Repeating Unit-c2)

In addition to the above repeating units-a2-1 and - b2, the component (A) of the inventive bio-electrode composition may have a repeating unit-c2 having a hydrogen atom, an alkyl group, or an aryl group in order to improve mixing property with the component (B) resin. The alkyl group or aryl group may have a hydroxy group, a halogen atom, an ester group, an ether group, a carboxyl group, a thiol group, a (meth)acrylic group, a cyano group, or a nitro group. The specific example of the repeating unit-c2 can include those disclosed in paragraph [0071] in JP 2020-851 A.

As a method for synthesizing the silicone compound having the unit a2-1 of the component (A), the compound can be obtained by a hydrosilylation reaction between a sulfonamide salt having a double bond and a silicone compound having an Si-H group in the presence of a platinum catalyst. The repeating unit -b2 can be obtained by a hydrosilylation reaction between a compound having a double bond and an ether group and a silicone compound having an Si-H group in the presence of a platinum catalyst. Both of the repeating unit a2-1 and the repeating unit b2 or c2 may be included in one silicone molecule. Alternatively, a silicone compound having the repeating unit a2-1 and a silicone compound having the repeating unit b2 or c2 may be blended. When the a2-1 unit is copolymerized with the units b2 and c2, the copolymerization ratio satisfies 0<a2-1≤1.0, 0≤b2<1.0, and 0≤c2<1.0.

The silicone compound before the hydrosilylation reaction may be in a chain shape, a branched shape, or a cyclic shape, but it is preferably a polymer compound having a molecular weight of 300 or more and 1000000 or less. Incorporating the sulfonamide salt in the form of a pendant into the polymeric silicone prevents the salt from permeating the skin and causing an allergy.

In addition, as the component (A), the silicone compound which has a sulfonamide salt at the terminal and is represented by the formulae (2)-2-2 and (2)-2-3 can also be obtained by a hydrosilylation reaction between a sulfonamide salt having a double bond and a silicone compound having a Si-H group at the terminal in the presence of a platinum catalyst. The conditions of the reaction are as described above.

Specific example of the terminal portions of the silicone compounds represented by the above general formulae (2)-2-2 and (2)-2-3 are as follows. 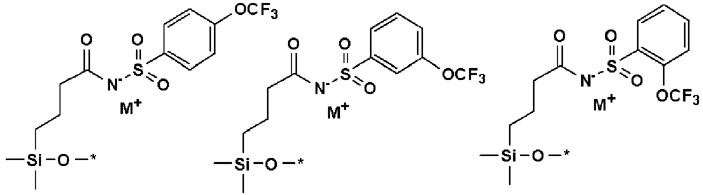 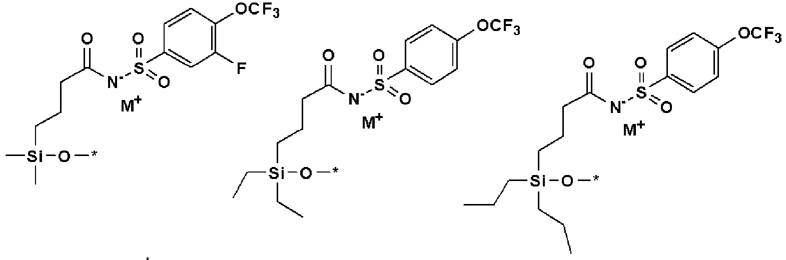 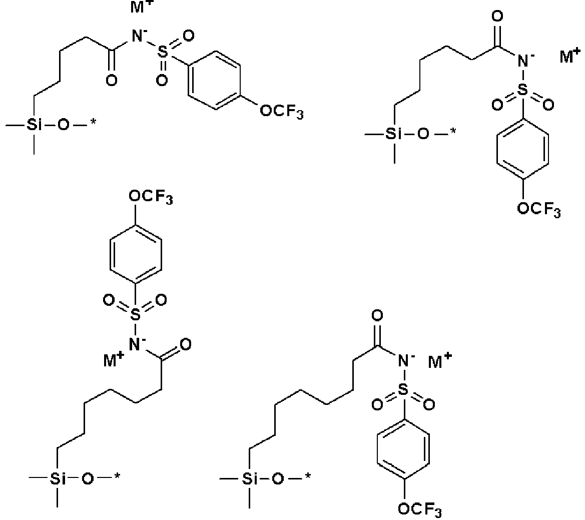 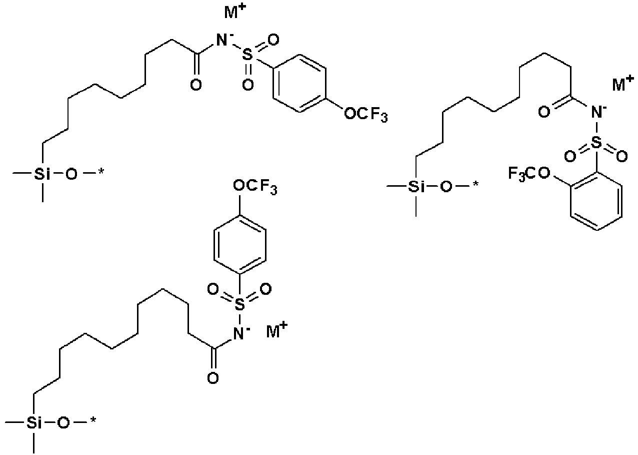 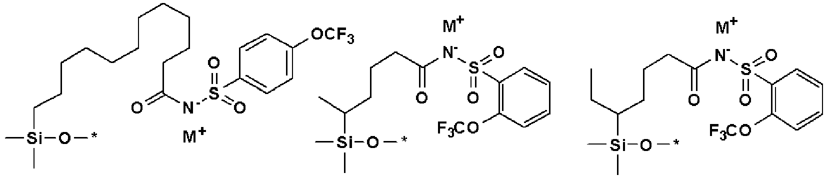 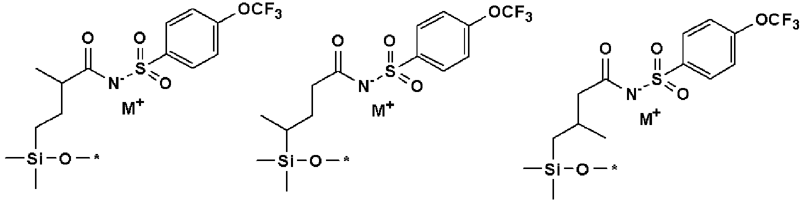 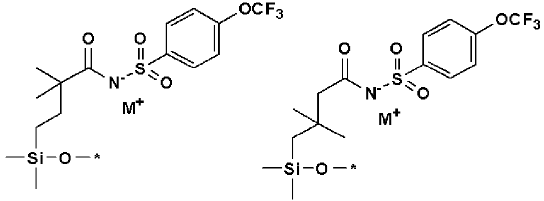 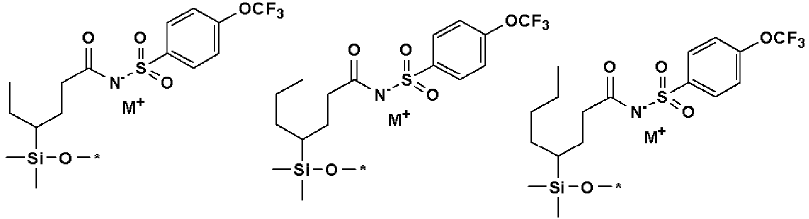 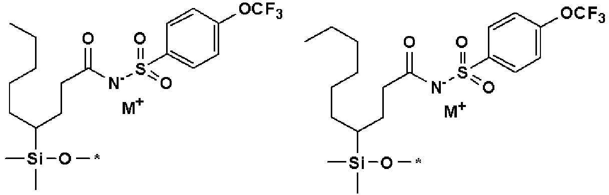 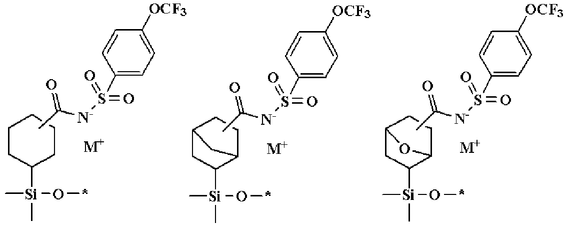 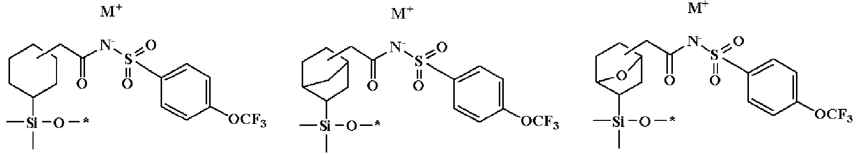 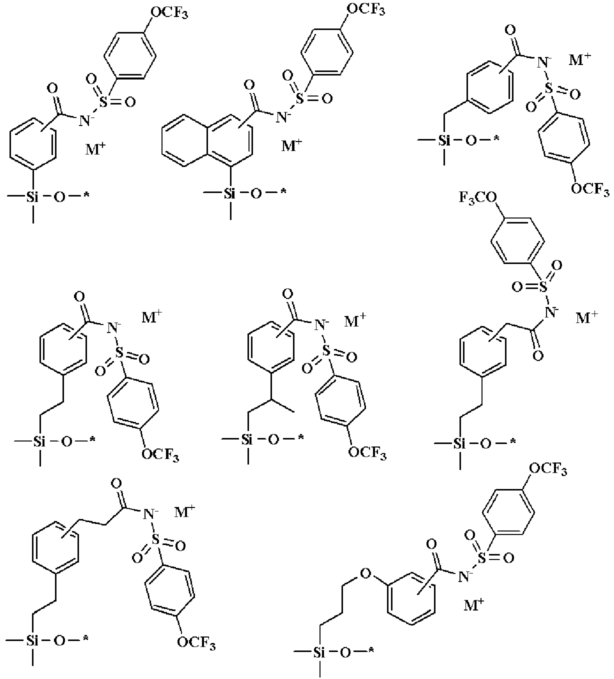 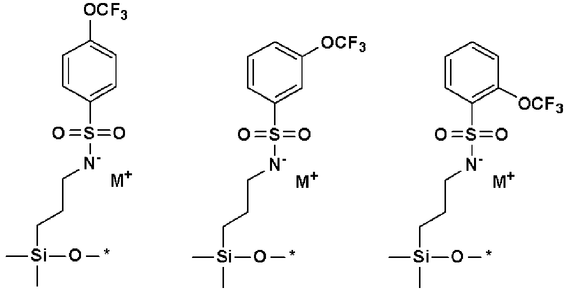 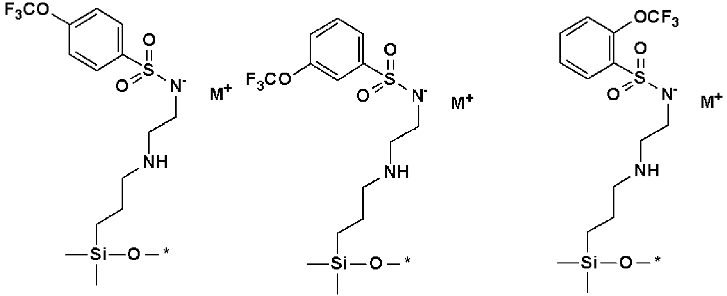 Here, * represents a bonding site to a silicone polymer, and M⁺ is as defined above.

The component (A) may be a silsesquioxane having a three-dimensional ladder structure containing the repeating unit a2-3 represented by the general formula (2)-2-4. Specific example of the structure of the repeating unit a2-3 can include the following. 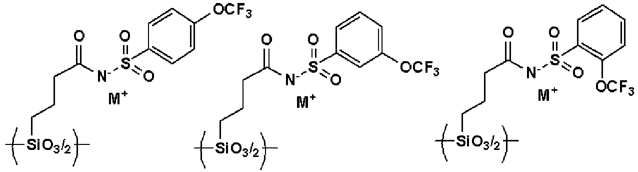 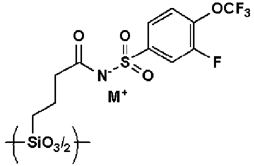 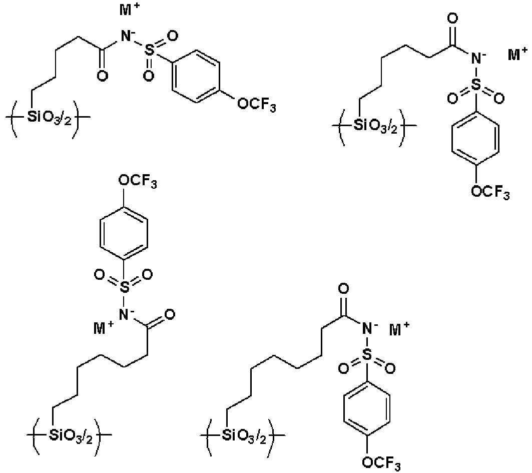 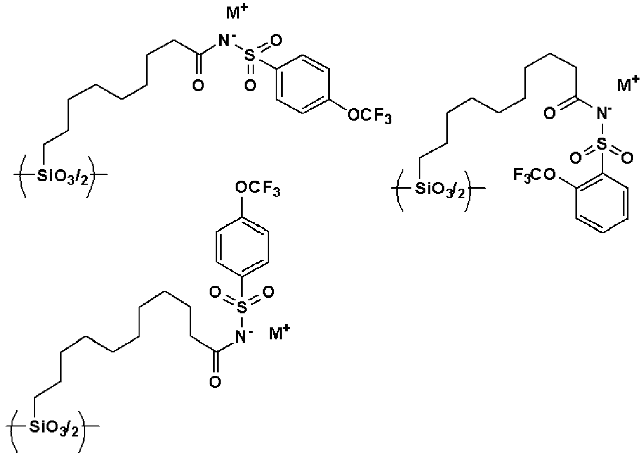 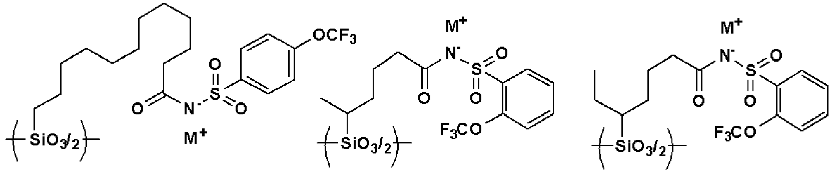 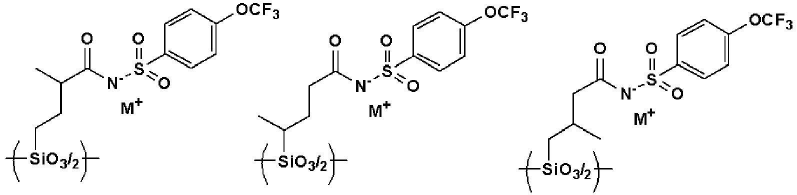 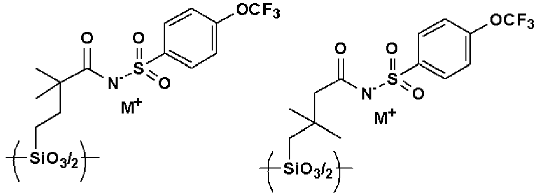 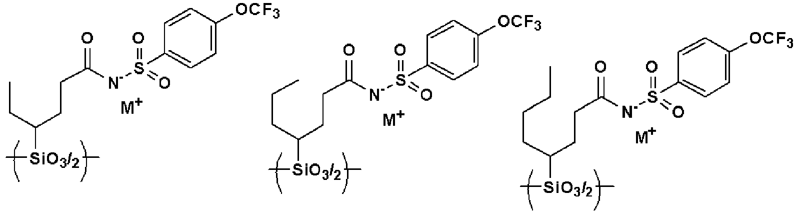 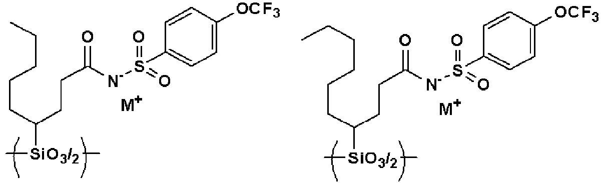 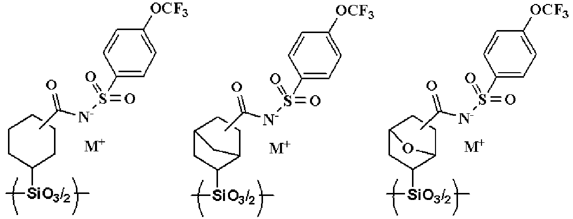 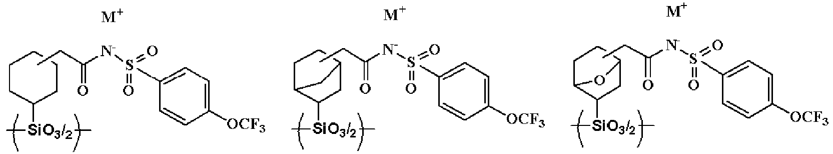 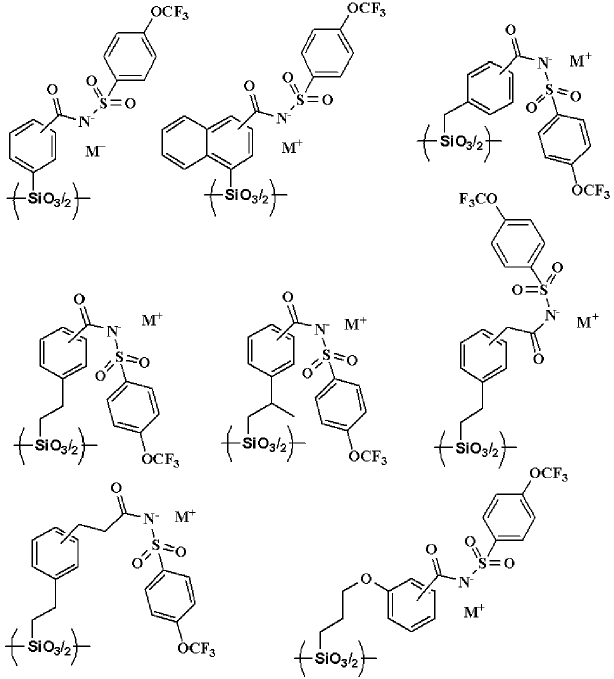 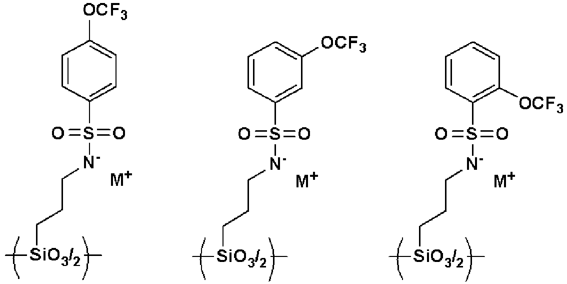 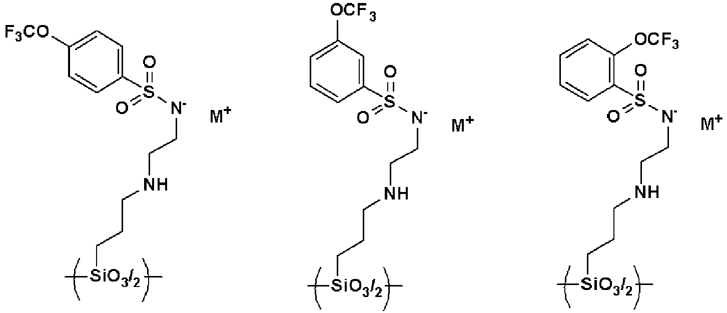 Here, M⁺ is the same are as defined above.

### (Repeating Unit-b3)

In addition to the above repeating unit a2-3, the component (A) of the inventive bio-electrode composition may have a repeating unit-b3 having a glyme chain in order to enhance the electric conductivity. Specific examples of the repeating unit-b3 can include those disclosed in paragraphs [0069] and [0070] of JP 2020-6069 A.

### (Repeating Unit-c3)

In addition to the above repeating units-a2-3 and - b3, the component (A) of the inventive bio-electrode composition may have a repeating unit-c3 having a hydrogen atom, an alkyl group, or an aryl group in order to improve mixing property with the component (B) resin. The alkyl group or aryl group may have a hydroxy group, a halogen atom, an ester group, an ether group, a carboxyl group, a thiol group, a (meth)acrylic group, a cyano group, or a nitro group. The specific example of the repeating unit-c3 can include those disclosed in paragraphs [0072] and [0073] in JP 2020-6069 A.

### [Urethane Resin]

The polyurethane resin having the repeating unit-a3 represented by formulae (2)-3-1 can be obtained by a reaction between a monomer having a dihydroxy group represented by the following general formula (2)-3-1-1 and a compound having an isocyanate group. 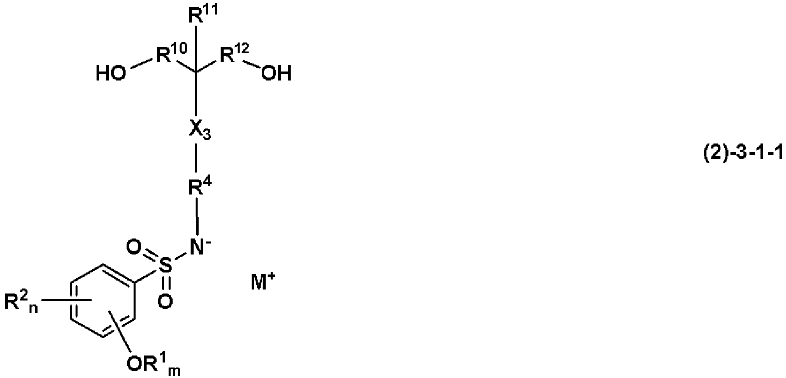 In the above general formula, R¹, R², R⁴, R¹⁰ to R¹², X₃, M⁺, "m", and "n" are as defined above.

Specific example of the monomer having a dihydroxy group represented by the general formula (2)-3-1-1 can include the following.

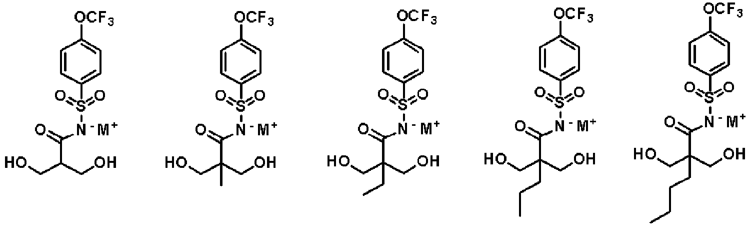

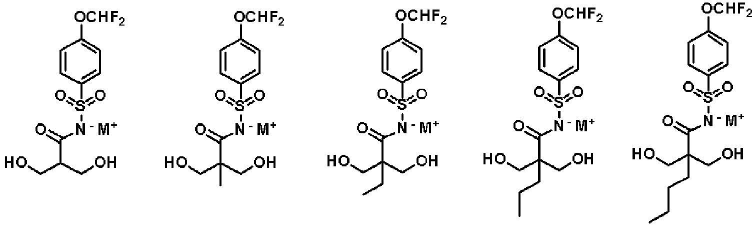

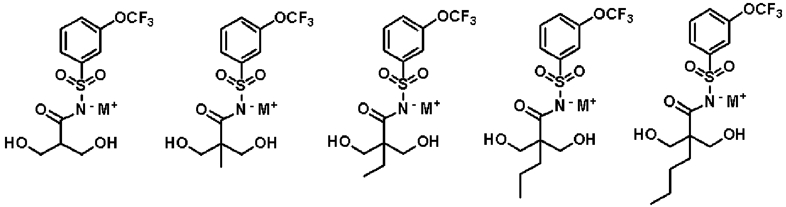

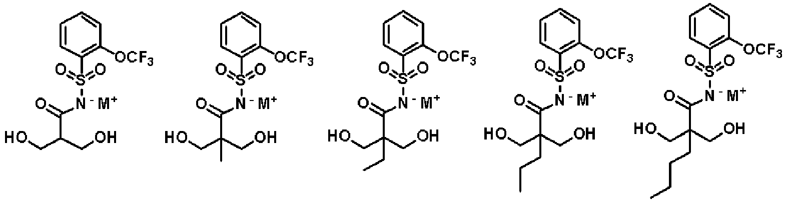

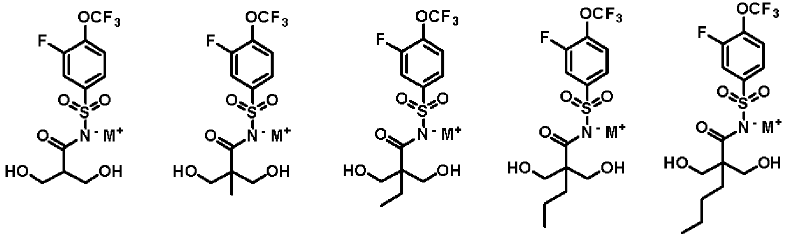

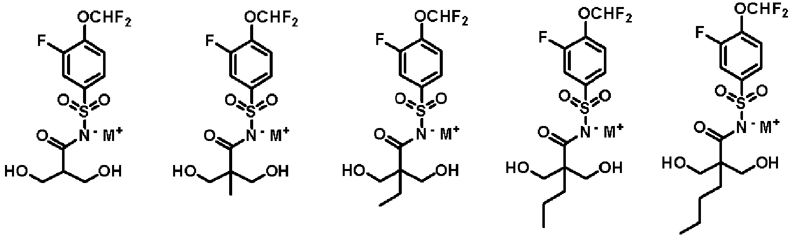

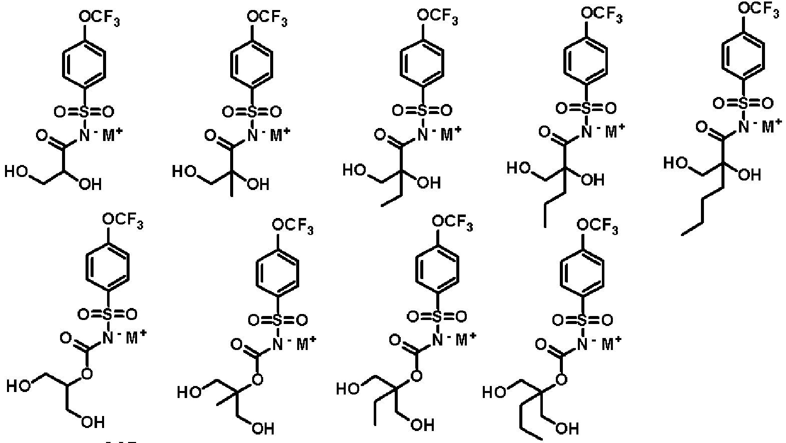

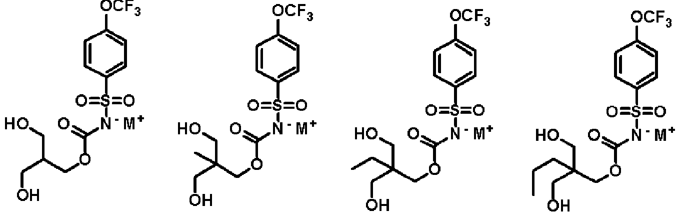

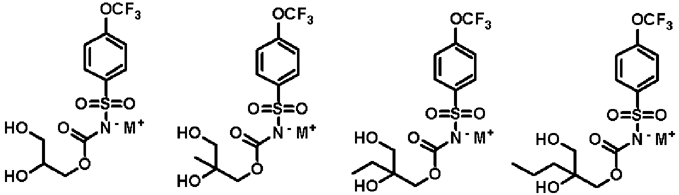

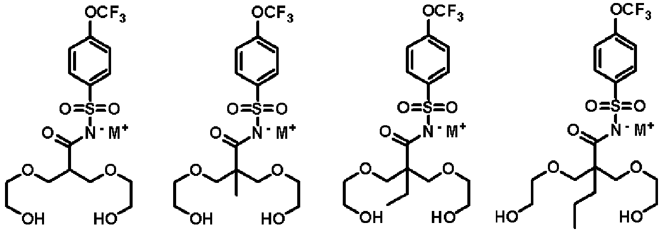

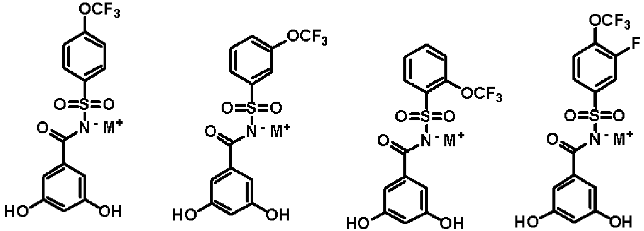

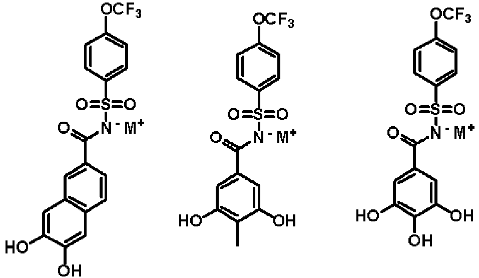

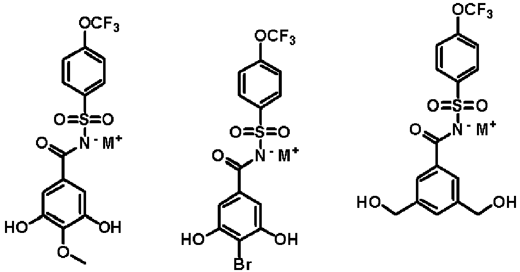

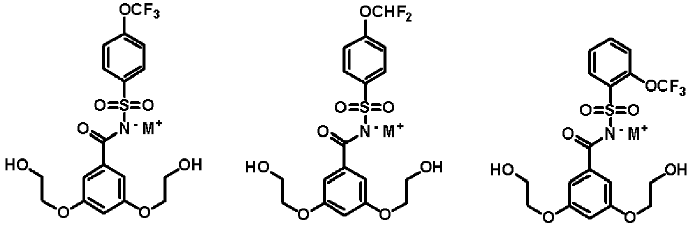

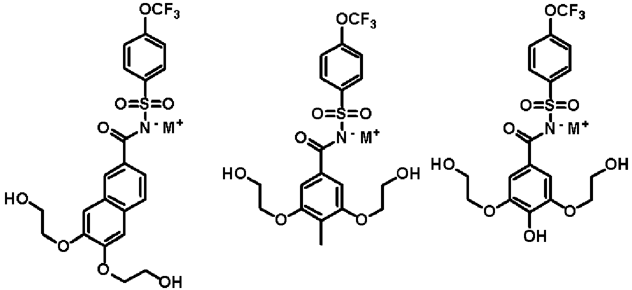

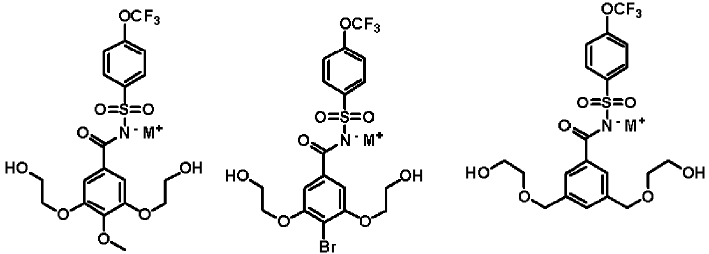

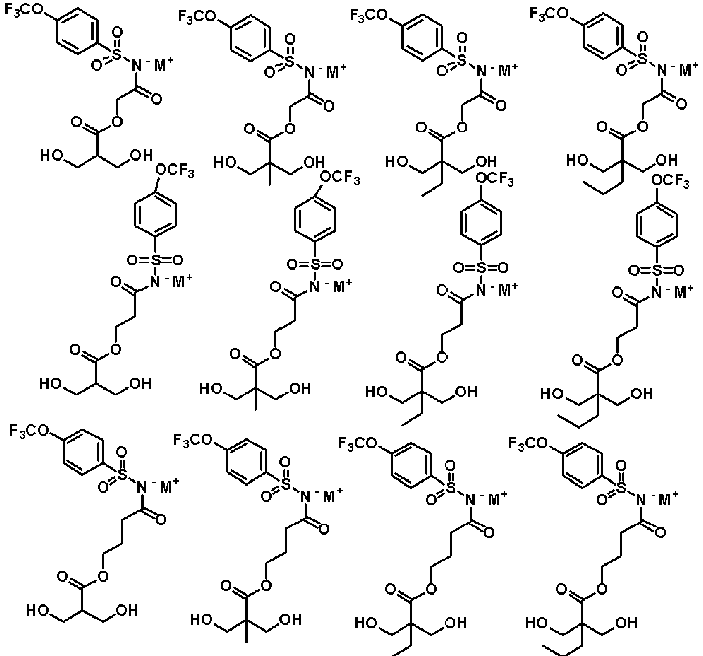

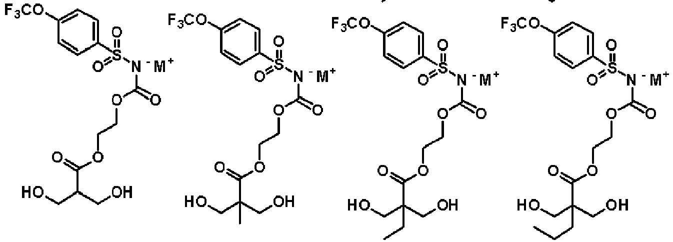

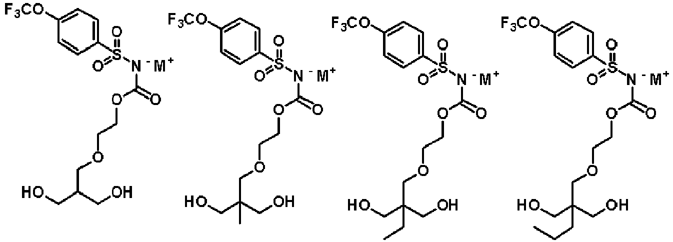

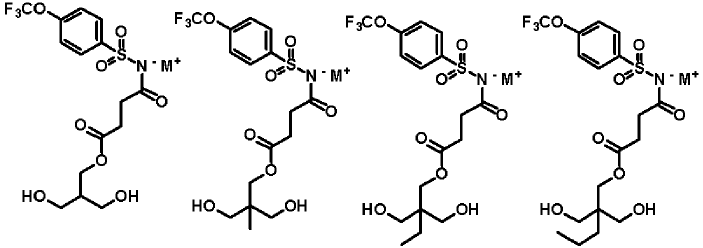

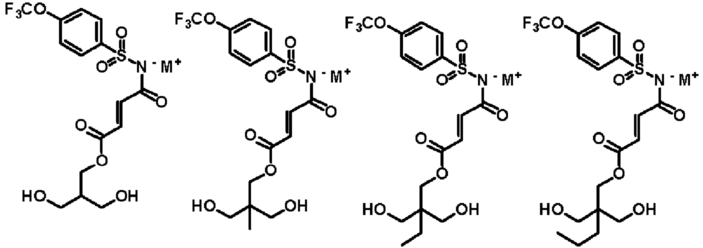

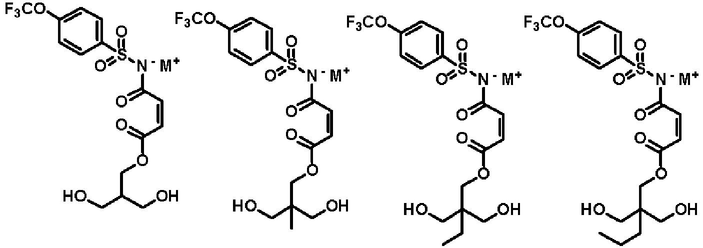

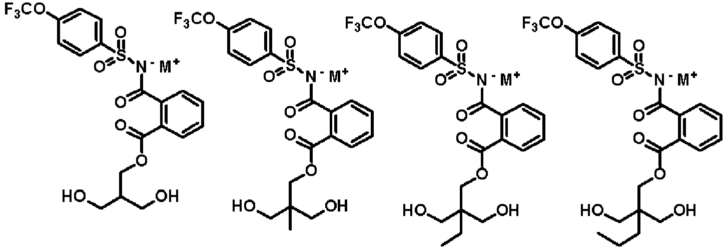

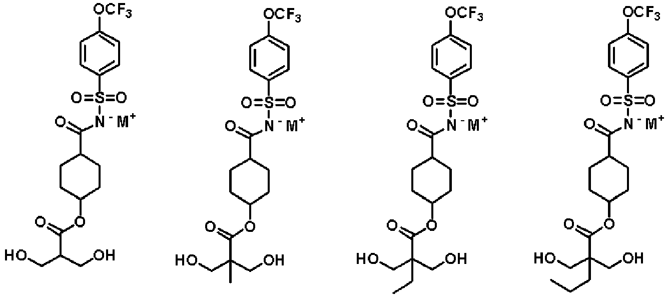

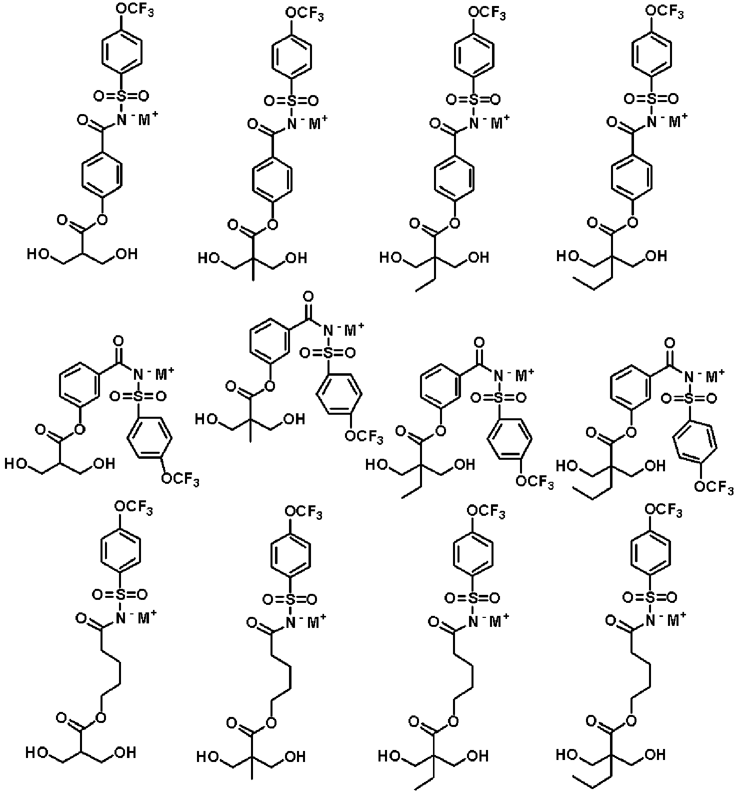

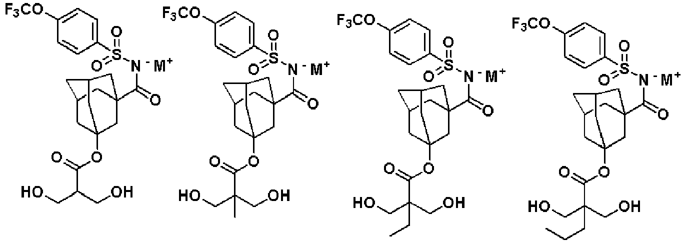

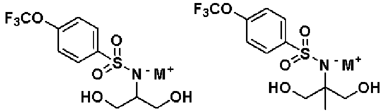

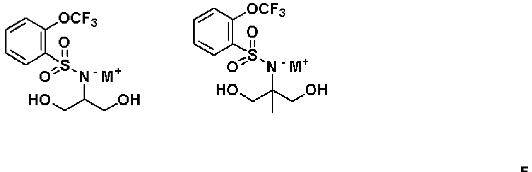

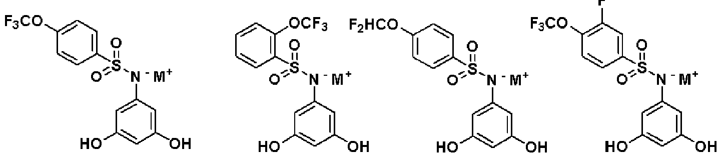

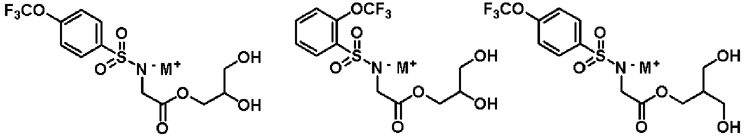

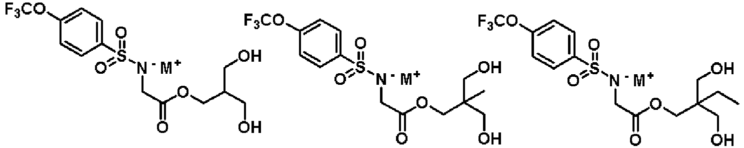

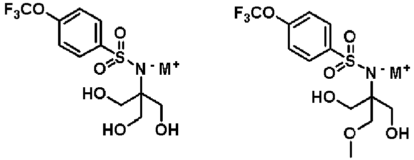

As the synthesis method for the dihydroxy group-containing sulfonamide salt described above, a sulfonamide is synthesized according to the method described in paragraph [0177] of JP 2022-078861 A, and then subjected to neutralization or ion exchange with ammonium hydroxide, ammonium chloride, sodium hydroxide, sodium chloride, potassium hydroxide, potassium chloride, silver hydroxide, silver chloride, silver nitrate, etc to obtain the product.

The resin having the repeating unit -a3 represented by the general formula (2)-3-1 can be obtained by a reaction between a monomer represented by the above general formula (2)-3-1-1 and having a dihydroxy group and a compound having an isocyanate group. Examples of the compound having an isocyanate group can include those disclosed in the paragraph [0067] and [0068] of JP 2020-55300 A.

Since isocyanate compounds have high reactivity with hydroxyl group-containing compounds, it may be difficult to control this. Further, since isocyanate compounds may react with moisture in the atmosphere during storage and the isocyanate groups may be deactivated, sufficient care must be taken during storage, such as sufficiently preventing humidity. Therefore, in order to prevent these events, a compound having a blocked isocyanate group in which the isocyanate group is protected with a substituent group is sometimes used.

The blocked isocyanate group becomes an isocyanate group after the blocked group is deprotected by heating, and the specific examples include an isocyanate group substituted with alcohols, phenols, thioalcohols, imines, ketimines, amines, lactams, pyrazoles, oximes, β-diketones, etc.

In order to lower the deprotection temperature of the blocked isocyanate group, it is possible to add a catalyst. The catalysts known for this includes organic tins such as dibutyltin dilaurate; bismuth salts; and zinc carboxylates such as zinc 2-ethylhexanoate and zinc acetate.

Especially, JP 2012-152725 A discloses that the deprotection temperature can be lowered by containing α,β-unsaturated zinc carboxylate as a carboxylic acid and as a blocked isocyanate dissociation catalyst.

It is also possible to copolymerize with a compound having a hydroxy group other than the monomer represented by the general formula (2)-3-1-1. Example of the compounds having a hydroxy group other than the monomer represented by the general formula (2)-3-1-1 include those disclosed in paragraphs [0054] to [0064] and [0077] to [0082] of JP 2020-55300 A.

In polyurethane polymerization, in order to promote a formation reaction of urethane bonding, a catalyst is preferably added as the occasion demands.

The urethanization catalyst can be selected from publically known catalysts accordingly, and examples thereof include amine catalysts, ammonium salt catalysts, potassium salt catalysts, organometallic catalysts, etc.

Examples of amine catalysts include triethylamine, N,N-dimethylcyclohexylamine, triethylenediamine, 2-methyltriethylenediamine, N,N,N',N'-tetramethylethylenediamine, N,N,N',N'-tetramethylpropylenediamine, N,N,N',N",N"-pentamethyldiethylenetriamine, N,N,N',N",N"-pentamethyl-(3-aminopropyl)ethylenediamine, N,N,N',N",N"-pentamethyldipropylenetriamine, N,N,N',N'-tetramethylhexamethylenediamine, bis(2-dimethylaminoethyl)ether, dimethylethanolamine, dimethylisopropanolamine, dimethylaminoethoxyethanol, N,N-dimethylhexanolamine, N,N-dimethyl-N'-(2-hydroxyethyl) ethylenediamine, N,N-dimethyl-N'-(2-hydroxyethyl)propanediamine, N,N,N'-trimethylaminoethylethanolamine, bis(dimethylaminopropyl)amine, bis(dimethylaminopropyl)isopropanolamine, N-methyl-N'-(2-dimethylaminoethyl)piperazine, N-methyl-N'-(2-hydroxyethyl)piperazine, N-methylmorpholine, N-ethylmorpholine, 1-methylimidazole, 1,2-dimethylimidazole, 1-isobutyl-2-methylimidazole, 1-dimethylaminopropylimidazole, 1-(2-hydroxyethyl)imidazole, 1-(2-hydroxypropyl)imidazole, 1-(2-hydroxyethyl)-2-methylimidazole, 1-(2-hydroxypropyl)-2-methylimidazole, etc.

Examples of ammonium salt catalysts include: quaternary ammonium salts such as tetraethylhydroxylammonium; ammonium salts of 1,8-diazabicyclo(5,4,0)-undecene-7 or 1,5-diazabicyclo(4,3,0)-nonene-5, formed with octylic acid, olein acid, p-toluenesulfonic acid, formic acid, phenolic acid, orthophthalic acid, acetic acid, maleic acid, or boric acid; etc. acid.

Examples of potassium salt catalysts include potassium carbonate, potassium acetate, potassium octylate, etc.

Examples of organometallic catalysts include: organic tin compounds such as tin acetate, tin octylate (tin 2-ethylhexanoate), tin oleate, tin laurate, dibutyltin diacetate, dimethyltin dilaurate, dibutyltin dilaurate, dibutyltin dimercaptide, dibutyltin maleate, dibutyltin dineodecanoate, dioctyltin dimercaptide, dioctyltin dilaurylate, and dibutyltin dichloride; organic lead compounds such as lead octoate, lead naphthenat; organic nickel compounds such as nickel naphthenate; organic cobalt compounds such as cobalt naphthenate; organocopper compounds such as copper octenoate; and organic bismuth compounds such as bismuth octylate, bismuth neodecanoate.

One of these urethanization catalyst may be used alone or two or more thereof may be used in combination. The amount of urethanization catalyst used is preferably 0 parts by mass or more and 5 parts by mass or less, more preferably 0.1 parts by mass or more and 2 parts by mass or less relative to 100 parts by mass of entire polyurethane components.

The component (A) may also be a resin having a sulfonamide salt at the end by bonding to a urethane group represented by the general formula (2)-3-2. This resin can be obtained by a reaction between a monomer having a hydroxy group represented by the following general formula (2)-3-2-1 and a compound having an isocyanate group. 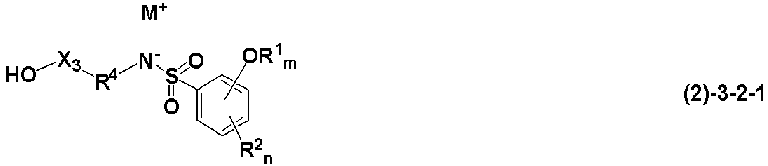 In the above general formula, R¹, R², R⁴, X₃, M⁺, "m", and "n" are as defined above.

Examples of the compound having an isocyanate group include the above-mentioned compounds or a polyurethane compound having an isocyanate group at its terminal and obtained by reacting a compound having an isocyanate group and a compound having a dihydroxy group. The conditions of the reaction are as described above.

The blended amount of the component (A) in the inventive bio-electrode composition is not particularly limited, but, for example, the blended amount of the component (A) is preferably 1 to 95 parts by mass, more preferably 1 to 50 parts by mass based on 100 parts by mass of the total composition. In the inventive bio-electrode composition, the blended amount of the component (A) is preferably 0.1 to 300 parts by mass, and more preferably 1 to 200 parts by mass, with respect to 100 parts by mass of the component (B) described later. Furthermore, the component (A) may be used alone or two or more thereof may be used in combination.

### [(B) Resin]

The resin (B) contained in the inventive bio-electrode composition is a component that is compatible with the above component (A) ionic resin to prevent elution of the salt. This component also serves to hold an electric conductivity improver such as a metal powder, a carbon powder, a silicon powder, and a lithium titanate powder, and enhance adhesiveness further. When the component (A) ionic resin has sufficient adhesiveness, the resin (B) is not necessarily needed. Note that, the resin may be any resin other than the above-described component (A), and is preferably either or both of a thermosetting resin and a photo-curable resin, is particularly preferably one or more resins selected from the group consisting of silicone-based, acrylic-based, and urethane-based resins.

Examples of the adhesive silicone-based resin include an addition reaction-curable type and a radical crosslinking reaction-curable type. As the addition reaction-curable type, for example, it is possible to use one that contains diorganosiloxane having an alkenyl group(s), an MQ resin having a R₃SiO_{0.5} unit and an SiO₂ unit, organohydrogenpolysiloxane having multiple SiH groups, a platinum catalyst, an addition-reaction inhibitor, and an organic solvent, as disclosed in JP 2015-193803 A. As the radical crosslinking reaction-curable type, for example, it is possible to use one that contains diorganopolysiloxane with or without an alkenyl group, an MQ resin having an R₃SiO_{0.5} unit and an SiO₂ unit, organic peroxide, and an organic solvent, as disclosed in JP 2015-193803 A. Here, R represents a substituted or unsubstituted monovalent hydrocarbon group having 1 to 10 carbon atoms.

It is also possible to use a polysiloxane-resin integrated compound that is formed by condensation reaction of an MQ resin and polysiloxane having silanol at the terminal or the side chain of the polymer. The MQ resin contains many silanols and adding the MQ resin improves adhesive strength. However, the MQ resin is not crosslinkable, and thus does not bind to the polysiloxane in molecular level. As described above, integrating the polysiloxane and the resin can increase the adhesive strength.

Further, the silicone-based resin may contain modified siloxane that has a group selected from the group consisting of an amino group, an oxirane group, an oxetane group, a polyether group, a hydroxy group, a carboxyl group, a mercapto group, a methacryl group, an acryl group, a phenol group, a silanol group, a carboxylic anhydride group, an aryl group, an aralkyl group, an amide group, an ester group, and a lactone ring. The addition of the modified siloxane improves dispersibility of the component (A) in the silicone resin. The modified siloxane may be modified at any part such as one terminal, both terminals, or a side chain of the siloxane.

As the adherent acrylic-based resin, for example, it is possible to use one having a hydrophilic (meth)acrylic ester and hydrophobic long chain (meth)acrylic ester as the repeating units disclosed in JP 2016-011338 A. In some cases, it is also possible to copolymerize a (meth)acrylic ester having a functional group or a (meth)acrylic ester having a siloxane bond.

As the adherent urethane-based resin, for example, it is possible to use one having a urethane bond with a polyether bond, a polyester bond, a polycarbonate bond, or a siloxane bond, which is disclosed in JP 2016-065238 A.

In the inventive bio-electrode composition, the component (B) resin preferably has high compatibility with the component (A) to prevent lowering of the electric conductivity due to separation of the component (A) from the living body contact layer. Further, in the inventive bio-electrode composition, the component (B) resin preferably has high adhesiveness to the conductive substrate to prevent peeling of the living body contact layer from the conductive substrate. In order to have higher compatibility with the conductive substrate and salts, a use of a resin with high polarity is effective. Examples of such a resin include resin having one or more moieties selected from the group of an ether bond, an ester bond, an amide bond, an imide bond, a urethane bond, a thiourethane bond, and a thiol group, a polyacrylic resin, a polyamide resin, a polyimide resin, a polyurethane resin, a polythiourethane resin; etc. On the other hand, the living body contact layer is to be contacted with a living body, thereby being susceptible to perspiration from the living body. Accordingly, in the inventive bio-electrode composition, it is preferable the component (B) resin has high repellency and difficulty in being hydrolyzed. To make the component (B) resin highly repellent and difficult in being hydrolyzed, the use of a silicon-containing resin is effective.

The silicon atom-containing polyacrylic resin includes a polymer that has a silicone in the main chain and a polymer that has a silicon atom(s) on the side chain, and either thereof can be suitably used. As the polymer having a silicone in the main chain, it is possible to use siloxane or silsesquioxane, or the like having a (meth)acrylpropyl group. In this case, addition of a photoradical generator allows the (meth)acryl moiety to polymerize to cure.

As the silicon atom-containing polyamide resin, it is possible to use suitably polyamide silicone resins disclosed in JP 2011-079946 A and US 5981680 A, for example. Such polyamide silicone resins can be synthesized, for example, by combining a silicone or non-silicone compound having amino groups at both terminals and a non-silicone or silicone compound having carboxyl groups at both terminals.

It is also possible to use polyamic acid before cyclization thereof, which is obtained by reacting carboxylic anhydride and amine. The carboxyl group of the polyamic acid may be crosslinked by using a crosslinking agent such as an epoxy type and an oxetane type. It is also possible to esterify the carboxyl group with hydroxyethyl (meth)acrylate to perform photoradical crosslinking of the (meth)acrylate moiety.

As the silicon atom-containing polyimide resin, it is possible to suitably use polyimide silicone resins and the like, which are disclosed in JP 2002-332305 A, for example. Although polyimide resins have very high viscosity, the viscosity can be decreased to be low by blending a (meth)acrylic monomer as a solvent and a crosslinking agent.

Examples of the silicon atom-containing polyurethane resin can include polyurethane silicone resins. Such polyurethane silicone resins can be crosslinked through urethane bond by blending a compound having isocyanate groups at both terminals and a compound having a hydroxy group(s) at the terminal(s) and then heating the mixture. In this case, a silicon atom(s) (siloxane bond) has to be contained in either or both of the compound having isocyanate groups at both terminals and the compound having a hydroxy group(s) at the terminal(s). Alternatively, polysiloxane and a urethane (meth)acrylate monomer can be blended and photo-crosslinked as disclosed in JP 2005-320418 A. It is also possible to photo-crosslink a polymer having both of a siloxane bond(s) and a urethane bond(s), with the terminal having a (meth)acrylate group(s). Particularly, a material having a polyurethane in the main chain and with a silicone chain on a side chain, which is disclosed in JP 2018-123304 A and JP 2019-70109 A, has properties of high strength and high stretchability, and thus is preferable.

The silicon atom-containing polythiourethane resin can be obtained by reaction between a compound having a thiol group(s) and a compound having an isocyanate group(s), provided that at least one of them contains a silicon atom(s). When (meth)acrylate groups are contained at the terminals, photo-curing is also possible.

The silicone-based resin can be improved in compatibility with the foregoing salt by adding modified siloxane that has a functional group selected from the group consisting of an amino group, an oxirane group, an oxetane group, a polyether group, a hydroxy group, a carboxyl group, a mercapto group, a methacryl group, an acryl group, a phenol group, a silanol group, a carboxylic anhydride group, an aryl group, an aralkyl group, an amide group, an ester group, and a lactone ring, in addition to the diorganosiloxane having the above alkenyl group(s), the MQ resin having an R₃SiO_{0.5} unit and an SiO₂ unit, and the organohydrogenpolysiloxane having multiple SiH groups.

The diorganosiloxane having an alkenyl group(s) and the organohydrogenpolysiloxane having multiple SiH groups can be crosslinked through an addition reaction with a platinum catalyst.

Examples of the platinum catalyst include platinum-based catalysts such as chloroplatinic acid, a alcohol solution of chloroplatinic acid, a reaction product of chloroplatinic acid and alcohol, a reaction product of chloroplatinic acid and an olefin compound, a reaction product of chloroplatinic acid and vinyl group-containing siloxane, a platinum-olefin complex, and a complex of platinum and vinyl group-containing siloxane; platinum group metal-based catalysts such as a rhodium complex and a ruthenium complex; etc. Further, these catalysts may be used after dissolved or dispersed in an alcoholic solvent, a hydrocarbon solvent, or a siloxane-based solvent.

Note that, the platinum catalyst is contained in an amount within preferably 5 to 2,000 ppm, particularly preferably 10 to 500 ppm, based on 100 parts by mass of the resin of components (A) and (B) combined.

In the inventive bio-electrode composition, the component (B) is preferably contained in an amount of 0 to 2000 parts by mass, more preferably 10 to 1000 parts by mass based on 100 parts by mass of the component (A) ionic resin. For each of components (A) and (B), one kind may be used alone, or two or more kinds thereof may be used in mixture.

When the addition-curable silicone resin is used, an addition-reaction inhibitor may be added. This addition-reaction inhibitor is added as a quencher to prevent the action of the platinum catalyst in the solution and under a low temperature circumstance after forming the coating film and before heat curing. Specific examples of the addition-reaction inhibitor include 3-methyl-1-butyn-3-ol, 3-methyl-1-pentyn-3-ol, 3,5-dimethyl-1-hexyn-3-ol, 1-ethynylcyclohexanol, 3-methyl-3-trimethylsiloxy-1-butyne, 3-methyl-3-trimethylsiloxy-1-pentyne, 3,5-dimethyl-3-trimethylsiloxy-1-hexyne, 1-ethynyl-1-trimethylsiloxycyclohexane, bis(2,2-dimethyl-3-butynoxy)dimethylsilane, 1,3,5,7-tetramethyl-1,3,5,7-tetravinylcyclotetrasiloxane, 1,1,3,3-tetramethyl-1,3-divinyldisiloxane, etc.

The addition-reaction inhibitor is added in an amount preferably within 0 to 10 parts by mass, particularly preferably 0.05 to 3 parts by mass, based on 100 parts by mass of the resin of the components (A) and (B) combined.

Further, as the component (B), a silicone resin having RₓSiO_{(4-x)/2} unit and SiO₂ unit is preferably contained, wherein R is a substituted or unsubstituted monovalent hydrocarbon group having 1 to 10 carbon atoms and x is in a range of 2.5 to 3.5.

When the component (B) has a double bond capable of radical crosslinking, it is effective to add a radical generator. Examples of radical generators include photoradical generators and thermal-radical generators.

Examples of the photoradical generator can include acetophenone, 4,4'-dimethoxybenzyl, benzyl, benzoin, benzophenone, 2-benzoylbenzoic acid, 4,4'-bis(dimethylamino)benzophenone, 4,4'-bis(diethylamino)benzophenone, benzoin methyl ether, benzoin ethyl ether, benzoin isopropyl ether, benzoin butyl ether, benzoin isobutyl ether, 4-benzoylbenzoic acid, 2,2'-bis(2-chlorophenyl)-4,4',5,5'-tetraphenyl-1,2'-biimidazole, methyl 2-benzoylbenzoate, 2-(1,3-benzodioxole-5-yl)-4,6-bis(trichloromethyl)-1,3,5-triazine, 2-benzyl-2-(dimethylamino)-4'-morpholinobutyrophenone, 4,4'-dichlorobenzophenone, 2,2-diethoxyacetophenone, 2,2-dimethoxy-2-phenylacetophenone, 2,4-diethylthioxanthene-9-one, diphenyl(2,4,6-trimethylbenzoyl)phosphine oxide (BAPO), 1,4-dibenzoylbenzene, 2-ethylanthraquinone, 1-hydroxycyclohexyl phenyl ketone, 2-hydroxy-2-methylpropiophenone, 2-hydroxy-4'-(2-hydroxyethoxy)-2-methylpropiophenone, 2-isonitrosopropiophenone, and 2-phenyl-2-(p-toluenesulfonyloxy)acetophenone.

The curing can also be performed by adding a radical generator of a heat decomposition type. Examples of the thermal-radical generator can include 2,2'-azobis(isobutyronitrile), 2,2'-azobis(2,4-dimethylvaleronitrile), 2,2'-azobis(2-methylbutyronitrile), 2,2'-azobis(methylpropionamidine) hydrochloride, 2,2'-azobis[2-(2-imidazolin-2-yl)propane] hydrochloride, 2,2'-azobis(4-methoxy-2,4-dimethylvaleronitrile), 2,2'-azobis(2-methylbutyronitrile), 2,2'-azobis(cyclohexane-1-carbonitrile), 1[(1-cyano-1-methylethyl)azo]formamide, 2,2'-azobis[2-methyl-N-(2-hydroxyethyl)propionamide], 2,2'-azobis[N-(2-propenyl)-2-methylpropionamide], 2,2'-azobis(N-butyl-2-methylpropionamide), dimethyl-2,2'-azobis(isobutylate), 4,4'-azobis(4-cyanopentanoic acid), dimethyl-2,2'-azobis(2-methylpropionate), benzoyl peroxide, tert-butyl hydroperoxide, cumene hydroperoxide, di-tert-butyl peroxide, di-tert-amyl peroxide, di-n-butyl peroxide, dicumyl peroxide, etc.

Note that, the radical generator is preferably contained in an amount of 0.1 to 50 parts by mass based on 100 parts by mass of the resin of components (A) and (B) combined.

As will be described later, the living body contact layer is a cured product of the bio-electrode composition. The curing makes the suitable adhesion of the living body contact layer to both of the skin and the conductive substrate. The curing means is not particularly limited, and common means, for example, such as crosslinking reaction by either or both of heat and light, or with an acid catalyst or a base catalyst can be used. The crosslinking reaction can be performed, for example, by appropriately selecting methods described in "Kakyou han-nou handbook (handbook of crosslinking reaction)", Chapter 2, pages 51-371, Yasuharu Nakayama, Maruzen Publishing Co., Ltd. (2013).

### [Ionic Polymer]

The inventive bio-electrode composition may be added with an ionic polymer other than the component (A). As the ionic polymer, those disclosed in JP 2018-126496 A and JP 2018-130533 A are preferably used.
The ionic polymer is preferably added in an amount within a range of 0.1 to 100 parts by mass based on 100 parts by mass of the resin of the components (A) and (B) combined.

### [Metal Powder]

The inventive bio-electrode composition may contain a metal powder selected from the group consisting of gold, silver, platinum, copper, tin, titanium, nickel, aluminum, tungsten, molybdenum, ruthenium, chromium, and indium in order to improve electron conductivity. The metal powder or the carbon powder is added in an amount preferably within a range of 1 to 50 parts by mass based on 100 parts by mass of the resin of the components (A) and (B) combined.

As the kind of the metal powder, gold, silver, and platinum are preferable from the viewpoint of electric conductivity; and silver, copper, tin, titanium, nickel, aluminum, tungsten, molybdenum, ruthenium, and chromium are preferable from the viewpoint of cost. From the viewpoint of biocompatibility, noble metals are preferable. From comprehensive viewpoint including the above, silver is most preferable.

The shape of the metal powder may include a spherical shape, a disk shape, a flaky shape, and a needle shape. The addition of flaky powder brings highest electric conductivity and is thus preferable. The metal powder is preferably a flake having relatively lower density and larger specific surface area, with a size of 100 µm or less, a tapped density of not more than 5 g/cm³, and a specific surface area of not less than 0.5 m²/g.

### [Carbon Material]

A carbon material can be contained as an electric conductivity improver. Examples of the carbon material can include carbon black, graphite, carbon nanotube, carbon fiber, graphene, etc. The carbon nanotube may be either single layer or multilayer, and the surface may be modified with an organic group(s). The carbon material is preferably contained in an amount range of 1 to 50 parts by mass based on 100 parts by mass of the resin of the components (A) and (B) combined.

### [Silicon Powder]

The inventive bio-electrode composition may contain a silicon powder to enhance ion reception sensitivity. Examples of the silicon powder can include powders of silicon, silicon monoxide, or silicon carbide. The particle diameter of the powder is preferably smaller than 100 µm, more preferably 1 µm or less. Since finer particles have a larger surface area, the resulting bio-electrode can receive a larger number of ions and has higher sensitivity. The silicon powder is added preferably in an amount within a range of 1 to 50 parts by mass based on 100 parts by mass of the resin of the components (A) and (B) combined.

### [Lithium Titanate Powder]

The inventive bio-electrode composition may contain a lithium titanate powder to enhance ion reception sensitivity. Examples of the lithium titanate powder can include those represented by molecular formulae Li₂TiO₃, LiTiO₂, and Li₄Ti₅O₁₂ with a spinel structure. The lithium titanate powder preferably has a spinel structure. It is also possible to use carbon-incorporated lithium titanate particles. The particle diameter of the powder is preferably smaller than 100 µm, more preferably 1 µm or less. Since finer particles have a larger surface area, the bio-electrode can receive a larger number of ions, and has higher sensitivity. These may be composite powders with carbon. The lithium titanate powder is added preferably in an amount within a range of 1 to 50 parts by mass based on 100 parts by mass of the resin of the components (A) and (B) combined.

### [Crosslinking Agent]

The inventive bio-electrode composition may contain an epoxy-type crosslinking agent. This crosslinking agent is a compound having multiple epoxy groups or oxetane groups in one molecule. The amount of the crosslinking agent added is preferably 1 to 30 parts by mass based on 100 parts by mass of the resin of the components (A) and (B) combined.

### [Crosslinking Catalyst]

The inventive bio-electrode composition may also contain a catalyst for crosslinking the epoxy groups or the oxetane groups. As this catalyst, those disclosed in paragraphs [0027] to [0029] of JP 2019-503406 A can be used. The amount of the catalyst added is preferably 0.01 to 10 parts by mass based on 100 parts by mass of the resin of the components (A) and (B) combined.

### [Ionic Additive]

The inventive bio-electrode composition may contain an ionic additive to enhance the ionic conductivity. In consideration of biocompatibility, examples of the ionic additive can include sodium chloride, potassium chloride, calcium chloride, magnesium chloride, saccharin sodium salt, acesulfame potassium, sodium carboxylate, potassium carboxylate, calcium carboxylate, sodium sulfonate, potassium sulfonate, calcium sulfonate, sodium phosphate, potassium phosphate, calcium phosphate, magnesium phosphate, betaine, and salts disclosed in JP 2018-44147 A, JP 2018-59050 A, JP 2018-59052 A, and JP 2018-130534 A.

### [Organic Solvent]

In addition, the inventive bio-electrode composition may contain an organic solvent as a component (D). Specific examples of the organic solvent can include: aromatic hydrocarbon solvents such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; aliphatic hydrocarbon solvents such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0^{2,7}]undeca-4-ene, n-dodecane, n-tridecane, n-pentadecane, n-hexadecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffins; ketone solvents such as cyclohexanone, cyclopentanone, 2-octanone, 2-nonanone, 2-heptanone, 3-heptanone, 4-heptanone, 2-hexanone, 3-hexanone, diisobutyl ketone, methylcyclohexanone, and methyl n-pentyl ketone; alcohol solvents such as 3-methoxybutanol, 3-methyl-3-methoxybutanol, 1-methoxy-2-propanol, and 1-ethoxy-2-propanol; ether solvents such as propylene glycol monomethyl ether, ethylene glycol monomethyl ether, propylene glycol monoethyl ether, ethylene glycol monoethyl ether, propylene glycol dimethyl ether, diethylene glycol monomethyl ether, diethylene glycol monoethyl ether, diethylene glycol monopropyl ether, diethylene glycol monobutyl ether, diethylene glycol monopentyl ether, diethylene glycol monoheptyl ether, diethylene glycol diethyl ether, diethylene glycol dipropyl ether, diethylene glycol dibutyl ether, diisopropyl ether, diisobutyl ether, diisopentyl ether, di-n-pentyl ether, methyl cyclopentyl ether, methyl cyclohexyl ether, di-n-butyl ether, di-sec-butyl ether, diisopentyl ether, di-sec-pentyl ether, di-tert-amyl ether, di-n-hexyl ether, and anisole; ester solvents such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactone solvents such as γ-butyrolactone; water; etc.

Note that, the component (D) organic solvent is added preferably in an amount within a range of 10 to 50,000 parts by mass based on 100 parts by mass of the resin of the components (A) and (B) combined.

In the case where the component (B) resin is a silicone adhesive that performs crosslinking through a hydrosilylation reaction, when the water content in the solvent is 0.2% by mass or less, the risk that the progress of the hydrosilylation reaction is hindered is decreased further. It is preferable to proceed crosslinking reaction sufficiently from the viewpoint of exhibiting sufficient adhesiveness and leaving no residue of the bio-electrode composition on the skin after removing an electrode from the skin. Therefore, it is particularly preferable to remove water from a solution in which the inventive ionic polymer is dissolved. For water removal, methods such as azeotropic dehydration, centrifugation, adsorption using a molecular sieve, and filtration using a water removal filter and the like can be applied.

### [Other Additives]

The inventive bio-electrode composition may be mixed with silica particles, polyether silicone, and polyglycerin silicone. Silica particles have hydrophilic surfaces and favorable compatibility with the hydrophilic ion polymer, polyether silicone, and polyglycerin silicone, and can improve the dispersibility of the ionic polymer, polyether silicone, and polyglycerin silicone in the hydrophobic silicone adhesive. The silica particles may be preferably used in either dry type or wet type.

### [Silicone Compound Having Polyglycerin Structure]

The inventive bio-electrode composition may contain a silicone compound having a polyglycerin structure to enhance the sensitivity to ions released from the skin and the ionic conductivity by enhancing the moisture-holding property of the film. The silicone compound having a polyglycerin structure is blended preferably in an amount of 0.01 to 100 parts by mass, more preferably 0.5 to 60 parts by mass, based on 100 parts by mass of the total of the components (A) and (B). Additionally, the silicone compound having a polyglycerin structure may be used alone, or two or more kinds thereof may be used in mixture.

The silicone compound having a polyglycerin structure is preferably represented by any of the following general formulae (4)' and (5)'. 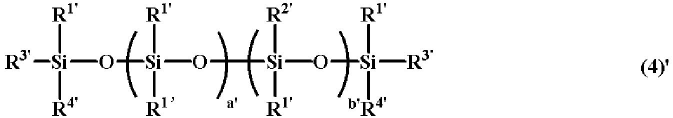 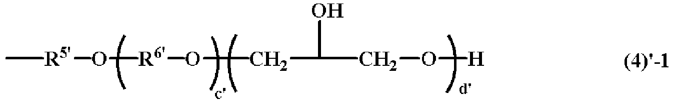 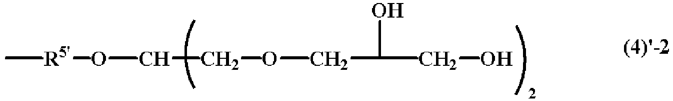 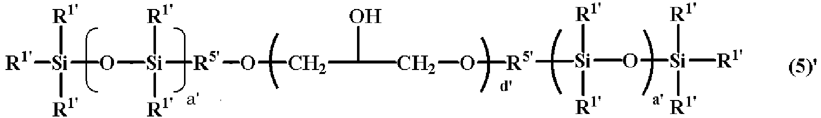 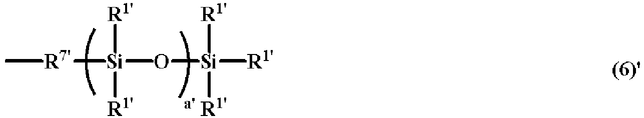 In the formulae (4)' and (5)', each R^{1'} is identical to or different from one another, and independently represents a hydrogen atom, a linear or branched alkyl group having 1 to 50 carbon atoms, a phenyl group, or a silicone chain represented by a general formula (6)', and may have an ether group. R^{2'} represents a group having a polyglycerin group structure represented by the formula (4)'-1 or (4)'-2. Each R^{3'} is identical to or different from the other, and independently represents the R^{1'} group or the R^{2'} group. Each R^{4'} may be identical to or different from the other, and independently represents the R^{1'} group, the R^{2'} group, or an oxygen atom. When R^{4'} represents an oxygen atom, the two R^{4'} groups bond to each other and optionally constitute an ether group to form a ring together with silicon atoms. Each a' is identical to or different from each another and represents 0 to 100, b' represents 0 to 100, and a'+b' is 0 to 200. Nevertheless, when b' is 0, at least one R^{3'} is the R^{2'} group. In the formulae (4)'-1, (4)'-2, (5)', and (6)', R^{5'} represents an alkylene group having 2 to 10 carbon atoms or an aralkylene group having 7 to 10 carbon atoms. R^{6'} and R^{7'} represent an alkylene group having 2 to 6 carbon atoms, and R^{7'} may represent an ether bond. c' represents 0 to 20. d' represents 1 to 20.

Examples of such a silicone compound having a polyglycerin structure can include the following. 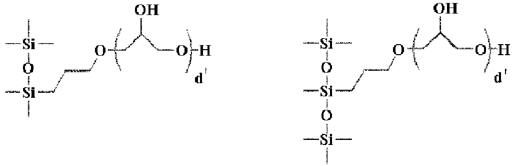 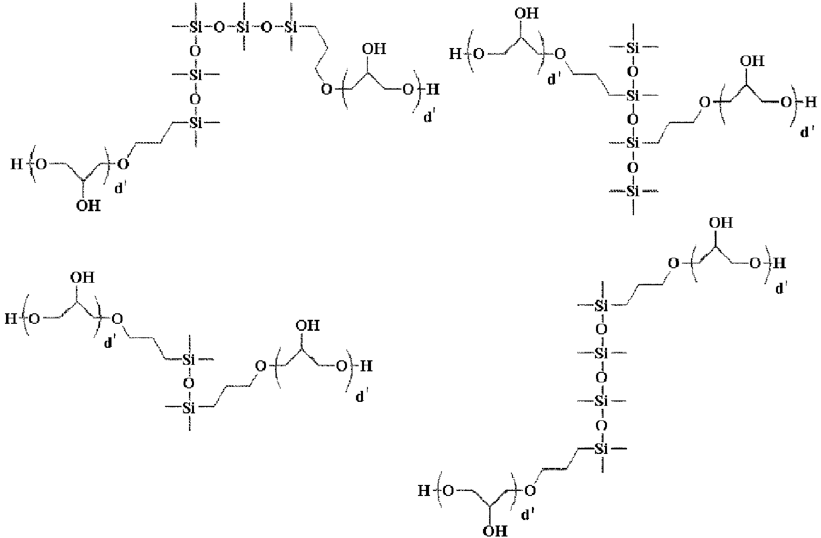 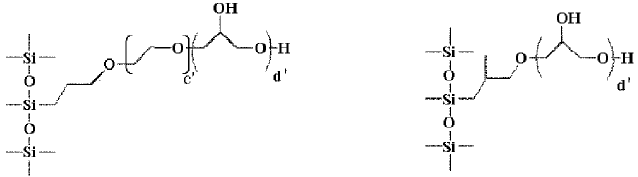 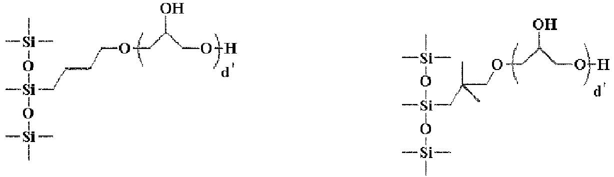 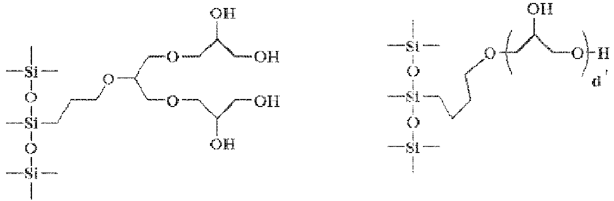 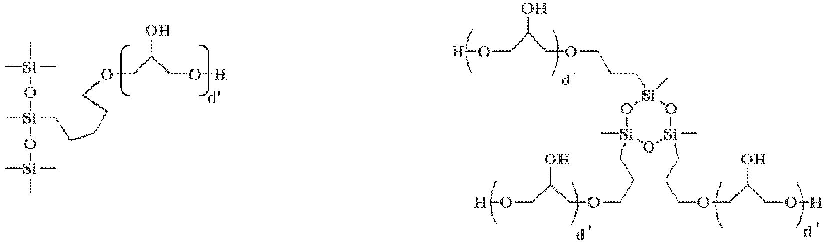 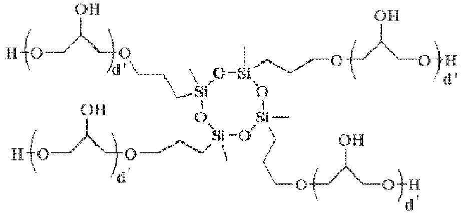 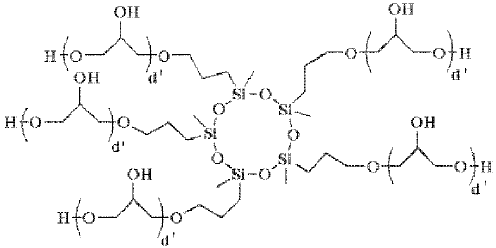 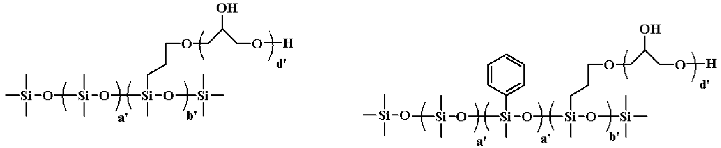 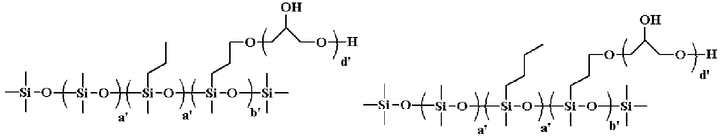 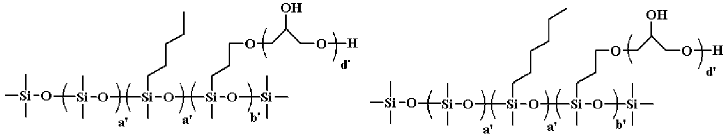 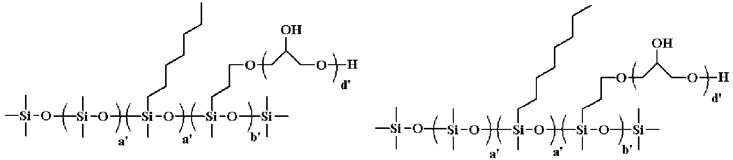 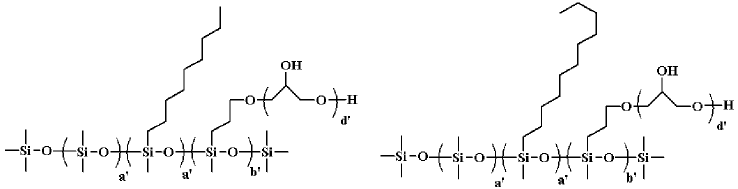 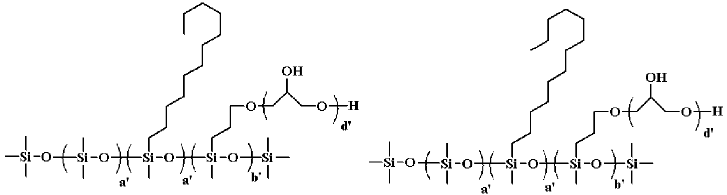 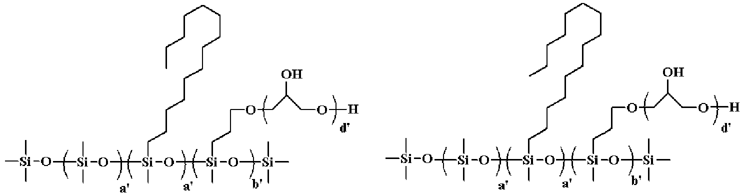 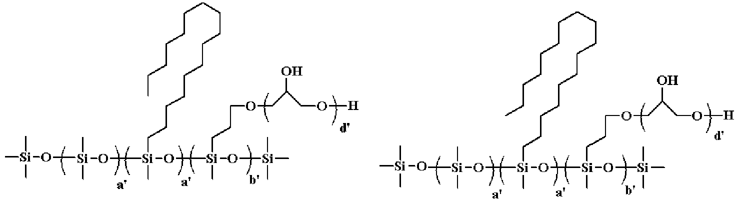 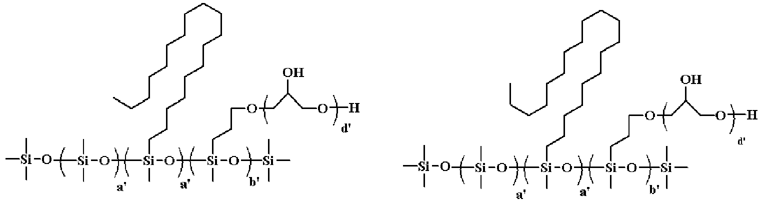 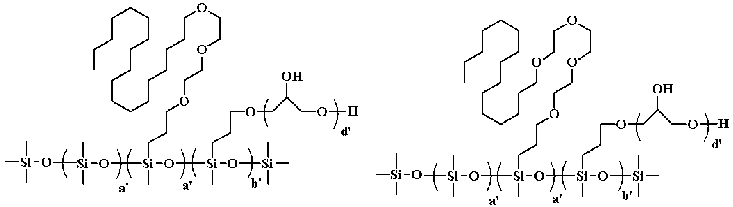 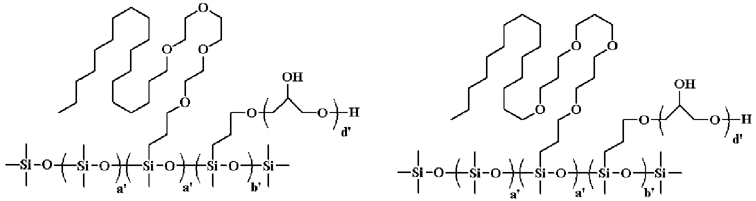 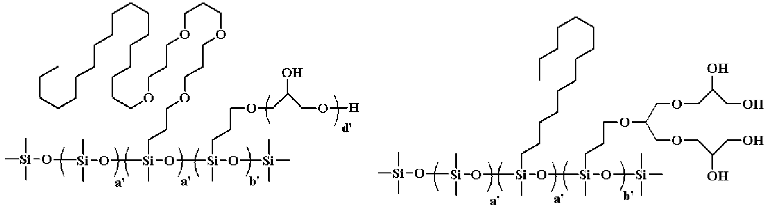 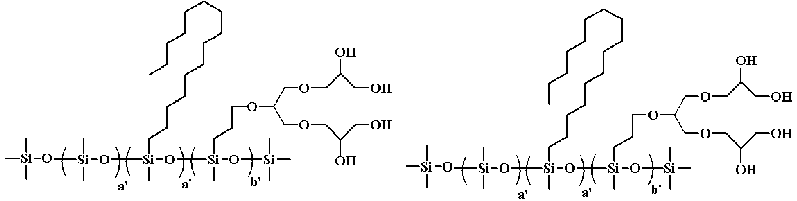 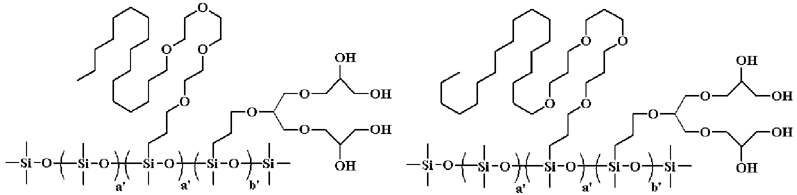 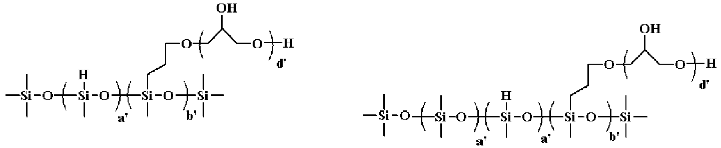 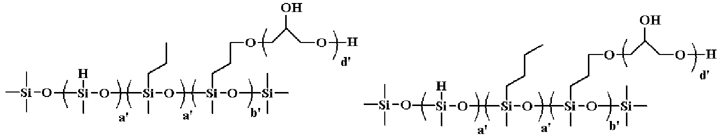 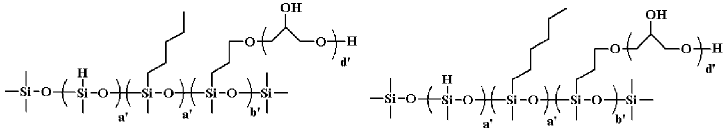 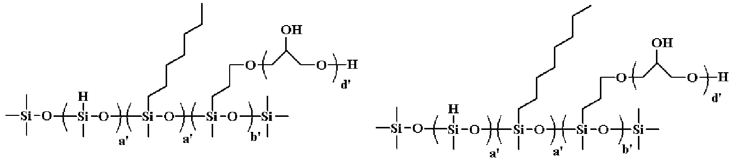 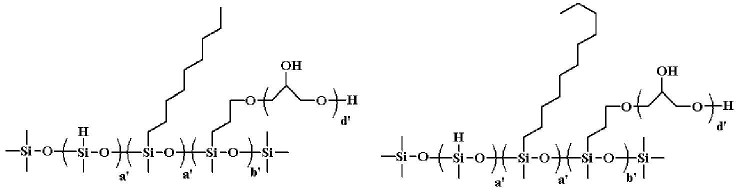 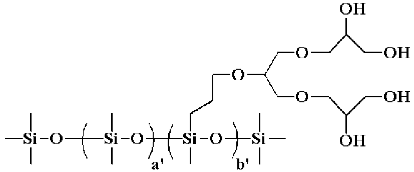 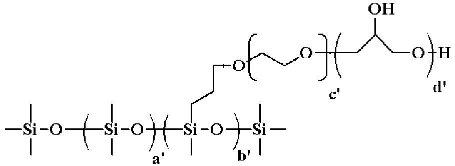 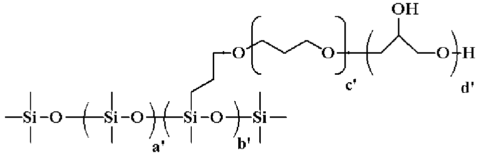 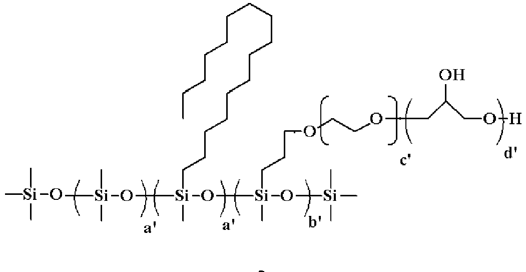 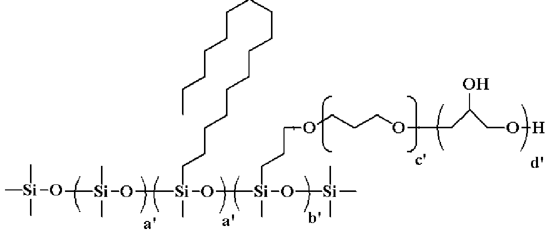 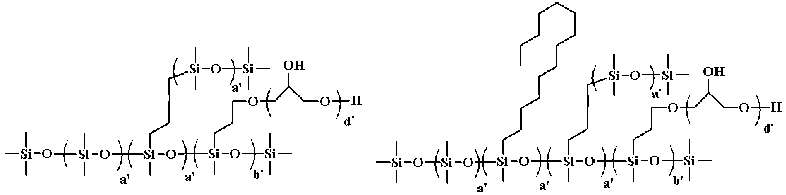 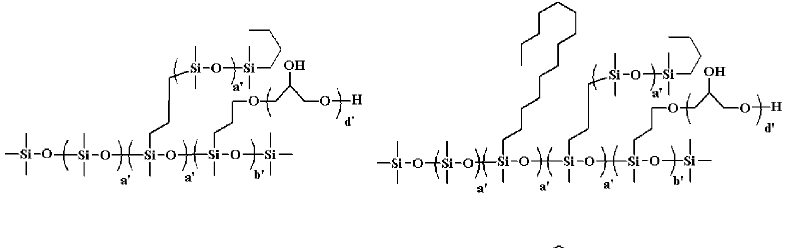 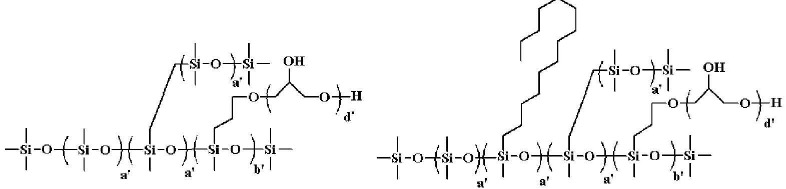 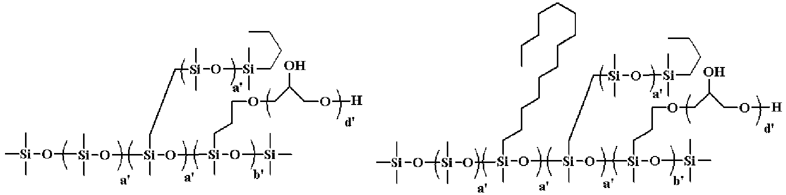 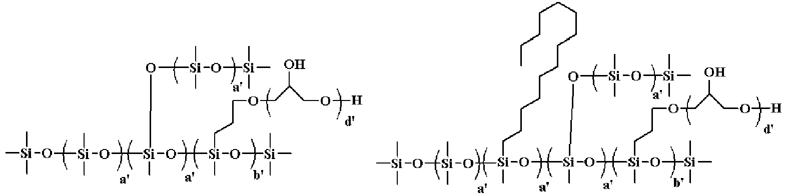 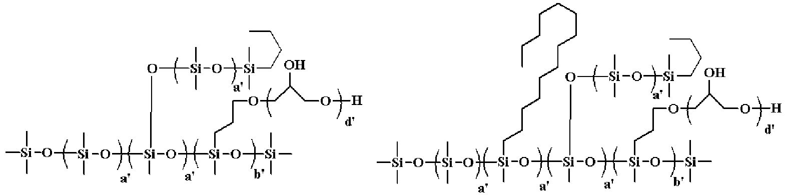 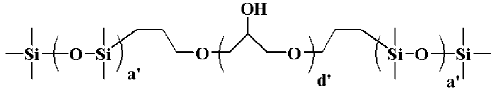 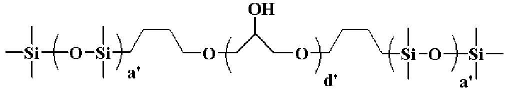 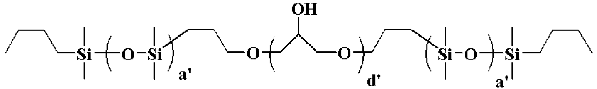 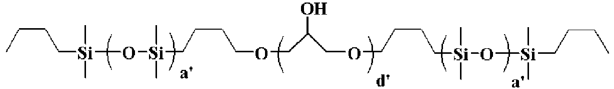 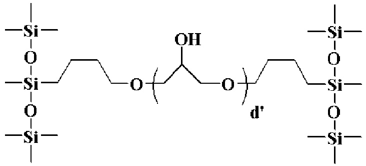 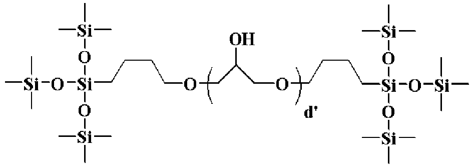 In the formulae, a', b', c', and d' are as defined above.

When such a silicone compound having a polyglycerin structure is incorporated, the resulting bio-electrode composition is capable of forming a living body contact layer that can exhibit more excellent moisture-holding property and consequently exhibit more excellent sensitivity to ions released from the skin.

As described above, the inventive bio-electrode composition makes it possible to form a living body contact layer for a bio-electrode that is: highly adhesive to have sufficient adhesiveness even when detached from the skin and then reattached; capable of conducting electric signals from the skin efficiently to a device (i.e., excellent in electric conductivity); free from a risk of causing allergies even when the bio-electrode is attached to the skin for a long period (i.e., excellent in biocompatibility); light-weight; manufacturable at low cost; and free from significant reduction in the electric conductivity even when the bio-electrode is wetted with water or dried. Moreover, it is possible to further enhance the electric conductivity by adding a carbon material, and it is possible to manufacture a bio-electrode with particularly high adhesive strength and high stretchability by combining with a resin having adhesiveness and stretchability. Further, the stretchability and the adhesiveness to the skin can be enhanced with an additive and the like. The stretchability and the adhesiveness can also be controlled by appropriately adjusting the composition of the resin and the thickness of the living body contact layer.

### <Bio-Electrode>

Furthermore, the present invention also provides a bio-electrode including a conductive substrate and a living body contact layer formed on the conductive substrate, the living body contact layer being a cured product of the inventive bio-electrode composition described above.

Hereinafter, the inventive bio-electrode will be described in detail with reference to the drawings, but the present invention is not limited thereto.

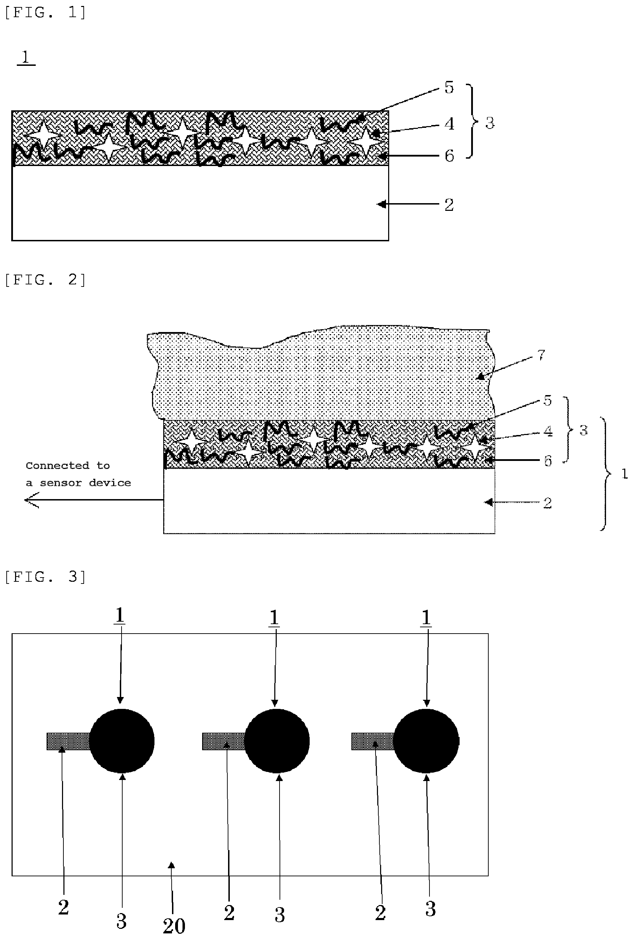

FIG. 1 is a schematic sectional view showing an example of the inventive bio-electrode. In FIG. 1, a bio-electrode 1 has a conductive substrate 2 and a living body contact layer 3 formed on the conductive substrate 2. The living body contact layer 3 is formed from a cured product of the inventive bio-electrode composition. The living body contact layer 3 contains the ionic resin (A) 5. The living body contact layer 3 can further contain a resin (B) 6 other than the ionic resin (A) 5 and an electro-conductive powder 4. Hereinbelow, with reference to FIGs. 1 and 2, the living body contact layer 3 is described as a layer in which the ionic resin (A) 5 and the electro-conductive powder 4 are dispersed in the resin (B) 6, but the inventive bio-electrode is not limited to this embodiment.

When the bio-electrode 1 as shown in FIG. 1 is used, the living body contact layer 3 (i.e., the layer in which the ionic resin (A) 5 and the electro-conductive powder 4 are dispersed in the resin (B) 6) is brought into contact with a living body 7 as shown in FIG. 2. Electric signals are picked from the living body 7 through the component (A) ionic resin 5 and the electro-conductive powder 4, and then conducted to a sensor device or the like (not shown) via the conductive substrate 2. As described above, the inventive bio-electrode is capable of achieving both electric conductivity and biocompatibility by using the component (A) ionic resin 5, and obtaining electric signals from the skin stably in high sensitivity because the contact area with the skin is kept constant due to the adhesiveness thereof.

Hereinafter, each component of the inventive bio-electrode will be described more specifically.

### [Conductive Substrate]

The inventive bio-electrode has a conductive substrate. This conductive substrate is usually connected electrically with a sensor device etc., and conducts electrical signals picked from a living body through the living body contact layer to the sensor device etc.

The conductive substrate is not particularly limited, as long as it has electric conductivity. The conductive substrate preferably contains one or more kinds selected from the group consisting of gold, silver, silver chloride, platinum, aluminum, magnesium, tin, tungsten, iron, copper, nickel, stainless steel, chromium, titanium, carbon, and electro-conductive polymer, for example.

The conductive substrate is not limited particularly, and may be a hard electro-conductive substrate, an electro-conductive film having flexibility, a cloth with the surface being coated with electro-conductive paste, a cloth into which electro-conductive polymer is kneaded. The conductive substrate may be flat, uneven, or mesh-form of woven metal wires, which can be appropriately selected in accordance with the use of the bio-electrode and so forth.

### [Living Body Contact Layer]

The inventive bio-electrode has a living body contact layer formed on the conductive substrate. This living body contact layer is a part to be actually in contact with a living body when using the bio-electrode, and has electric conductivity and adhesiveness. The living body contact layer is a cured product of the inventive bio-electrode composition described above, and, that is, is an adhesive resin layer formed of a cured product of a composition containing the component (A), and, if needed, the component (B), the component (C), the component (D), or the other component(s).

Note that, the living body contact layer preferably has an adhesive strength in a range of 0.01 N/25mm or more and 20 N/25mm or less. The adhesive strength is commonly measured according to the method described in JIS Z 0237, in which a metal substrate such as a stainless steel (SUS) substrate or a polyethylene terephthalate (PET) substrate can be used as a base material. Alternatively, human skin can be used for the measurement. Human skin has lower surface energy than metals and various plastics, which is as low as that of Teflon (registered trademark). Human skin is hard to adhere.

In applications where adhering and peeling are repeated many times, removability is more important than adhesiveness, so adhesiveness is not necessarily required.

The living body contact layer of the bio-electrode has a thickness of preferably 1 µm or more and 5 mm or less, more preferably 2 µm or more and 3 mm or less. As the living body contact layer is thinner, the adhesive strength lowers, but the compatibility with the skin is improved because of improved flexibility and decreased weight. The thickness of the living body contact layer can be selected based on the balance of adhesiveness and texture to the skin.

The inventive bio-electrode may be additionally provided with an adherent film on the living body contact layer as conventional bio-electrodes (e.g., the bio-electrode disclosed in JP 2004-033468 A) in order to prevent peeling off of the bio-electrode from a living body during the use. When the adherent film is provided separately, the adherent film may be formed by using a raw material for the adherent film such as an acrylic type, a urethane type, and a silicone type. Particularly, the silicone type is suitable because of: the high oxygen permeability, which enables dermal respiration while the electrode is attached to the skin; the high water repellency, which prevent lowering of adhesiveness due to perspiration; and the low irritation to the skin. It is to be noted that the inventive bio-electrode does not necessarily require the adherent film that is provided separately, because peeling off from a living body can be prevented by adding a tackifier to the bio-electrode composition or using a resin having good adhesiveness to a living body as described above.

When the inventive bio-electrode is used as a wearable device, wiring between the bio-electrode and a sensor device, and other components are not limited to particular ones. For example, it is possible to employ those disclosed in JP 2004-033468 A.

As described above, since the inventive bio-electrode includes the living body contact layer formed of the cured product of the aforementioned inventive bio-electrode composition, the inventive bio-electrode is capable of efficiently conducting electric signals from the skin to a device (i.e., excellent in electric conductivity), does not cause allergies even after long-period attachment to the skin (i.e., excellent in biocompatibility), is light-weight and manufacturable at low cost, and prevents significant reduction in the electric conductivity even when wetted with water or dried. In addition, it is possible to further improve the electric conductivity by adding an electro-conductive powder, and it is possible to manufacture a bio-electrode with particularly high adhesive strength and high stretchability by combining the inventive bio-electrode composition with a resin having adhesiveness and stretchability. Further, the stretchability and adhesiveness to the skin can be improved with an additive and the like. The stretchability and adhesiveness can also be controlled by appropriately adjusting the composition of the resin and the thickness of the living body contact layer. Accordingly, the inventive bio-electrode as described above is particularly suitable as a bio-electrode used for a medical wearable device.

### <Method for Manufacturing Bio-Electrode>

Further, the present invention also provides a method for manufacturing a bio-electrode having a conductive substrate and a living body contact layer formed on the conductive substrate, the method including: applying the inventive bio-electrode composition onto the conductive substrate; and curing the bio-electrode composition to form the living body contact layer.

Note that, the conductive substrate etc. used in the inventive method for manufacturing a bio-electrode may be the same as those described above.

The method for applying the bio-electrode composition onto the conductive substrate is not particularly limited. Examples of suitable method include dip coating, spray coating, spin coating, bar coating, comma coating, die coating, roll coating, flow coating, doctor coating, calendar coating, screen printing, flexographic printing, gravure printing, inkjet printing, etc.

The method for curing the resin is not particularly limited and may be appropriately selected based on the kind of the components (A) and (B) used for the bio-electrode composition. For example, the bio-electrode composition is preferably cured by either or both of heat and light. The foregoing bio-electrode composition can also be cured by adding a catalyst in advance to generate acid or base to the bio-electrode composition, which causes a crosslinking reaction.

The heating temperature is not particularly limited and may be appropriately selected based on the kind of the components (A) and (B) used for the bio-electrode composition, but is preferably about 50 to 250°C for example.

When the heating and the light irradiation are combined, it is possible to perform the heating and the light irradiation simultaneously, to perform the light irradiation and then the heating, or to perform the heating and then the light irradiation. It is also possible to perform air-drying to evaporate the solvent before heating the coating film.

Water droplets may be attached to the surface of the cured film; alternatively, the film surface may be sprayed with water vapor or mist. These treatments improve the compatibility with the skin, and enable quick collection of biological signals. Water mixed with alcohol can be used to reduce the size of water droplets in the water vapor or the mist. The film surface may be wetted by bringing an absorbent cotton or cloth containing water into contact therewith.

The water for making the surface of the cured film wet may contain a salt. The water-soluble salt mixed with the water is selected from the group consisting of sodium salts, potassium salts, calcium salts, magnesium salts, and betaines.

Specifically, the water-soluble salt can be a salt selected from the group consisting of sodium chloride, potassium chloride, calcium chloride, magnesium chloride, a saccharin sodium salt, acesulfame potassium, sodium carboxylate, potassium carboxylate, calcium carboxylate, sodium sulfonate, potassium sulfonate, calcium sulfonate, sodium phosphate, potassium phosphate, calcium phosphate, magnesium phosphate, and betaines. It should be noted that the ionic resin (A) described above are excluded from the water-soluble salt.

More specific examples of the water-soluble salt include, besides the aforementioned examples, sodium acetate, sodium propionate, sodium pivalate, sodium glycolate, sodium butyrate, sodium valerate, sodium caproate, sodium enanthate, sodium caprylate, sodium pelargonate, sodium caprate, sodium undecylate, sodium laurate, sodium tridecylate, sodium myristate, sodium pentadecylate, sodium palmitate, sodium margarate, sodium stearate, sodium benzoate, disodium adipate, disodium maleate, disodium phthalate, sodium butyrate, sodium 2-hydroxybutyrate, sodium 3-hydroxybutyrate, sodium 2-oxobutyrate, sodium stearate, sodium gluconate, sodium methanesulfonate, sodium 1-nonanesulfonate, sodium 1-decanesulfonate, sodium 1-dodecanesulfonate, sodium 1-undecanesulfonate, sodium cocoyl isethionate, sodium lauroyl methylalanine, sodium methyl cocoyl taurate, sodium cocoyl glutamate, sodium cocoyl sarcosinate, sodium lauroyl methyl taurate, lauramidopropyl, potassium isobutyrate, potassium propionate, potassium pivalate, potassium glycolate, potassium gluconate, potassium methanesulfonate, calcium stearate, calcium glycolate, calcium gluconate, calcium 3-methyl-2-oxobutyrate, and calcium methanesulfonate. The term betaines is a general term for inner salts. Specific examples thereof include amino acid compounds whose amino group is added with three methyl groups. More specific examples can include trimethylglycine, carnitine, trimethylglycine, and proline betaines.

The water for wetting the surface of the cured film can further contain a monohydric alcohol or polyhydric alcohol having 1 to 4 carbon atoms. The alcohol is preferably selected from the group consisting of ethanol, isopropyl alcohol, ethylene glycol, diethylene glycol, triethylene glycol, glycerin, polyethylene glycol, polypropylene glycol, polyglycerin, diglycerin, and a silicone compound having a polyglycerin structure. More preferably, the silicone compound having a polyglycerin structure is shown by the general formula (4)' or (5)'.

In the pretreatment methods with the aqueous solution containing the water-soluble salt, the cured bio-electrode film can be wetted by a spraying method, a droplet-dispensing method, etc. The bio-electrode film can also be wetted under a high-temperature, high-humidity condition like a sauna. To prevent drying after the wetting, a protective film can be further stacked on the permeated layer to cover the surface. Since the protective film needs to be removed immediately before the bio-electrode is attached to the skin, it is possible to use the protective film that is coated with a release agent, or a peelable Teflon (registered trademark) film may be used. For long-time storage, the dry electrode covered with the peelable film is preferably sealed in a bag that is covered with aluminum etc. To prevent drying in the bag covered with aluminum, it is preferable to include water therein.

Before the inventive bio-electrode is attached to the skin, the skin may be moisturized with water, alcohol, etc., or the skin may be wiped with a cloth or absorbent cotton containing water, alcohol, etc. The water and the alcohol may contain the above-described salts.

As has been described above, the inventive method for manufacturing a bio-electrode makes it possible to manufacture the inventive bio-electrode easily and at low cost, with the bio-electrode being excellent in electric conductivity and biocompatibility, light-weight, and capable of preventing significant reduction in the electric conductivity even when wetted with water or dried.

### EXAMPLE

Hereinafter, the present invention will be specifically described with reference to Examples and Comparative Examples, but the present invention is not limited thereto.

### Synthesis examples of (2)-1 monomer having a double bond

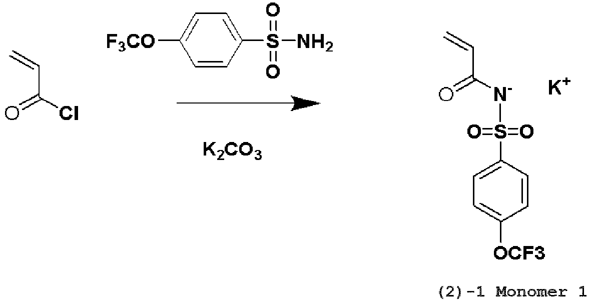

A mixed solution of 22 g of 4-trifluoromethoxybenzenesulfonamide, 14 g of potassium carbonate, and 60 g of acetonitrile was cooled with ice, and then a mixed solution of 10 g of acrylic acid chloride and 30 g of acetonitrile was added dropwise under ice cooling. After raising the temperature to room temperature, the mixture was stirred for 22 hours and dried under reduced pressure to obtain the desired (2)-1 monomer 1. (2)-1 monomers 2 to 7, 9, and 10 were synthesized in a similar manner.

The (2)-1 monomer 8 was synthesized by first reacting 4-trifluoromethoxybenzenesulfonic acid chloride with ethanolamine and then reacting with methacrylic acid chloride.

The (2)-1 monomers 1 to 10 are shown below.

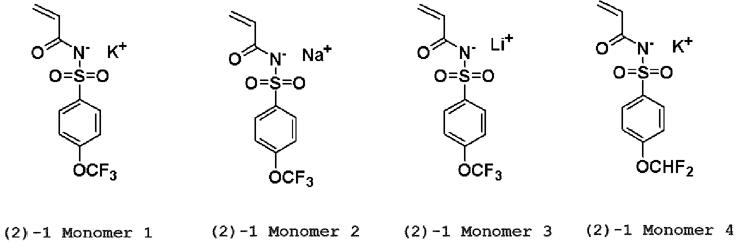

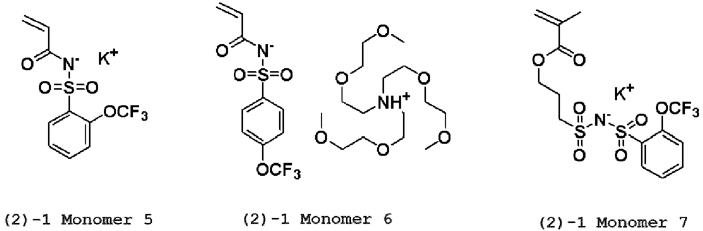

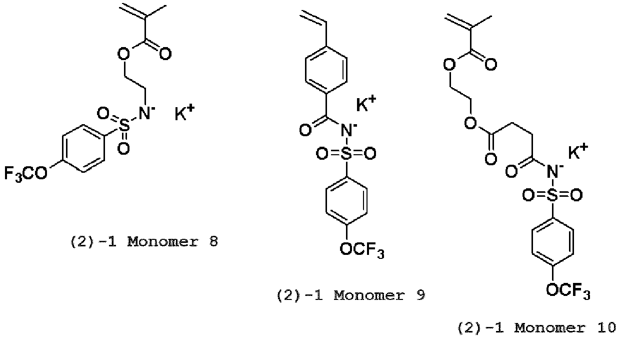

Ionic resins 1-1 to 1-11, which were blended as ionic materials (conductive materials) into the bio-electrode composition solutions, were synthesized as follows. First, 30 mass% solutions of respective monomers in cyclopentanone were introduced into a reaction vessel and mixed. The reaction vessel was cooled to -70°C under a nitrogen atmosphere, and subjected to vacuum degassing and nitrogen blowing three times. After raising the temperature to room temperature, azobisisobutyronitrile (AIBN) was added thereto as a polymerization initiator in an amount of 0.02 moles per 1 mole of all the monomers. The mixture was warmed to 60°C and then allowed to react for 15 hours. After drying the solvent, the composition of the resulting polymer was determined by ¹H-NMR. The molecular weight (Mw) and the dispersity (Mw/Mn) of the obtained polymer were determined by gel permeation chromatography (GPC) using tetrahydrofuran (THF) as a solvent. Ionic resins 1-1 to 1-11 synthesized in this way are shown below.

Ionic resin 1-1
Mw=66,800
Mw/Mn=2.62

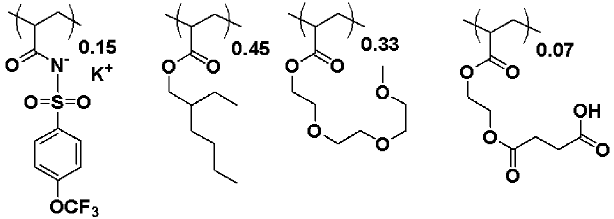

Ionic resin 1-2
Mw=43,100
Mw/Mn=2.46

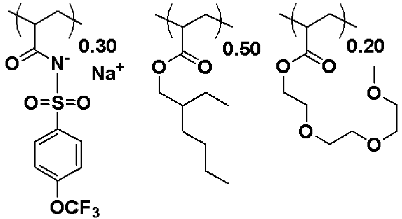

Ionic resin 1-3
Mw=55, 600
Mw/Mn=2.34

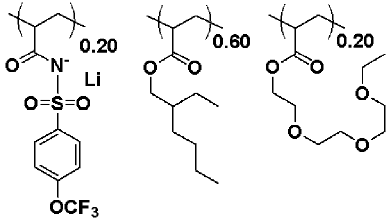

Ionic resin 1-4
Mw=48, 100
Mw/Mn=2.67

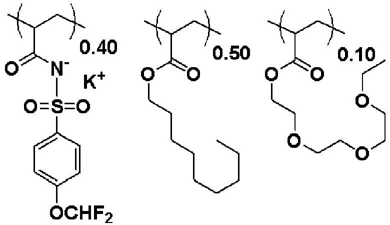

Ionic resin 1-5
Mw=56, 900
Mw/Mn=2.61

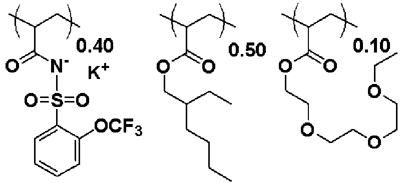

Ionic resin 1-6
Mw=71, 300
Mw/Mn=2.67

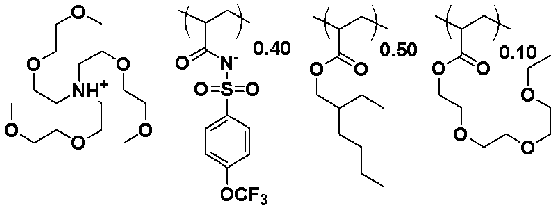

Ionic resin 1-7
Mw=39, 400
Mw/Mn=2.26 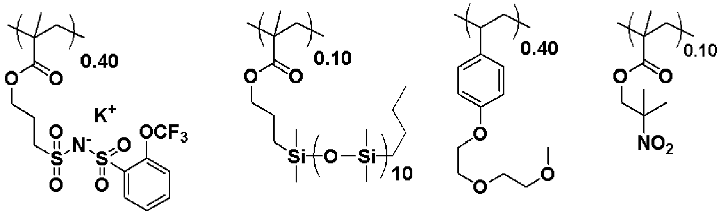
The repeating number in the formula shows the average value.

Ionic resin 1-8
Mw=26,700
Mw/Mn=1.91 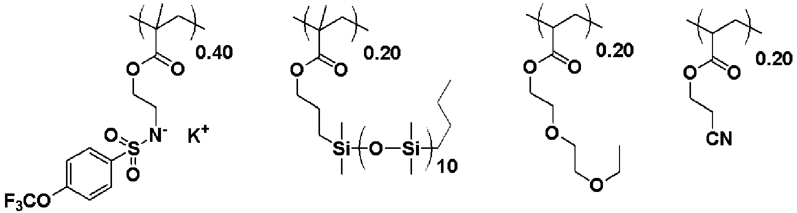
The repeating number in the formula shows the average value.

Ionic resin 1-9
Mw=34, 400
Mw/Mn=2.22 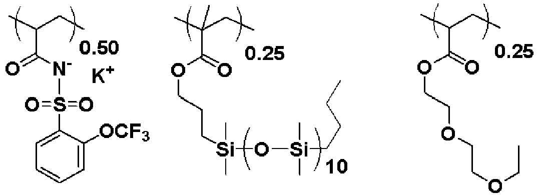
The repeating number in the formula shows the average value.

Ionic resin 1-10
Mw=28,500
Mw/Mn=1.98

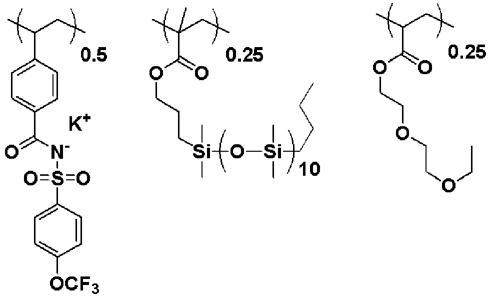

Ionic resin 1-11
Mw=24,700
Mw/Mn=1.98

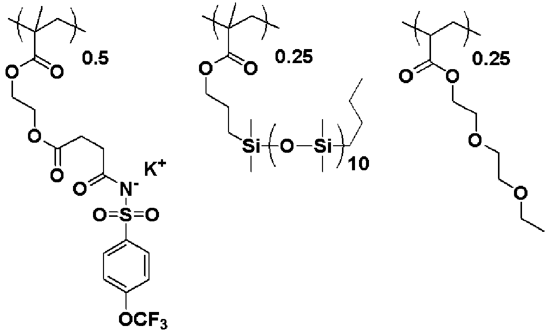

The ionic resins 2-1 to 2-6 blended into the bio-electrode composition solutions as ionic materials (conductive materials) were synthesized by carrying out a hydrosilylation reaction between silicone compounds having an SiH group and sulfonamide salt compounds (the (2)-2 monomers 1 to 3 below) having an alkenyl group in the presence of a platinum catalyst.

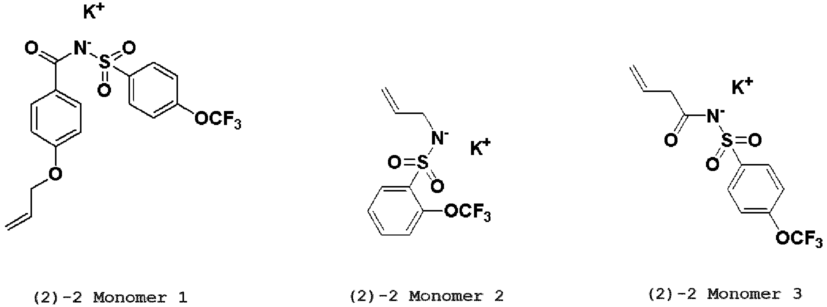

Ionic resin 2-1
Mw=661
Mw/Mn=1.0

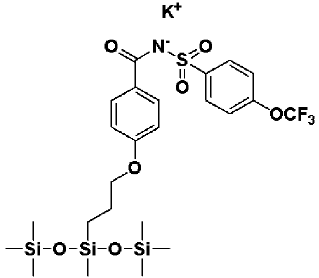

Ionic resin 2-2
Mw=7100
Mw/Mn=1.60

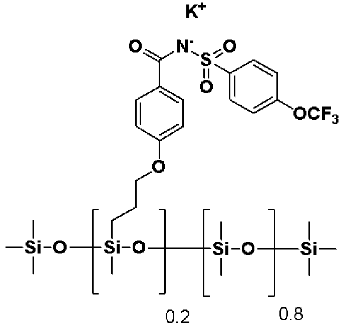

Ionic resin 2-3
Mw=4,400
Mw/Mn=1.46

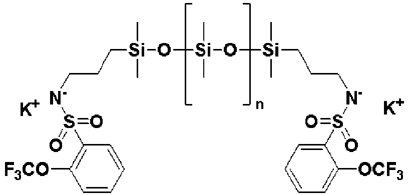

Ionic resin 2-4
Mw=5, 600
Mw/Mn=1.63

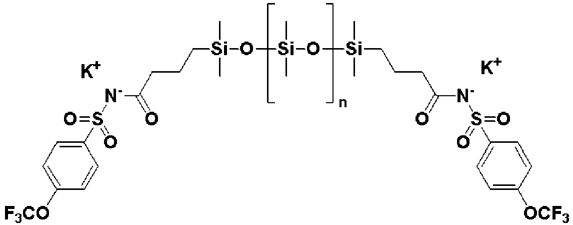

Ionic resin 2-5
Mw=1, 690
Mw/Mn=1.10

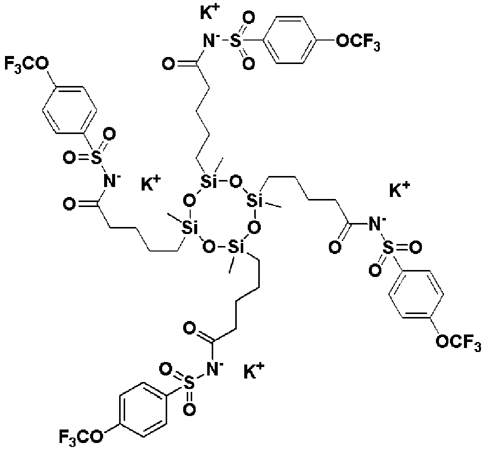

Ionic resin 2-6
Mw=5, 600
Mw/Mn=1.63

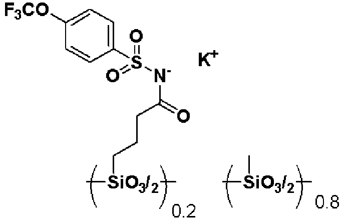

With using the following (2)-3 monomers 1 to 11, an isocyanate compound, in some cases a dihydroxy compound for elongating chain length, a monohydroxy compound or a monoisocyanate compound for sealing a terminal, a solvent propylene glycol monomethyl ether acetate in an amount twice the mass of the raw material compounds, and 0.02% by mass of catalyst XK-640 (manufactured by King Industries, Inc.) based on 100 parts by mass of the raw material compounds, were mixed. The mixture was reacted at 90°C for 9 hours to synthesize the ionic resins 3-1 to 3-14. 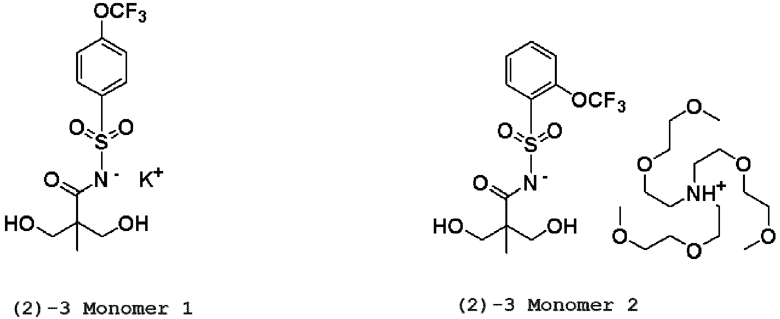 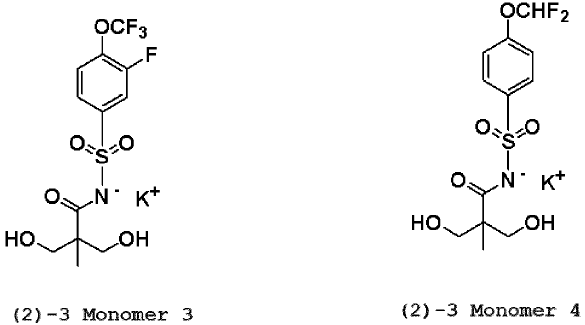 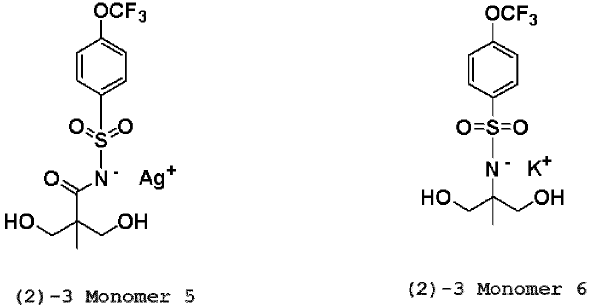 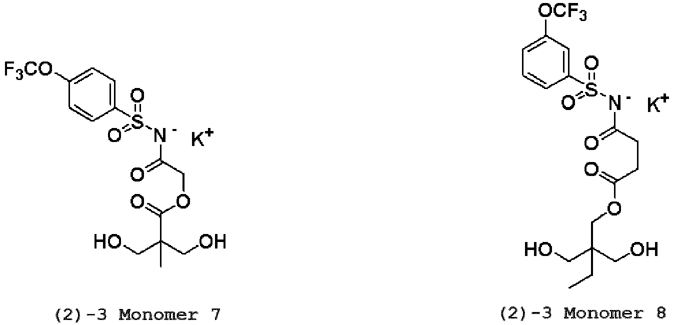 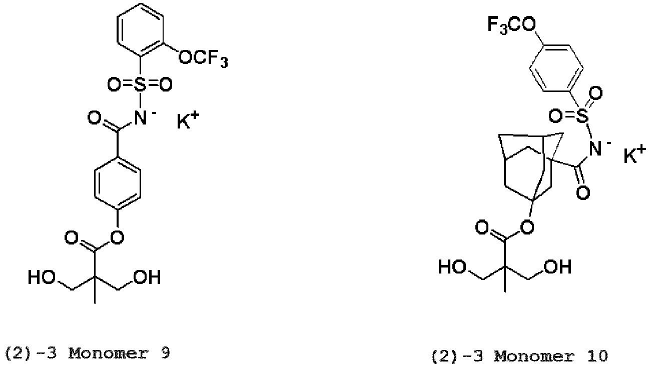 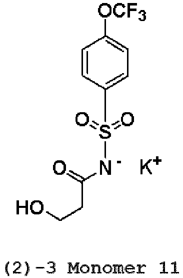 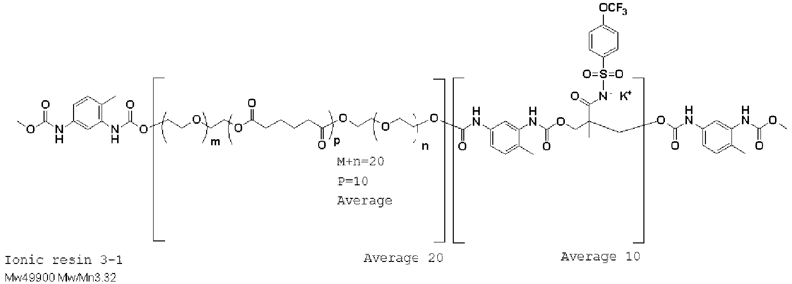 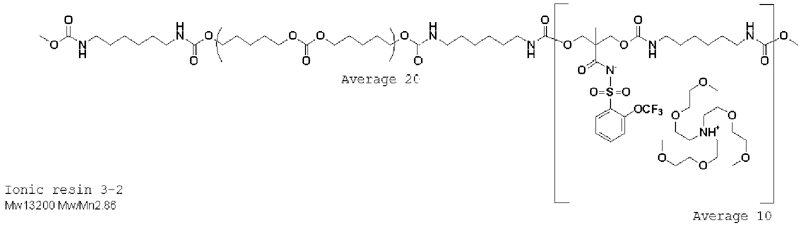 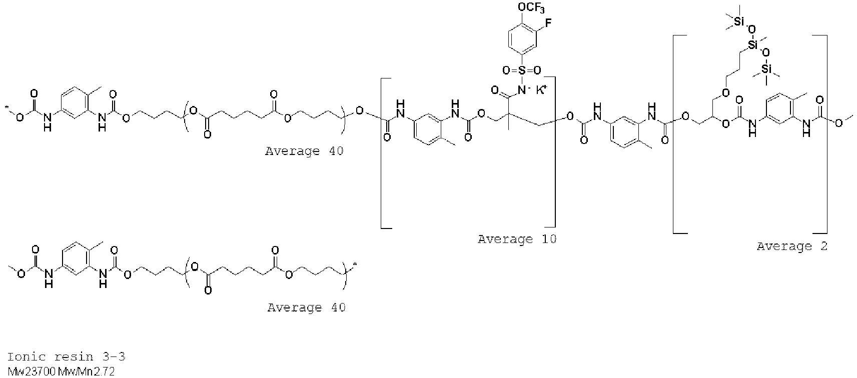 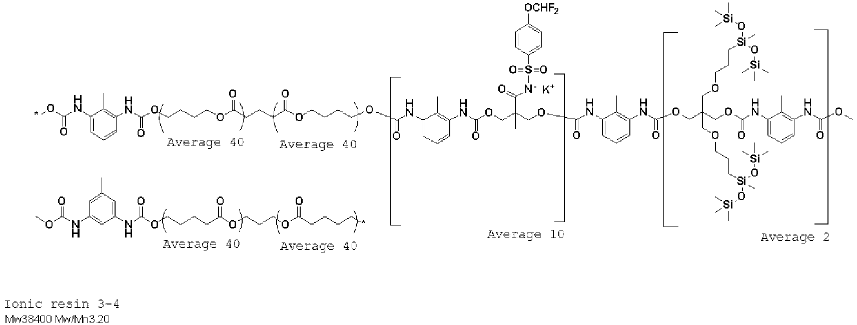 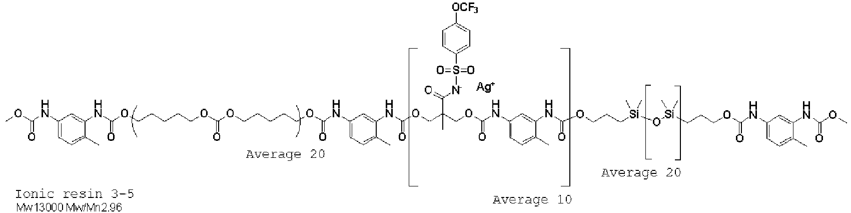 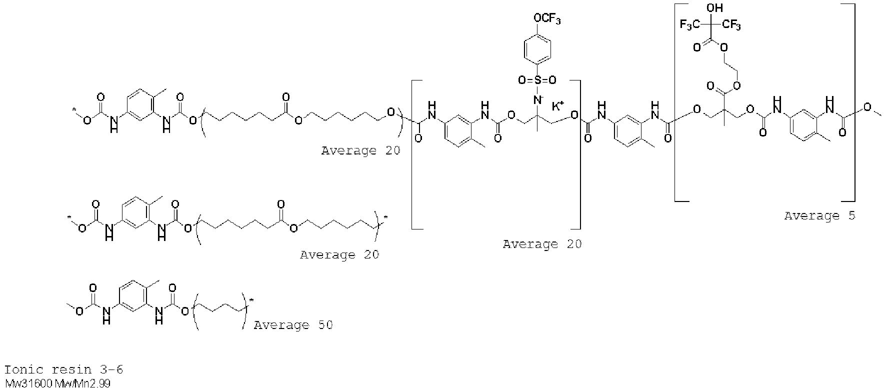 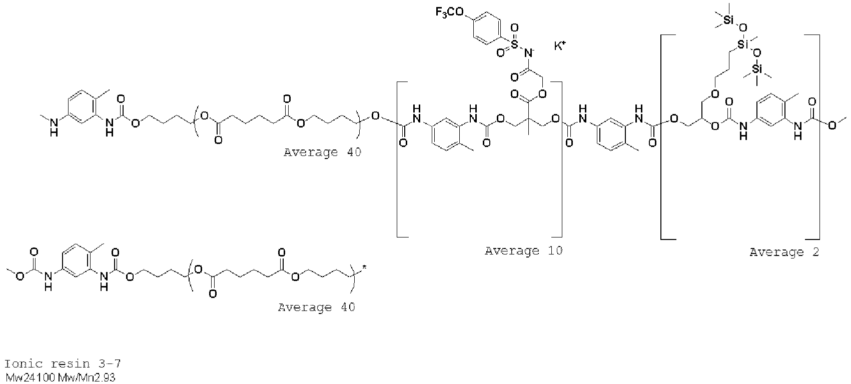 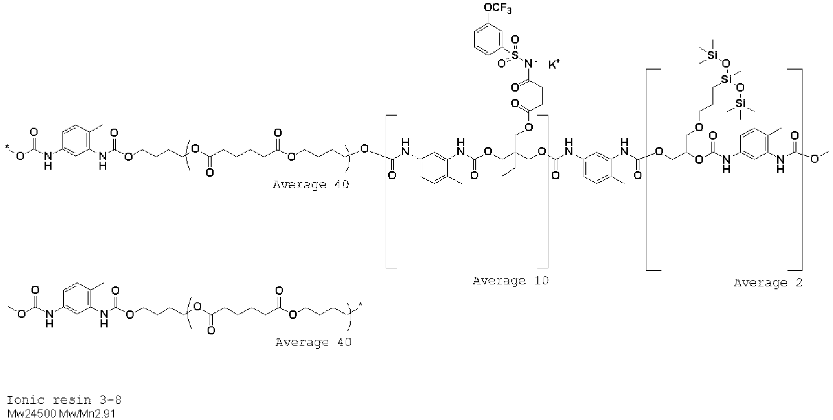 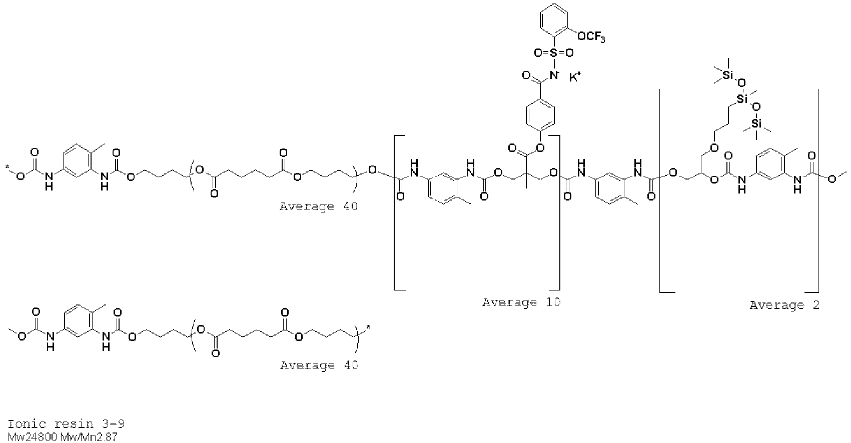 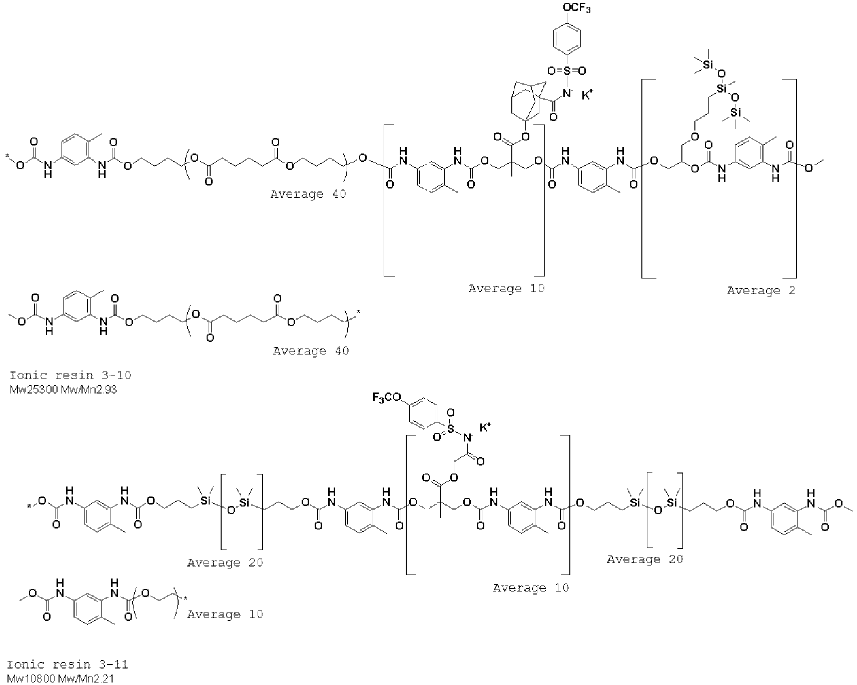 In the figure, * represents a bonding site.

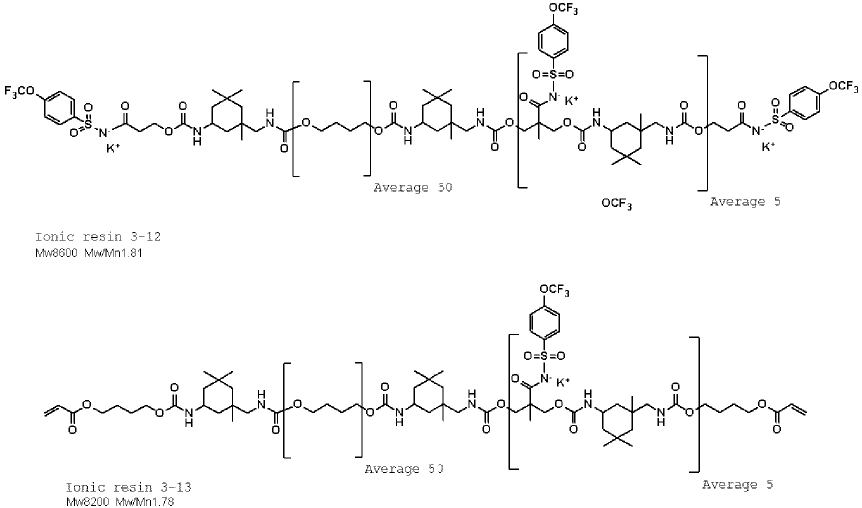

Ionic resin 3-14
Mw=1,580
Mw/Mn=1.09

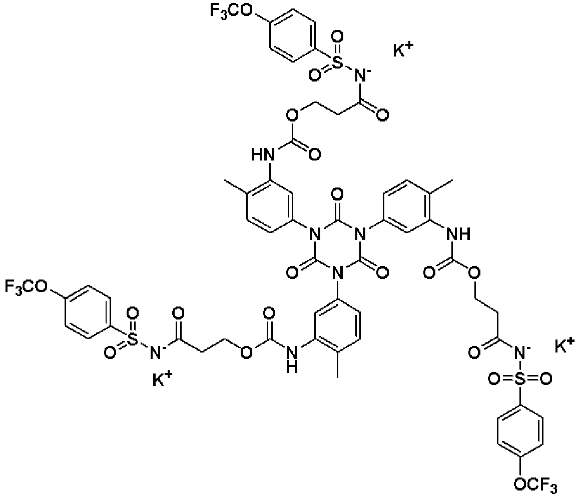

### Comparative ionic resin 1

Mw=26,500
Mw/Mn=1.85

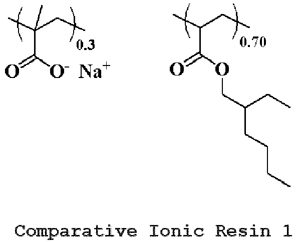

Siloxane compounds 1 to 4, which were blended as silicone-based resin to the bio-electrode composition solutions, are shown below.

### (Siloxane compound 1)

Siloxane Compound 1 was vinyl group-containing polydimethylsiloxane that has a viscosity of 27,000 mPa·s in a 30% toluene solution, an alkenyl group content of 0.007 mol/100 g, and the terminals of molecular chain capped with SiMe₂Vi group.

### (Siloxane compound 2)

Siloxane compound 2 was a 60% toluene solution of polysiloxane of MQ resin composed of a Me₃SiO_{0.5} unit and a SiO₂ unit (Me₃SiO_{0.5} unit/SiO₂ unit = 0.8).

### (Siloxane compound 3)

Siloxane compound 3 was polydimethylsiloxane-bonded MQ resin obtained by heating a solution (composed of 40 parts by mass of vinyl group-containing polydimethylsiloxane having a 30% toluene solution viscosity of 42,000 mPa·s, an alkenyl group-content of 0.007 mol/100 g, and the terminals of molecular chain capped with OH; 100 parts by mass of a 60% toluene solution of polysiloxane of MQ resin composed of an Me₃SiO_{0.5} unit and an SiO₂ unit (Me₃SiO_{0.5} unit/SiO₂ unit = 0.8); and 26.7 parts by mass of toluene) with refluxing for 4 hours, followed by cooling.

### (Siloxane compound 4)

As methylhydrogensilicone oil, KF-99 manufactured by Shin-Etsu Chemical Co., Ltd. was used.

Acrylic resins blended into the bio-electrode composition solutions as acrylic resins are shown below.
Acrylic resin 1
Mw=129,000
Mw/Mn=2.45

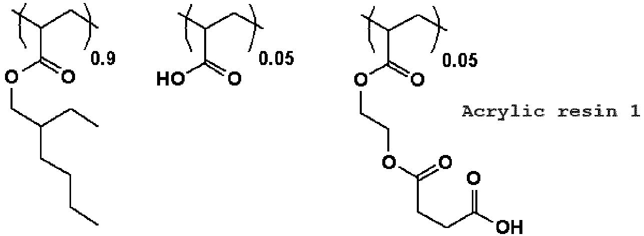

Urethane resins blended into the bio-electrode composition solutions as urethane resins are shown below.
Urethane resin 1
Mw=83,000
Mw/Mn=4.02

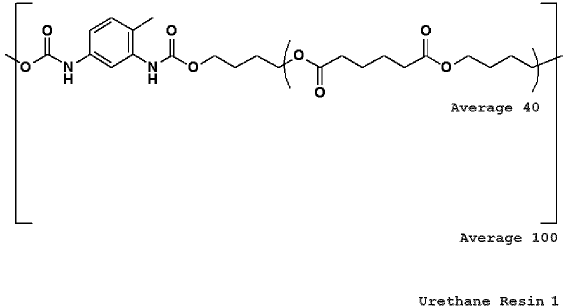

Urethane resin 2
Mw=84,000
Mw/Mn=4.22

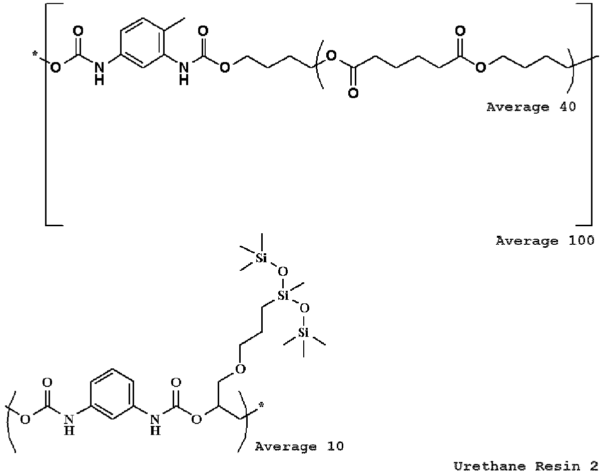

The polyglycerin silicone compound blended into the bio-electrode composition solutions is shown below.

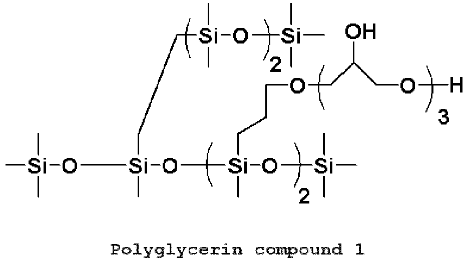

The cross-linking agent added to the bio-electrode composition solutions is shown below.
Epoxy cross-linking agent 1

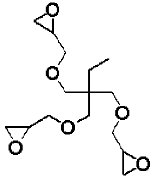

Organic solvents blended to the bio-electrode composition solutions are shown below.
EDE: diethylene glycol diethyl ether
ISOPAR G^{™}: isoparaffin base solvent, manufactured by Standard Sekiyu CO., LTD.
ISOPAR M^{™}: isoparaffin base solvent, manufactured by Standard Sekiyu CO., LTD.

Lithium titanate powder, a radical generator, platinum catalyst, and electric conductivity improver (carbon black, carbon nanotube), which were blended as additives to the bio-electrode composition solutions, are shown below.
Lithium titanate powder, spinel: with the size of 200 nm or less, manufactured by Sigma-Aldrich Co., LLC.
Radical generator: Irgacure TPO, manufactured BASF SE
Platinum catalyst: CAT-PL-50T, manufactured by Shin-Etsu Chemical Co., Ltd.
Carbon black: DENKA BLACK Li-400, manufactured by Denka Co., Ltd.
Multilayer carbon nanotube: with the diameter of 110 to 170 nm and length of 5 to 9 µm, manufactured by Sigma-Aldrich Co., LLC.

### [Examples 1 to 31, Comparative Example 1]

According to the compositions shown in Tables 1 to 3, ionic resins, resins, organic solvents, and additives (Radical generator, platinum catalyst, electric conductivity improver) were blended to prepare the bio-electrode compositions (Bio-electrode solutions 1 to 31, Comparative bio-electrode solutions 1).

**[Table 1]**

| Bio-electrode solution | Ionic polymer (parts by mass) | Resin (parts by mass) | Organic solvent (parts by mass) | Additive (parts by mass) |
|---|---|---|---|---|
| Bio-electrode solution 1 | Ionic resin 1-1 (100) | - | EDE (120) | Epoxy crosslinking agent 1 (0.2) |
| | | | | Carbon black (8) |
| Bio-electrode solution 2 | Ionic resin 1-2 (70) | Acrylic resin 1 (30) | EDE (60) | Epoxy crosslinking agent 1 (0.2) |
| | | | Cyclopentanone (47) | Carbon black (8) |
| Bio-electrode solution 3 | Ionic resin 1-3 (30) | Acrylic resin 1 (70) | EDE (60) | Epoxy crosslinking agent 1 (0.2) |
| | | | Cyclopentanone (47) | Carbon black (8) |
| Bio-electrode solution 4 | Ionic resin 1-4 (30) | Acrylic resin 1 (70) | EDE (60) | Epoxy crosslinking agent 1 (0.2) |
| | | | Cyclopentanone (47) | Carbon black (8) |
| Bio-elect rode solution 5 | Ionic resin 1-5 (40) | Acrylic resin 1 (60) | EDE (60) | Epoxy crosslinking agent 1 (0.2) |
| | | | Cyclopentanone (47) | Carbon black (8) |
| Bio-electrode solution 6 | Ionic resin 1-6 (40) | Acrylic resin 1 (60) | EDE (60) | Epoxy crosslinking agent 1 (0.2) |
| | | | Cyclopentanone (47) | Multilayer carbon nanotube (3) |
| Bio-electrode solution 7 | Ionic resin 1-7 (20) | Siloxane compound 1 (40) | ISOPAR G (60) | CAT-PL-50T (1.5) |
| | | | | Carbon black (8) |
| | | Siloxane compound 2 (100) | Cyclopentanone (47) | |
| | | Siloxane compound 4 (3) | | Polyglycerol compound 1 (8) |
| Bio-electrode solution 8 | Ionic resin 1-8 (10) | Siloxane 1 (40)compound | ISOPAR G (60) | CAT-PL-50T (1.5) |
| | | | | Carbon black (8) |
| | | Siloxane compound 2 (100) | Cyclopentanone (47) | |
| | | Siloxane compound 4 (3) | | Polyglycerol compound 1 (8) |
| Bio-elect rode solution 9 | Ionic resin 1-9 (20) | Siloxane compound 3 (126) | ISOPAR M (60) | CAT-PL-50T (1.5) |
| | | | | Carbon black (8) |
| | | Siloxane compound 4 (3) | Cyclopentanone (47) | Polyglycerol compound 1 (8) |
| Bio-electrode solution 10 | Ionic resin 1-10 (12) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |
| Bio-elect rode solution 11 | Ionic resin 1-11 (12) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |
| Bio-elect rode solution 12 | Ionic resin 2-1 (12) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |
| Bio-electrode solution 13 | Ionic resin 2-2 (14) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |
| Bio-electrode solution 14 | Ionic resin 2-3 (15) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |
| Bio-electrode solution 15 | Ionic resin 2-4 (20) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |
| Bio-electrode solution 16 | Ionic resin 2-5 (20) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |
| Bio-electrode solution 17 | Ionic resin 2-6 (20) | Siloxane compound 3 (126) | ISOPAR M (60) | CAT-PL-50T (1.5) |
| | | | | Carbon black (12) |
| | | Siloxane compound 4 (3) | Cyclopentanone (47) | Polyglycerol compound 1 (8) |

**[Table 2]**

| Bio-electrode solution | Ionic polymer (parts by mass) | Resin (parts by mass) | Organic solvent (parts by mass) | Additive (parts by mass) |
|---|---|---|---|---|
| Bio-electrode solution 18 | Ionic resin 3-1 (100) | - | EDE (120) | Carbon black (8) |
| Bio-electrode solution 19 | Ionic resin 3-2 (70) | Urethane resin 1 (30) | EDE (60) | Carbon black (8) |
| | | | Cyclopentanone (47) | |
| Bio-electrode solution 20 | Ionic resin 3-3 (30) | Urethane resin 2 (70) | EDE (60) | Carbon black (8) |
| | | | Cyclopentanone (47) | |
| Bio-electrode solution 21 | Ionic resin 3-4 (30) | Urethane resin 2 (70) | EDE (60) | Carbon black (8) |
| | | | Cyclopentanone (47) | |
| Bio-electrode solution 22 | Ionic resin 3-5 (40) | Urethane resin 2 (60) | EDE (60) | Carbon black (8) |
| | | | Cyclopentanone (47) | |
| Bio-electrode solution 23 | Ionic resin 3-6 (40) | Urethane resin 2 (60) | EDE (60) | Carbon black (8) |
| | | | Cyclopentanone (47) | |
| Bio-electrode solution 24 | Ionic resin 3-7 (40) | Urethane resin 2 (60) | EDE (60) | Carbon black (8) |
| | | | Cyclopentanone (47) | |
| Bio-electrode solution 25 | Ionic resin 3-8 (40) | Urethane resin 2 (60) | EDE (60) | Carbon black (8) |
| | | | Cyclopentanone (47) | |
| Bio-electrode solution 26 | Ionic resin 3-9 (40) | Urethane resin 2 (60) | EDE (60) | Carbon black (8) |
| | | | Cyclopentanone (47) | |
| Bio-electrode solution 27 | Ionic resin 3-10 (40) | Urethane resin 2 (60) | EDE (60) | Carbon black (8) |
| | | | Cyclopentanone (47) | |
| Bio-electrode solution 28 | Ionic resin 3-11 (40) | Urethane resin 2 (60) | EDE (60) | Carbon black (8) |
| | | | Cyclopentanone (47) | |
| Bio-electrode solution 29 | Ionic resin 3-12 (40) | Urethane resin 1 (60) | EDE (60) | Carbon black (8) |
| | | | Cyclopentanone (47) | |
| Bio-electrode solution 30 | Ionic resin 3-13 (100) | - | EDE (60) | Irgacure TPO (1) |
| | | | | Polyglycerin compound 1 (8) |
| | | | Cyclopentanone (47) | |
| | | | | Lithium titanate (8) |
| Bio-electrode solution 31 | Ionic resin 3-14 (20) | Urethane resin 1 (60) | EDE (60) | Carbon black (6) |
| | | | Cyclopentanone (47) | Polyglycerin compound 1 (8) |

**[Table 3]**

| Bio-electrode solution | Ionic resin (parts by mass) | Resin (parts by mass) | Organic solvent (parts by mass) | Additive (parts by mass) |
|---|---|---|---|---|
| Comparative bio-electrode solution 1 | Comparative ionic resin 1 (20) | Siloxane compound 1 (40) | ISOPAR G (60) | CAT-PL-50T (1.5) |
| | | Siloxane compound 2 (100) | | |
| | | Siloxane compound 4 (3) | Cyclopentanone (47) | Carbon black (8) |

### (Evaluation of Biological Signals)

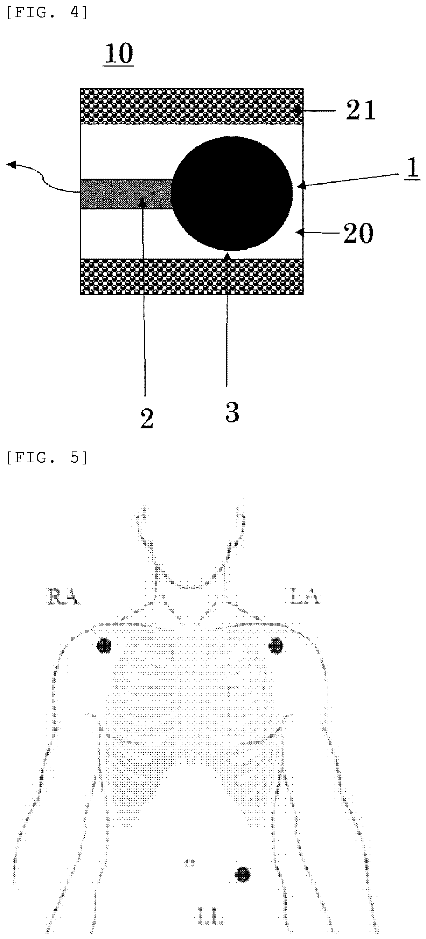

As shown in FIG. 3, a thermoplastic urethane (TPU) film 20 (ST-604, manufactured by Bemis Associates Inc.) was coated with an electro-conductive paste (DOTITE FA-333, manufactured by Fujikura Kasei Co., Ltd.) by screen printing. The coating film was baked in an oven at 120°C for 10 minutes to print a keyhole-shaped electro-conductive pattern 2 having a circular portion with a diameter of 2 cm. Then, one of the bio-electrode solutions shown in Tables 1 to 3 was applied onto the circular portion by screen printing. After being air-dried at room temperature for 10 minutes, the coating film was baked using an oven at 125°C for 10 minutes to evaporate the solvent, and the resultant was cured to form the living body contact layer 3 to prepare the bio-electrode 1. In the case of bio-electrode solution 30, the composition-coated film was cured by light irradiating of 500 mJ/cm² with a 1,000 w xenon lamp under a nitrogen atmosphere. Next as shown FIG. 4, the urethane film having the bio-electrodes printed thereon was cut out and double-sided tapes 21 were attached the film. Thus, three bio-electrode samples 10 were prepared for each of the bio-electrode solutions.

### (Measurement of Thickness of Living Body Contact Layer)

For the bio-electrode produced as described above, the thickness of the living body contact layer was measured by using a micrometer. Table 4 shows the results.

### (Biological Signal Measurement)

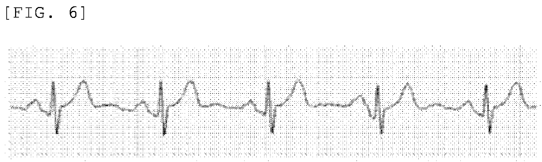

The electro-conductive wiring pattern formed from the electro-conductive paste of each bio-electrode was connected to a portable electrocardiograph (HCG-901, manufactured by OMRON HEALTHCARE Co., Ltd.) through an electro-conductive wire. The positive electrode of the electrocardiograph was attached to the location LA in FIG. 5 on a human body, the negative electrode was attached to the location LL, and the earth was attached to the location RA. Immediately after the attachments, the electrocardiogram measurement was started to measure the time until an electrocardiogram waveform including P, Q, R, S, and T waves appeared as shown in FIG. 6. Table 4 shows the result.

**[Table 4]**

| Example | Bio-electrode solution | Resin thickness (µm) | Time (min.) until ECG signal appeared |
|---|---|---|---|
| Example 1 | Bio-electrode solution 1 | 34 | 1 |
| Example 2 | Bio-electrode solution 2 | 40 | 3 |
| Example 3 | Bio-electrode solution 3 | 48 | 4 |
| Example 4 | Bio-electrode solution 4 | 42 | 5 |
| Example 5 | Bio-electrode solution 5 | 41 | 1 |
| Example 6 | Bio-electrode solution 6 | 46 | 3 |
| Example 7 | Bio-electrode solution 7 | 43 | 1 |
| Example 8 | Bio-electrode solution 8 | 49 | 1 |
| Example 9 | Bio-electrode solution 9 | 49 | 1 |
| Example 10 | Bio-electrode solution 10 | 46 | 0 |
| Example 11 | Bio-electrode solution 11 | 52 | 1 |
| Example 12 | Bio-electrode solution 12 | 48 | 2 |
| Example 13 | Bio-electrode solution 13 | 56 | 4 |
| Example 14 | Bio-electrode solution 14 | 58 | 0 |
| Example 15 | Bio-electrode solution 15 | 44 | 2 |
| Example 16 | Bio-electrode solution 16 | 55 | 4 |
| Example 17 | Bio-electrode solution 17 | 48 | 1 |
| Example 18 | Bio-electrode solution 18 | 55 | 1 |
| Example 19 | Bio-electrode solution 19 | 60 | 0 |
| Example 20 | Bio-electrode solution 20 | 56 | 1 |
| Example 21 | Bio-electrode solution 21 | 47 | 3 |
| Example 22 | Bio-electrode solution 22 | 54 | 6 |
| Example 23 | Bio-electrode solution 23 | 56 | 4 |
| Example 24 | Bio-electrode solution 24 | 49 | 3 |
| Example 25 | Bio-electrode solution 25 | 48 | 2 |
| Example 26 | Bio-electrode solution 26 | 52 | 1 |
| Example 27 | Bio-electrode solution 27 | 53 | 1 |
| Example 28 | Bio-electrode solution 28 | 56 | 3 |
| Example 29 | Bio-electrode solution 29 | 55 | 2 |
| Example 30 | Bio-electrode solution 30 | 59 | 10 |
| Example 31 | Bio-electrode solution 31 | 60 | 2 |
| Comparative Example 1 | Comparative bio-electrode solution 1 | 44 | not appeared |

As shown in Table 4, biological signals were obtained within a short time after the attachment to the body in Examples 1 to 31, in which the living body contact layers were each formed using the inventive bio-electrode composition blended with the resin having a structure selected from the group consisting of an ammonium salt, a lithium salt, a sodium salt, a potassium salt, and silver salt formed with trifluoromethoxybenzenesulfonamide and/or difluoromethoxybenzenesulfonamide. In contrast, no biological signals were obtained in the case in which an ion component having a specific structure was not contained.

The present description includes the following inventions.

[1]: A bio-electrode composition comprising an (A) ionic resin, wherein the component (A) contains a resin having a structure selected from the group consisting of an ammonium salt, a lithium salt, a sodium salt, a potassium salt, and a silver salt formed with trifluoromethoxybenzenesulfonamide and/or difluoromethoxybenzenesulfonamide.
[2]: The bio-electrode composition of the above [1], wherein the resin having a structure selected from the group consisting of an ammonium salt, a lithium salt, a sodium salt, a potassium salt, and silver salt formed with trifluoromethoxybenzenesulfonamide and/or difluoromethoxybenzenesulfonamide has a chemical structure represented by the following general formula (1) , 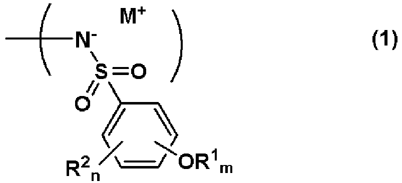 wherein R¹ represents a trifluoromethyl group or a difluoromethyl group; R² represents a hydrogen atom, a linear, branched, or cyclic alkyl group or alkoxy group having 1 to 6 carbon atoms, a hydroxy group, a carboxyl group, or a halogen atom; "m" represents an integer of 1 to 5; "n" represent an integer of 0 to 4; and M⁺ represents an ammonium ion, a lithium ion, a sodium ion, a potassium ion, or a silver ion.
[3]: The bio-electrode composition of the above [2], wherein the chemical structure represented by the general formula (1) bonds to a resin selected from the group consisting of a resin made by polymerizing a monomer having a double bond, a silicone resin, and a polyurethane resin.
[4]: The bio-electrode composition of the above [2] or [3], wherein the resin bonded to the chemical structure represented by the general formula (1) is any one of: a resin having a repeating unit represented by the following general formula (2)-1 and being made by polymerizing a monomer having a double bond; a silicone resin selected from the group consisting of a silicone resin having a repeating unit represented by the following general formula (2)-2-1, a silicone resin represented by the following general formula (2)-2-2, a silicone resin represented by the following general formula (2)-2-3, and a silicone resin having a repeating unit represented by the following general formula (2)-2-4; a polyurethane resin having a repeating unit represented by the following general formula (2)-3-1; and a urethane resin having a terminal structure represented by the following general formula (2)-3-2: 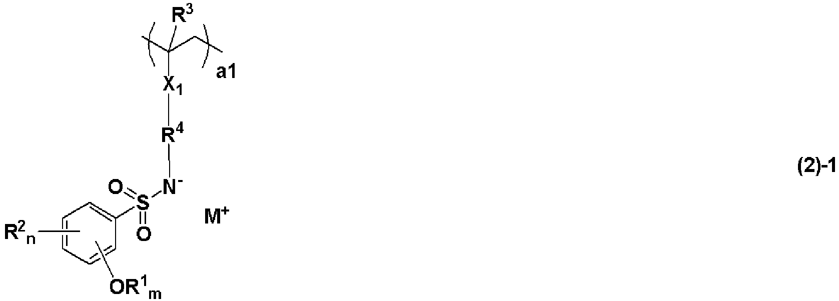 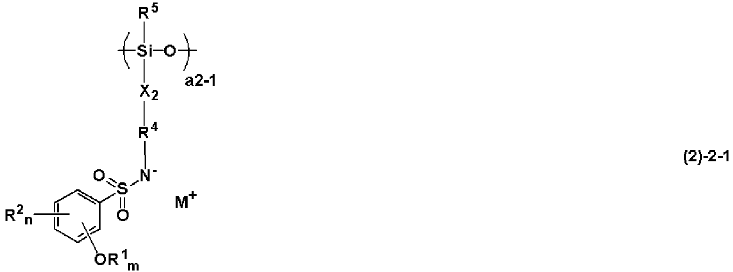 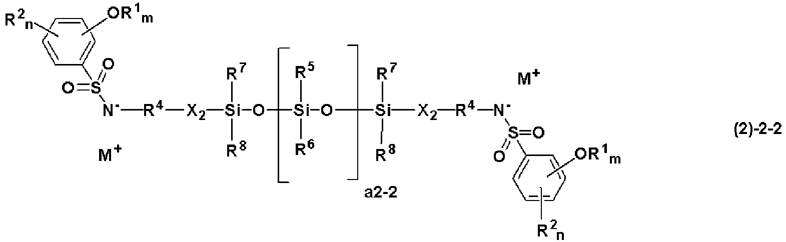 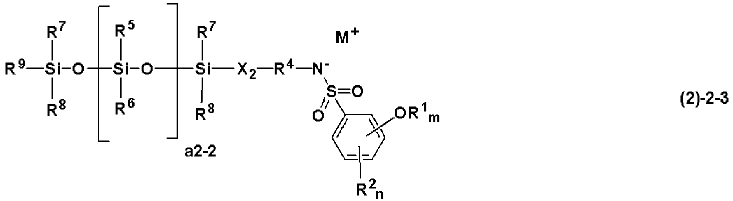 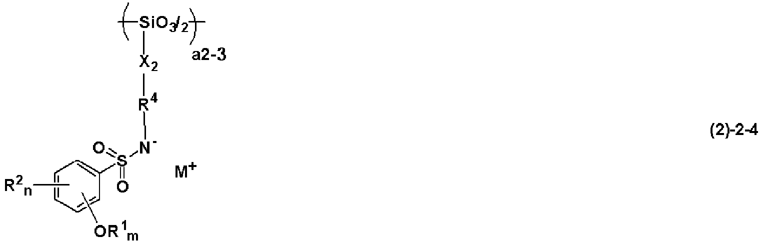 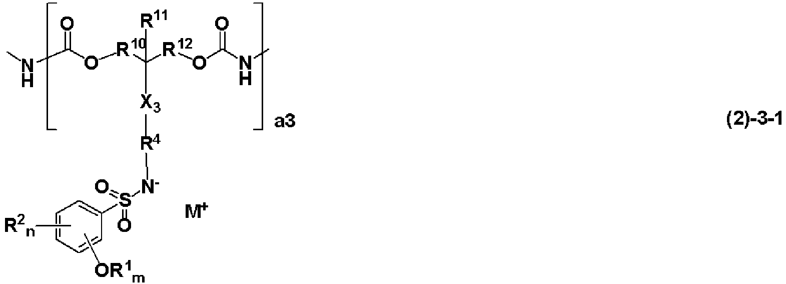 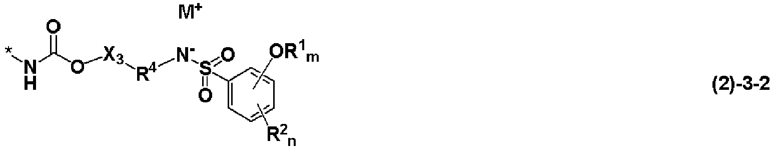 Wherein, R¹, R², "m", "n", and M⁺ are as described above; R³ is a hydrogen atom or a methyl group; R⁴ is a single bond, a linear, branched, or cyclic alkylene group having 1 to 15 carbon atoms, or a divalent hydrocarbon group, and may have an aromatic group, an ester group, an ether group, a carbonyl group, a carbon-carbon double bond, a sulfonyl group, or a sulfonic acid ester group; X₁ is a single bond, a phenylene group, an ester group, a carbonyl group, a sulfonyl group, or a sulfonic acid ester group; X₂ is a linear, branched, or cyclic alkylene group having 1 to 14 carbon atoms, an arylene group having 6 to 12 carbon atoms, or a bonded substance thereof, and may have an ether group, an ester group, a carbonyl group, a nitrogen atom, a sulfide group, a sulfonyl group, or a sulfonic acid ester group; R⁵ to R⁸ are a linear, branched, or cyclic alkyl group having 1 to 66 carbon atoms, or an aryl group having 6 to 10 carbon atoms; R⁹ is a linear or branched alkyl group having 1 to 20 carbon atoms, and may have an ether group or an ester group; R¹⁰ and R¹² are a single bond or a linear or branched alkylene group having 1 to 4 carbon atoms, the alkylene group may have an oxygen atom, and there is no case where both of R¹⁰ and R¹² are single bonds; R¹¹ is a hydrogen atom or a linear or branched alkyl group having 1 to 4 carbon atoms, and may have a hydroxyl group or an ether group; alternatively, R¹⁰ to R¹² and the carbon atom to which they are bonded in the formula may be combined to form a trivalent hydrocarbon group having 6 to 15 carbon atoms, and the trivalent hydrocarbon group may have an aromatic ring and/or a heteroatom; X₃ is a single bond, a carbonyl group, an ester group, or a linear, branched, or cyclic alkylene group having 1 to 10 carbon atoms, may have an aromatic compound, and may be bonded with R¹¹ to form a ring, which may be an aromatic ring; "a1" represents a ratio of repeating units, and satisfies 0<a1≤1.0; "a2-1" and "a2-3" represent a ratio of repeating units, and satisfies 0<a2-1≤1.0 and 0<a2-3≤1.0; "a2-2" represents the number of repeating units and is a number of 0 or more; and "a3" represents the number of repeating units and is a number greater than 0.
[5]: The bio-electrode composition of any one of the above [2] to [4], wherein the component (A) comprises an ammonium ion represented by the following general formula (3) as the M⁺, 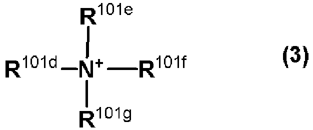 wherein, R^{101d}, R^{101e}, R^{101f}, and R^{101g} are each a hydrogen atom, a linear, branched, or cyclic alkyl group having 1 to 12 carbon atoms, a linear, branched, or cyclic alkenyl or alkynyl group having 2 to 12 carbon atoms, or an aromatic group having 4 to 20 carbon atoms, and may have one or more kinds selected from the group consisting of an ether group, a carbonyl group, an ester group, a hydroxy group, an amino group, a nitro group, a sulfonyl group, a sulfinyl group, a halogen atom, and a sulfur atom; R^{101d} and R^{101e}, or R^{101d}, R^{101e}, and R^{101f} may form a ring together with the nitrogen atom to which they are bonded, and when they form a ring, R^{101d} and R^{101e}, and R^{101d}, R^{101e}, and R^{101f} are alkylene groups having 3 to 10 carbon atoms, or form a heteroaromatic ring having the nitrogen atom described in the formula in the ring.
[6]: The bio-electrode composition of any one of the above [1] to [5], further containing a resin as a component (B), which is different from the ionic resin as the component (A).
[7]: The bio-electrode composition of the above [6], wherein, the component (B) is one or more kinds selected from the group consisting of a silicone resin, a (meta) acrylate resin, and a urethane resin.
[8]: The bio-electrode composition of the above [6] or [7], wherein, the resin as the component (B) has adhesiveness.
[9]: The bio-electrode composition of any one of the above [6] to [8], further comprising as the component (B) a silicone resin having RₓSiO_{(4-x)/2} unit and SiO₂ unit, wherein R is a substituted or unsubstituted monovalent hydrocarbon group having 1 to 10 carbon atoms and x is in a range of 2.5 to 3.5.
[10]: The bio-electrode composition of any one of the above [1] to [9], further comprising carbon powder and/or metal powder as a component (C).
[11]: The bio-electrode composition of the above [10], wherein the carbon powder is either or both of carbon black and carbon nanotube.
[12]: The bio-electrode composition of the above [10] or [11], wherein the metal powder is selected from the group consisting of gold, silver, platinum, copper, tin, titanium, nickel, aluminum, tungsten, molybdenum, ruthenium, chromium, and indium.
[13]: The bio-electrode composition of any one of the above [10] to [12], wherein the metal powder is silver powder.
[14]: The bio-electrode composition of any one of the above [1] to [13], further comprising an organic solvent as a component (D).
[15]: A bio-electrode comprising a conductive substrate and a living body contact layer formed on the conductive substrate, wherein the living body contact layer is a cured product of the bio-electrode composition according to any one of the above [1] to [14].
[16]: The bio-electrode of the above [15], wherein the conductive substrate comprises one or more kinds selected from the group consisting of gold, silver, silver chloride, platinum, aluminum, magnesium, tin, tungsten, iron, copper, nickel, stainless steel, chromium, titanium, carbon, and conductive polymers.
[17]: A method for manufacturing a bio-electrode having a conductive substrate and a living body contact layer formed on the conductive substrate, comprising: applying the bio-electrode composition of any one of the above [1] to [14] onto the conductive substrate; and curing the bio-electrode composition to form the living body contact layer.
[18]: The method for manufacturing a bio-electrode of the above [17], wherein the conductive substrate comprises one or more kinds selected from the group consisting of gold, silver, silver chloride, platinum, aluminum, magnesium, tin, tungsten, iron, copper, nickel, stainless steel, chromium, titanium, carbon, and conductive polymers.

## Claims

1. A bio-electrode composition comprising an (A) ionic resin, wherein
the component (A) contains a resin having a structure selected from the group consisting of an ammonium salt, a lithium salt, a sodium salt, a potassium salt, and a silver salt formed with trifluoromethoxybenzenesulfonamide and/or difluoromethoxybenzenesulfonamide.

2. The bio-electrode composition according to claim 1, wherein the resin having a structure selected from the group consisting of an ammonium salt, a lithium salt, a sodium salt, a potassium salt, and silver salt formed with trifluoromethoxybenzenesulfonamide and/or difluoromethoxybenzenesulfonamide has a chemical structure represented by the following general formula (1), 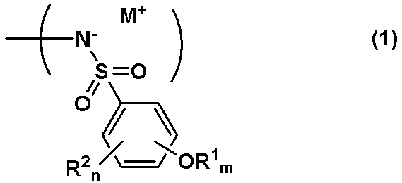 wherein R¹ represents a trifluoromethyl group or a difluoromethyl group; R² represents a hydrogen atom, a linear, branched, or cyclic alkyl group or alkoxy group having 1 to 6 carbon atoms, a hydroxy group, a carboxyl group, or a halogen atom; "m" represents an integer of 1 to 5; "n" represent an integer of 0 to 4; and M⁺ represents an ammonium ion, a lithium ion, a sodium ion, a potassium ion, or a silver ion.

3. The bio-electrode composition according to claim 2, wherein the chemical structure represented by the general formula (1) bonds to a resin selected from the group consisting of a resin made by polymerizing a monomer having a double bond, a silicone resin, and a polyurethane resin.

4. The bio-electrode composition according to claim 3, wherein the resin bonded to the chemical structure represented by the general formula (1) is any one of: a resin having a repeating unit represented by the following general formula (2)-1 and being made by polymerizing a monomer having a double bond; a silicone resin selected from the group consisting of a silicone resin having a repeating unit represented by the following general formula (2)-2-1, a silicone resin represented by the following general formula (2)-2-2, a silicone resin represented by the following general formula (2)-2-3, and a silicone resin having a repeating unit represented by the following general formula (2)-2-4; a polyurethane resin having a repeating unit represented by the following general formula (2)-3-1; and a urethane resin having a terminal structure represented by the following general formula (2)-3-2: 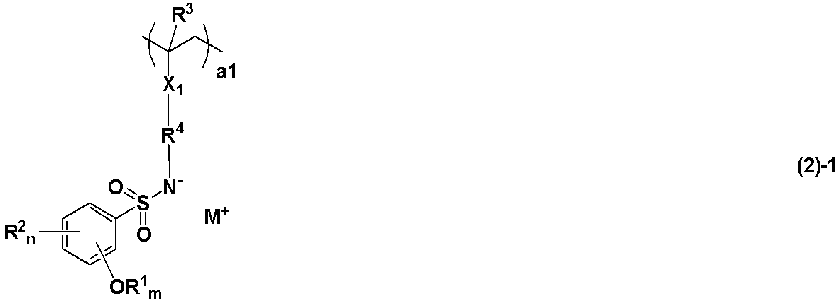 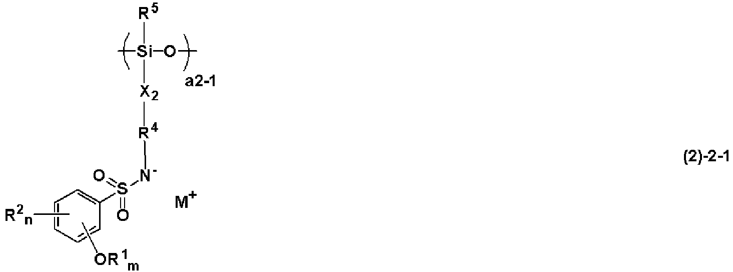 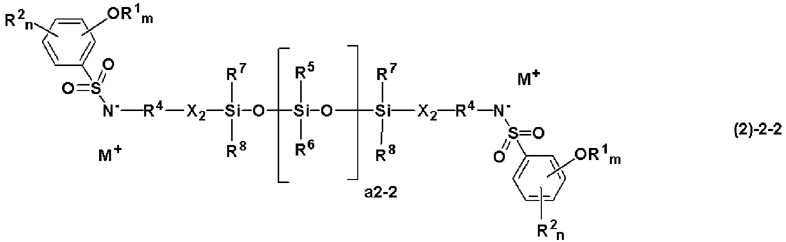 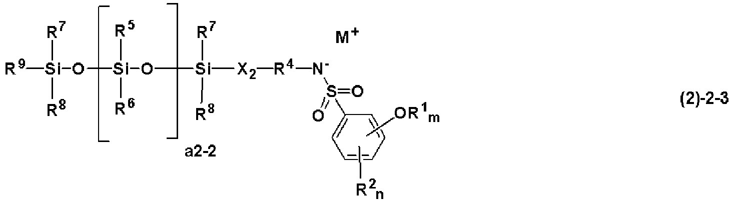 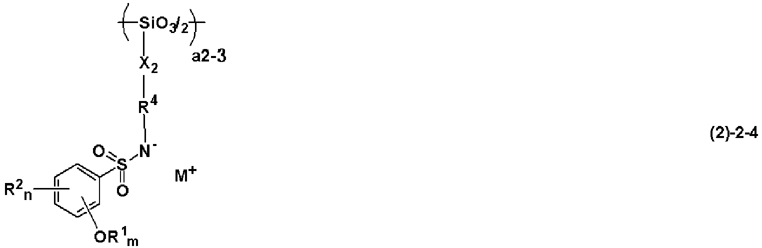 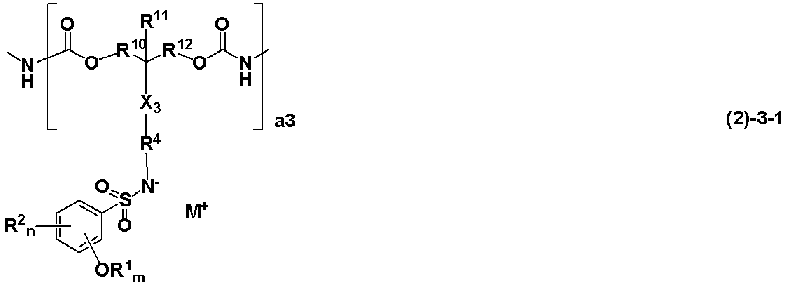 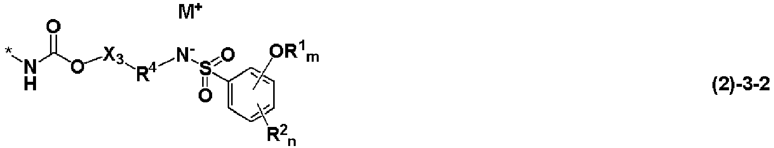 Wherein, R¹, R², "m", "n", and M⁺ are as described above; R³ is a hydrogen atom or a methyl group; R⁴ is a single bond, a linear, branched, or cyclic alkylene group having 1 to 15 carbon atoms, or a divalent hydrocarbon group, and may have an aromatic group, an ester group, an ether group, a carbonyl group, a carbon-carbon double bond, a sulfonyl group, or a sulfonic acid ester group; X₁ is a single bond, a phenylene group, an ester group, a carbonyl group, a sulfonyl group, or a sulfonic acid ester group; X₂ is a linear, branched, or cyclic alkylene group having 1 to 14 carbon atoms, an arylene group having 6 to 12 carbon atoms, or a bonded substance thereof, and may have an ether group, an ester group, a carbonyl group, a nitrogen atom, a sulfide group, a sulfonyl group, or a sulfonic acid ester group; R⁵ to R⁸ are a linear, branched, or cyclic alkyl group having 1 to 66 carbon atoms, or an aryl group having 6 to 10 carbon atoms; R⁹ is a linear or branched alkyl group having 1 to 20 carbon atoms, and may have an ether group or an ester group; R¹⁰ and R¹² are a single bond or a linear or branched alkylene group having 1 to 4 carbon atoms, the alkylene group may have an oxygen atom, and there is no case where both of R¹⁰ and R¹² are single bonds; R¹¹ is a hydrogen atom or a linear or branched alkyl group having 1 to 4 carbon atoms, and may have a hydroxyl group or an ether group; alternatively, R¹⁰ to R¹² and the carbon atom to which they are bonded in the formula may be combined to form a trivalent hydrocarbon group having 6 to 15 carbon atoms, and the trivalent hydrocarbon group may have an aromatic ring and/or a heteroatom; X₃ is a single bond, a carbonyl group, an ester group, or a linear, branched, or cyclic alkylene group having 1 to 10 carbon atoms, may have an aromatic compound, and may be bonded with R¹¹ to form a ring, which may be an aromatic ring; "a1" represents a ratio of repeating units, and satisfies 0<a1≤1.0; "a2-1" and "a2-3" represent a ratio of repeating units, and satisfies 0<a2-1≤1.0 and 0<a2-3≤1.0; "a2-2" represents the number of repeating units and is a number of 0 or more; and "a3" represents the number of repeating units and is a number greater than 0.

5. The bio-electrode composition according to claim 2, wherein the component (A) comprises an ammonium ion represented by the following general formula (3) as the M⁺, 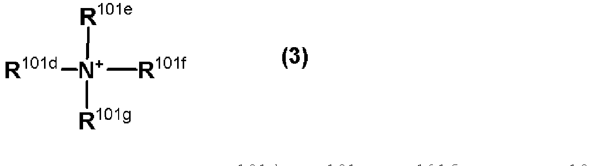 wherein, R^{101d}, R1^{101e}, R^{101f}, and R^{101g} are each a hydrogen atom, a linear, branched, or cyclic alkyl group having 1 to 12 carbon atoms, a linear, branched, or cyclic alkenyl or alkynyl group having 2 to 12 carbon atoms, or an aromatic group having 4 to 20 carbon atoms, and may have one or more kinds selected from the group consisting of an ether group, a carbonyl group, an ester group, a hydroxy group, an amino group, a nitro group, a sulfonyl group, a sulfinyl group, a halogen atom, and a sulfur atom; R^{101d} and R^{101e}, or R^{101d}, R^{101e}, and R^{101f} may form a ring together with the nitrogen atom to which they are bonded, and when they form a ring, R^{101d} and R^{101e}, and R^{101d}, R^{101e}, and R^{101f} are alkylene groups having 3 to 10 carbon atoms, or form a heteroaromatic ring having the nitrogen atom described in the formula in the ring.

6. The bio-electrode composition according to claim 1, further containing a resin as a component (B), which is different from the ionic resin as the component (A).

7. The bio-electrode composition according to claim 6, wherein, the component (B) is one or more kinds selected from the group consisting of a silicone resin, a (meta) acrylate resin, and a urethane resin.

8. The bio-electrode composition according to claim 6, wherein, the resin as the component (B) has adhesiveness.

9. The bio-electrode composition according to claim 7, further comprising as the component (B) a silicone resin having RₓSiO(_{4-x)/2} unit and SiO₂ unit, wherein R is a substituted or unsubstituted monovalent hydrocarbon group having 1 to 10 carbon atoms and x is in a range of 2.5 to 3.5.

10. The bio-electrode composition according to claim 6, further comprising carbon powder and/or metal powder as a component (C).

11. The bio-electrode composition according to claim 10, wherein the carbon powder is either or both of carbon black and carbon nanotube.

12. The bio-electrode composition according to claim 10, wherein the metal powder is selected from the group consisting of gold, silver, platinum, copper, tin, titanium, nickel, aluminum, tungsten, molybdenum, ruthenium, chromium, and indium, preferably,
wherein the metal powder is silver powder.

13. The bio-electrode composition according to claim 6, further comprising an organic solvent as a component (D).

14. A bio-electrode comprising a conductive substrate and a living body contact layer formed on the conductive substrate, wherein the living body contact layer is a cured product of the bio-electrode composition according to any one of claims 1 to 13, preferably,
wherein the conductive substrate comprises one or more kinds selected from the group consisting of gold, silver, silver chloride, platinum, aluminum, magnesium, tin, tungsten, iron, copper, nickel, stainless steel, chromium, titanium, carbon, and conductive polymers.

15. A method for manufacturing a bio-electrode having a conductive substrate and a living body contact layer formed on the conductive substrate, comprising:
applying the bio-electrode composition according to any one of claim 1 to 13 onto the conductive substrate; and
curing the bio-electrode composition to form the living body contact layer, preferably,
wherein the conductive substrate comprises one or more kinds selected from the group consisting of gold, silver, silver chloride, platinum, aluminum, magnesium, tin, tungsten, iron, copper, nickel, stainless steel, chromium, titanium, carbon, and conductive polymers.

## Patentansprüche

1. Bioelektrodenzusammensetzung, umfassend ein (A) ionisches Harz, wobei
die Komponente (A) ein Harz mit einer Struktur enthält, ausgewählt aus der Gruppe bestehend aus einem Ammoniumsalz, einem Lithiumsalz, einem Natriumsalz, einem Kaliumsalz und einem Silbersalz, gebildet mit Trifluormethoxybenzolsulfonamid und/oder Difluormethoxybenzolsulfonamid.

2. Bioelektrodenzusammensetzung gemäß Anspruch 1, wobei das Harz mit einer Struktur, ausgewählt aus der Gruppe bestehend aus einem Ammoniumsalz, einem Lithiumsalz, einem Natriumsalz, einem Kaliumsalz und einem Silbersalz, gebildet mit Trifluormethoxybenzolsulfonamid und/oder Difluormethoxybenzolsulfonamid, eine chemische Struktur aufweist, die durch die folgende allgemeine Formel (1) dargestellt wird, 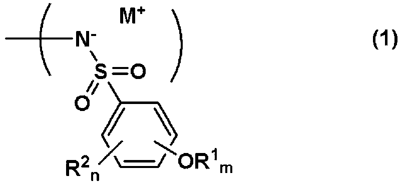 wobei R¹ eine Trifluormethylgruppe oder eine Difluormethylgruppe darstellt; R² ein Wasserstoffatom, eine lineare, verzweigte oder cyclische Alkylgruppe oder Alkoxygruppe mit 1 bis 6 Kohlenstoffatomen, eine Hydroxygruppe, eine Carboxylgruppe oder ein Halogenatom darstellt; "m" eine ganze Zahl von 1 bis 5 darstellt; "n" eine ganze Zahl von 0 bis 4 darstellt; und M⁺ ein Ammoniumion, ein Lithiumion, ein Natriumion, ein Kaliumion oder ein Silberion darstellt.

3. Bioelektrodenzusammensetzung gemäß Anspruch 2, wobei die durch die allgemeine Formel (1) dargestellte chemische Struktur an ein Harz gebunden ist, ausgewählt aus der Gruppe bestehend aus einem Harz, das durch Polymerisation eines Monomers mit einer Doppelbindung hergestellt wird, einem Silikonharz und einem Polyurethanharz.

4. Bioelektrodenzusammensetzung gemäß Anspruch 3, wobei das an die durch die allgemeine Formel (1) dargestellte chemische Struktur gebundene Harz eines der folgenden ist: ein Harz mit einer durch die folgende allgemeine Formel (2)-1 dargestellten Wiederholungseinheit, das durch Polymerisation eines Monomers mit einer Doppelbindung hergestellt wird; ein Silikonharz, ausgewählt aus der Gruppe bestehend aus einem Silikonharz mit einer Wiederholungseinheit, die durch die folgende allgemeine Formel (2)-2-1 dargestellt ist, einem Silikonharz, das durch die folgende allgemeine Formel (2)-2-2 dargestellt ist, einem Silikonharz, das durch die folgende allgemeine Formel (2)-2-3 dargestellt ist, und einem Silikonharz mit einer Wiederholungseinheit, die durch die folgende allgemeine Formel (2)-2-4 dargestellt ist; ein Polyurethanharz mit einer Wiederholungseinheit, die durch die folgende allgemeine Formel (2)-3-1 dargestellt ist; und ein Urethanharz mit einer Endstruktur, die durch die folgende allgemeine Formel (2)-3-2 dargestellt ist: 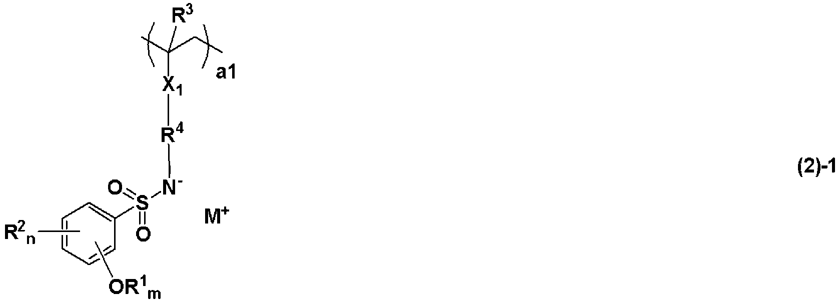 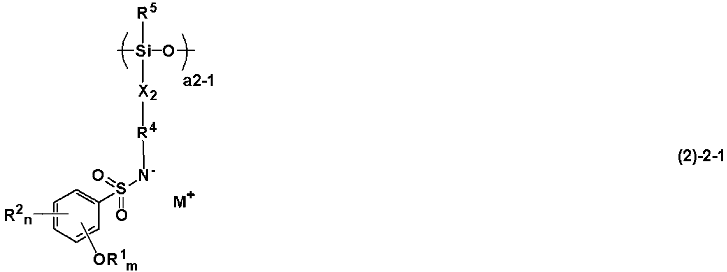 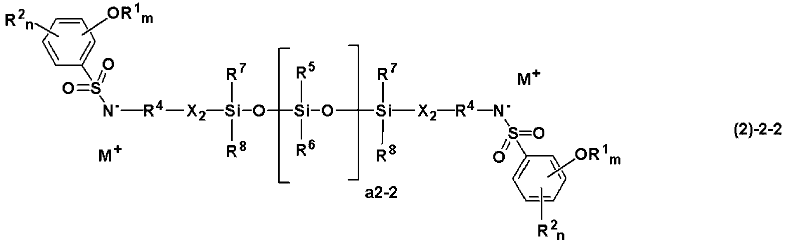 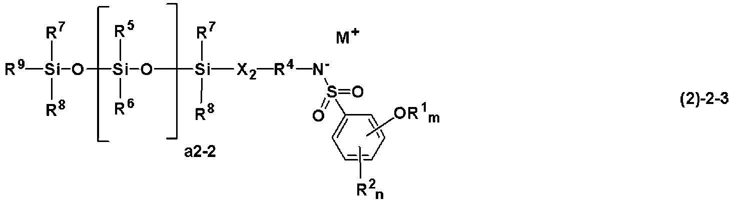 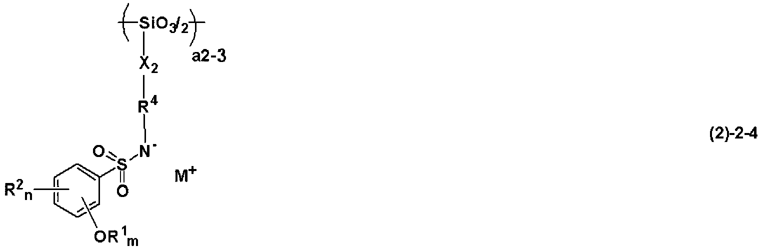 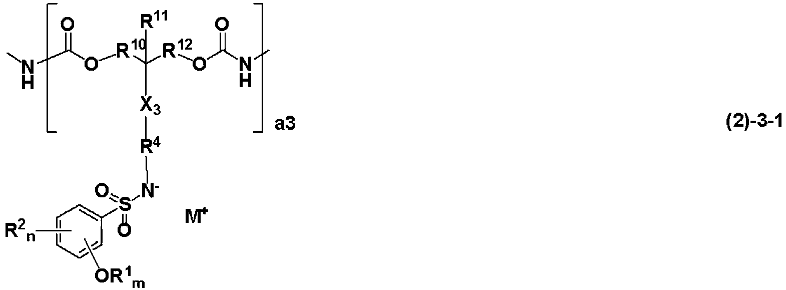 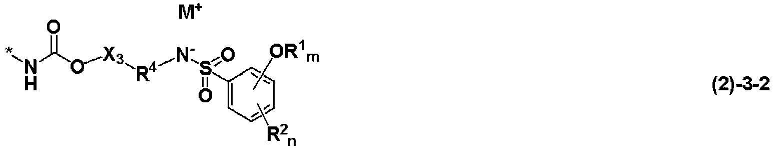 wobei R¹, R², "m", "n" und M⁺ wie oben beschrieben sind; R³ ein Wasserstoffatom oder eine Methylgruppe ist; R⁴ eine Einfachbindung, eine lineare, verzweigte oder cyclische Alkylengruppe mit 1 bis 15 Kohlenstoffatomen oder eine zweiwertige Kohlenwasserstoffgruppe ist und eine aromatische Gruppe, eine Estergruppe, eine Ethergruppe, eine Carbonylgruppe, eine Kohlenstoff-Kohlenstoff-Doppelbindung, eine Sulfonylgruppe oder eine Sulfonsäureestergruppe aufweisen kann; X₁ eine Einfachbindung, eine Phenylengruppe, eine Estergruppe, eine Carbonylgruppe, eine Sulfonylgruppe oder eine Sulfonsäureestergruppe ist; X₂ eine lineare, verzweigte oder cyclische Alkylengruppe mit 1 bis 14 Kohlenstoffatomen, eine Arylengruppe mit 6 bis 12 Kohlenstoffatomen oder eine gebundene Substanz davon ist und eine Ethergruppe, eine Estergruppe, eine Carbonylgruppe, ein Stickstoffatom, eine Sulfidgruppe, eine Sulfonylgruppe oder eine Sulfonsäureestergruppe aufweisen kann; R⁵ bis R⁸ eine lineare, verzweigte oder cyclische Alkylgruppe mit 1 bis 66 Kohlenstoffatomen oder eine Arylgruppe mit 6 bis 10 Kohlenstoffatomen sind; R⁹ eine lineare oder verzweigte Alkylgruppe mit 1 bis 20 Kohlenstoffatomen ist und eine Ethergruppe oder eine Estergruppe aufweisen kann; R¹⁰ und R¹² eine Einfachbindung oder eine lineare oder verzweigte Alkylengruppe mit 1 bis 4 Kohlenstoffatomen sind, wobei die Alkylengruppe ein Sauerstoffatom aufweisen kann und es keinen Fall gibt, in dem sowohl R¹⁰ als auch R¹² Einfachbindungen sind; R¹¹ ein Wasserstoffatom oder eine lineare oder verzweigte Alkylgruppe mit 1 bis 4 Kohlenstoffatomen ist und eine Hydroxylgruppe oder eine Ethergruppe aufweisen kann; R¹⁰ bis R¹² und das Kohlenstoffatom, an das sie in der Formel gebunden sind, alternativ zu einer dreiwertigen Kohlenwasserstoffgruppe mit 6 bis 15 Kohlenstoffatomen kombiniert sein können, und die dreiwertige Kohlenwasserstoffgruppe einen aromatischen Ring und/oder ein Heteroatom aufweisen kann; X₃ eine Einfachbindung, eine Carbonylgruppe, eine Estergruppe oder eine lineare, verzweigte oder cyclische Alkylengruppe mit 1 bis 10 Kohlenstoffatomen ist, eine aromatische Verbindung aufweisen kann und mit R¹¹ verbunden sein kann, um einen Ring zu bilden, der ein aromatischer Ring sein kann; "a1" ein Verhältnis von Wiederholungseinheiten darstellt und 0<a1≤1,0 erfüllt; "a2-1" und "a2-3" ein Verhältnis von Wiederholungseinheiten darstellen und 0<a2-1≤1,0 und 0<a2-3≤ 1,0 erfüllen; "a2-2" die Anzahl der Wiederholungseinheiten darstellt und eine Zahl von 0 oder mehr ist; und "a3" die Anzahl der Wiederholungseinheiten darstellt und eine Zahl größer als 0 ist.

5. Bioelektrodenzusammensetzung gemäß Anspruch 2, wobei die Komponente (A) ein Ammoniumion als das M⁺ umfasst, dargestellt durch die folgende allgemeine Formel (3): 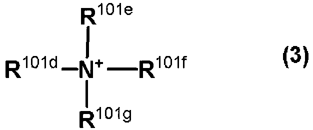 wobei R^{101d} , R^{101e} , R^{101f} und R^{101g} jeweils ein Wasserstoffatom, eine lineare, verzweigte oder cyclische Alkylgruppe mit 1 bis 12 Kohlenstoffatomen, eine lineare, verzweigte cyclische Alkenyl- oder Alkinylgruppe mit 2 bis 12 Kohlenstoffatomen oder eine aromatische Gruppe mit 4 bis 20 Kohlenstoffatomen sind und eine oder mehrere Arten aufweisen können, ausgewählt aus der Gruppe bestehend aus einer Ethergruppe, einer Carbonylgruppe, einer Estergruppe, einer Hydroxygruppe, einer Aminogruppe, einer Nitrogruppe, einer Sulfonylgruppe, einer Sulfinylgruppe, einem Halogenatom und einem Schwefelatom; R^{101d} und R^{101e} oder R^{101d}, R^{101e} und R^{101f} zusammen mit dem Stickstoffatom, an das sie gebunden sind, einen Ring bilden können, und wenn sie einen Ring bilden, sind R^{101d} und R^{101e} und R^{101d}, R^{101e} und R^{101f} Alkylengruppen mit 3 bis 10 Kohlenstoffatomen, oder bilden einen heteroaromatischen Ring mit dem in der Formel beschriebenen Stickstoffatom im Ring.

6. Bioelektrodenzusammensetzung gemäß Anspruch 1, ferner enthaltend ein Harz als eine Komponente (B), das sich von dem ionischen Harz als die Komponente (A) unterscheidet.

7. Bioelektrodenzusammensetzung gemäß Anspruch 6, wobei die Komponente (B) eine oder mehrere Arten ist, ausgewählt aus der Gruppe bestehend aus einem Silikonharz, einem (Meta)acrylatharz und einem Urethanharz.

8. Bioelektrodenzusammensetzung gemäß Anspruch 6, wobei das Harz der Komponente (B) Haftfähigkeit aufweist.

9. Bioelektrodenzusammensetzung gemäß Anspruch 7, ferner umfassend ein Silikonharz mit RₓSiO_{(4-x)/2}-Einheiten und SiO₂-Einheiten als die Komponente (B), wobei R eine substituierte oder unsubstituierte einwertige Kohlenwasserstoffgruppe mit 1 bis 10 Kohlenstoffatomen ist und x in einem Bereich von 2,5 bis 3,5 liegt.

10. Bioelektrodenzusammensetzung gemäß Anspruch 6, ferner umfassend Kohlenstoffpulver und/oder Metallpulver als eine Komponente (C).

11. Bioelektrodenzusammensetzung gemäß Anspruch 10, wobei das Kohlenstoffpulver entweder Ruß oder Kohlenstoffnanoröhren oder beides ist.

12. Bioelektrodenzusammensetzung gemäß Anspruch 10, wobei das Metallpulver ausgewählt ist aus der Gruppe bestehend aus Gold, Silber, Platin, Kupfer, Zinn, Titan, Nickel, Aluminium, Wolfram, Molybdän, Ruthenium, Chrom und Indium, vorzugsweise
wobei das Metallpulver Silberpulver ist.

13. Bioelektrodenzusammensetzung gemäß Anspruch 6, ferner umfassend ein organisches Lösungsmittel als eine Komponente (D).

14. Bioelektrode, umfassend ein leitfähiges Substrat und eine auf dem leitfähigen Substrat ausgebildete Lebendkörper-Kontaktschicht, wobei die Lebendkörper-Kontaktschicht ein ausgehärtetes Produkt der Bioelektrodenzusammensetzung gemäß einem der Ansprüche 1 bis 13 ist, vorzugsweise
wobei das leitfähige Substrat eine oder mehrere Arten umfasst, ausgewählt aus der Gruppe bestehend aus Gold, Silber, Silberchlorid, Platin, Aluminium, Magnesium, Zinn, Wolfram, Eisen, Kupfer, Nickel, Edelstahl, Chrom, Titan, Kohlenstoff und leitfähigen Polymeren.

15. Verfahren zur Herstellung einer Bioelektrode mit einem leitfähigen Substrat und einer auf dem leitfähigen Substrat ausgebildeten Lebendkörper-Kontaktschicht, umfassend:
Aufbringen der Bioelektrodenzusammensetzung gemäß einem der Ansprüche 1 bis 13 auf das leitfähige Substrat; und
Aushärten der Bioelektrodenzusammensetzung, um die Lebendkörper-Kontaktschicht zu bilden, vorzugsweise
wobei das leitfähige Substrat eine oder mehrere Arten umfasst, ausgewählt aus der Gruppe bestehend aus Gold, Silber, Silberchlorid, Platin, Aluminium, Magnesium, Zinn, Wolfram, Eisen, Kupfer, Nickel, Edelstahl, Chrom, Titan, Kohlenstoff und leitfähigen Polymeren.

## Revendications

1. Composition de bioélectrode comprenant une résine ionique (A), dans laquelle
le constituant (A) contient une résine dont la structure est choisie dans le groupe constitué d'un sel d'ammonium, d'un sel de lithium, d'un sel de sodium, d'un sel de potassium et d'un sel d'argent pourvu de trifluorométhoxybenzènesulfonamide et/ou de difluorométhoxybenzènesulfonamide.

2. Composition de bioélectrode selon la revendication 1, dans laquelle la résine dont la structure est choisie dans le groupe constitué d'un sel d'ammonium, d'un sel de lithium, d'un sel de sodium, d'un sel de potassium et d'un sel d'argent pourvu de trifluorométhoxybenzènesulfonamide et/ou de difluorométhoxybenzènesulfonamide a une structure chimique représentée par la formule générale suivante (1), 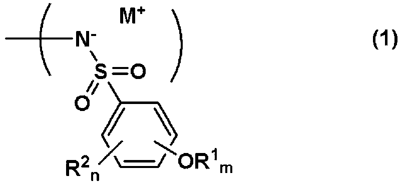 dans laquelle R¹ représente un groupe trifluorométhyle ou un groupe difluorométhyle; R² représente un atome d'hydrogène, un groupe alkyle linéaire, ramifié ou cyclique ou un groupe alcoxy ayant de 1 à 6 atomes de carbone, un groupe hydroxy, un groupe carboxyle ou un atome d'halogène; « m » représente un nombre entier de 1 à 5; « n » représente un nombre entier de 0 à 4; et M⁺ représente un ion ammonium, un ion lithium, un ion sodium, un ion potassium ou un ion argent.

3. Composition de bioélectrode selon la revendication 2, dans laquelle la structure chimique représentée par la formule générale (1) se lie à une résine choisie dans le groupe constitué d'une résine obtenue par polymérisation d'un monomère à double liaison, d'une résine de silicone et d'une résine de polyuréthane.

4. Composition de bioélectrode selon la revendication 3, dans laquelle la résine liée à la structure chimique représentée par la formule générale (1) est l'une parmi: une résine dont l'unité de répétition est représentée par la formule générale suivante (2)-1 et qui est obtenue par polymérisation d'un monomère à double liaison; une résine de silicone choisie dans le groupe constitué par une résine de silicone dont le motif de répétition est représenté par la formule générale suivante (2)-2-1, une résine de silicone représentée par la formule générale suivante (2)-2-2, une résine de silicone représentée par la formule générale suivante (2)-2-3, et une résine de silicone dont le motif de répétition est représentée par la formule générale suivante (2)-2-4; une résine de polyuréthane dont le motif de répétition est représenté par la formule générale suivante (2)-3-1; et une résine d'uréthane dont la structure terminale est représentée par la formule générale suivante (2)-3-2: 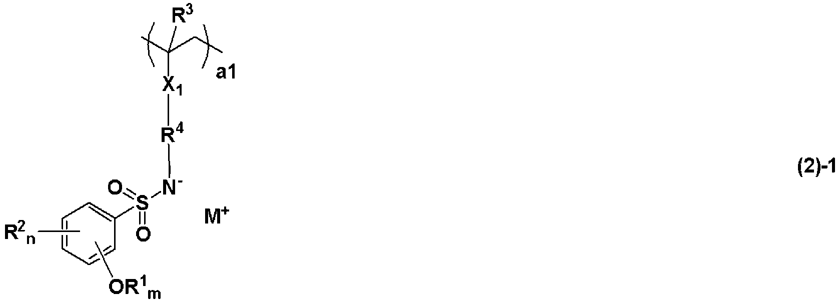 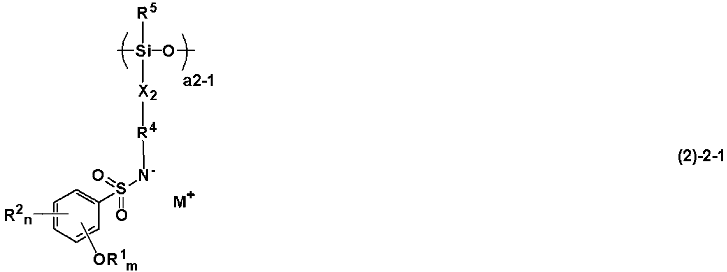 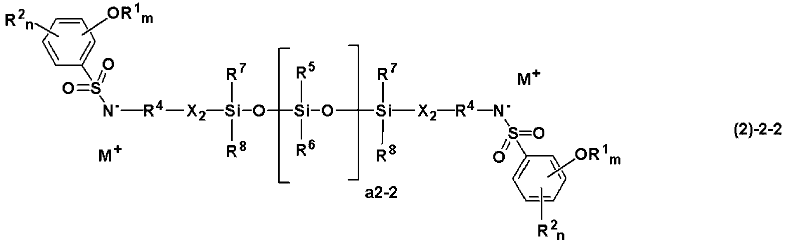 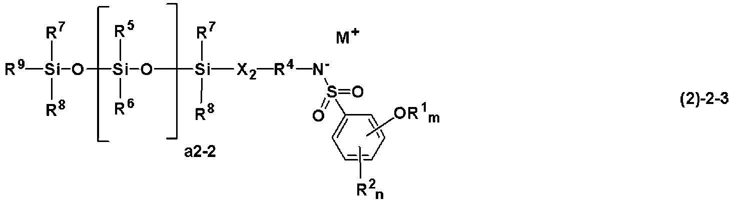 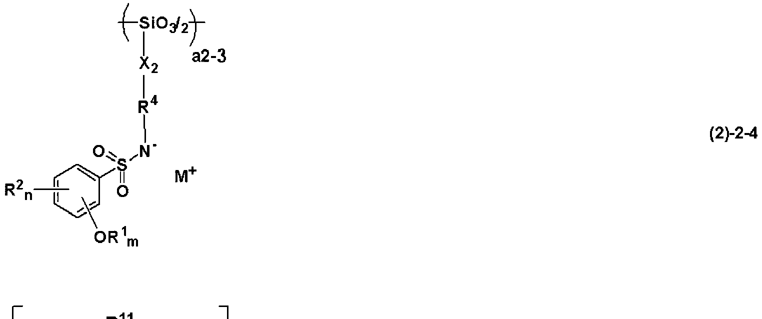 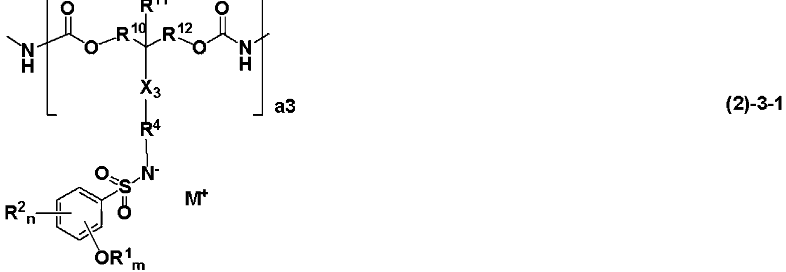 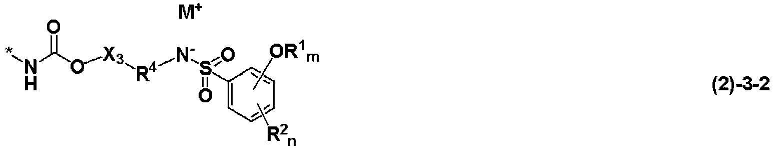 dans laquelle R¹, R², « m », « n », et M⁺ sont tels que décrits ci-dessus; R³ est un atome d'hydrogène ou un groupe méthyle; R⁴ est une liaison simple, un groupe alkylène à liaison simple, linéaire, ramifié ou cyclique ayant de 1 à 15 atomes de carbone, ou un groupe hydrocarbure divalent, et peut avoir un groupe aromatique, un groupe ester, un groupe éther, un groupe carbonyle, une double liaison carbone-carbone, un groupe sulfonyle ou un groupe ester d'acide sulfonique; X₁ est une liaison simple, un groupe phénylène, un groupe ester, un groupe carbonyle, un groupe sulfonyle ou un groupe ester d'acide sulfonique; X₂ est un groupe alkylène linéaire, ramifié ou cyclique ayant de 1 à 14 atomes de carbone, un groupe arylène ayant de 6 à 12 atomes de carbone, ou une substance liée associée, et peut avoir un groupe éther, un groupe ester, un groupe carbonyle, un atome d'azote, un groupe sulfure, un groupe sulfonyle, ou un groupe ester d'acide sulfonique; R⁵ à R⁸ sont chacun un groupe alkyle linéaire, ramifié ou cyclique ayant de 1 à 66 atomes de carbone, ou un groupe aryle ayant de 6 à 10 atomes de carbone; R⁹ est un groupe alkyle linéaire ou ramifié ayant de 1 à 20 atomes de carbone, et peut avoir un groupe éther ou un groupe ester; R¹⁰ et R¹² sont une liaison simple ou un groupe alkylène linéaire ou ramifié ayant de 1 à 4 atomes de carbone, le groupe alkylène peut avoir un atome d'oxygène, et il n'y a aucun cas où R¹⁰ et R¹² sont tous deux des liaisons simples; R¹¹ est un atome d'hydrogène ou un groupe alkyle linéaire ou ramifié ayant de 1 à 4 atomes de carbone, et peut avoir un groupe hydroxyle ou un groupe éther; en variante, R¹⁰ à R¹² et l'atome de carbone auquel ils sont liés dans la formule peuvent être combinés pour former un groupe hydrocarbure trivalent ayant de 6 à 15 atomes de carbone, et le groupe hydrocarbure trivalent peut avoir un noyau aromatique et/ou un hétéroatome; X₃ est une liaison simple, un groupe carbonyle, un groupe ester ou un groupe alkylène linéaire, ramifié ou cyclique ayant de 1 à 10 atomes de carbone, peut avoir un composé aromatique et peut être lié à R¹¹ pour former un noyau, qui peut être un noyau aromatique; « a1 » représente un rapport de motifs de répétition et correspond à 0 < a1 ≤ 1,0; « a2-1 » et « a2-3 » représentent un rapport de motifs de répétition et correspondent à 0 < a2-1 ≤ 1,0 et 0 < a2-3 ≤1,0; « a2-2 » représente le nombre de motifs de répétition et correspond à un nombre égal ou supérieur à 0; et « a3 » représente le nombre de motifs de répétition et est un nombre supérieur à 0.

5. Composition de bioélectrode selon la revendication 2, dans laquelle le constituant (A) comprend un ion ammonium représenté par la formule générale suivante (3) en tant que M⁺, 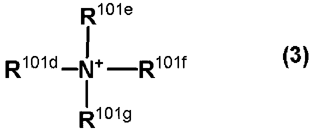 dans laquelle R^{101d}, R^{101e}, R^{101f}, et R^{101g} sont chacun un atome d'hydrogène, un groupe alkyle linéaire, ramifié ou cyclique ayant de 1 à 12 atomes de carbone, un groupe alcényle ou alcynyle linéaire, ramifié ou cyclique ayant de 2 à 12 atomes de carbone, ou un groupe aromatique ayant de 4 à 20 atomes de carbone, et peut avoir un ou plusieurs types choisis dans le groupe constitué d'un groupe éther, d'un groupe carbonyle, d'un groupe ester, d'un groupe hydroxy, d'un groupe amino, d'un groupe nitro, d'un groupe sulfonyle, d'un groupe sulfinyle, d'un atome d'halogène et d'un atome de soufre; R^{101d} et R^{101e}, ou R^{101d}, R^{101e}, et R^{101f} peuvent former un noyau avec l'atome d'azote auquel ils sont liés, et lorsqu'ils forment un noyau, R^{101d} et R^{101e}, et R^{101d}, R^{101e}, et R^{101f} sont des groupes alkylènes ayant de 3 à 10 atomes de carbone, ou forment un noyau hétéroaromatique ayant l'atome d'azote décrit dans la formule dans le noyau.

6. Composition de bioélectrode selon la revendication 1, contenant en outre une résine en tant que constituant (B), qui est différente de la résine ionique en tant que constituant (A).

7. Composition de bioélectrode selon la revendication 6, dans laquelle le constituant (B) est une ou plusieurs sortes choisies dans le groupe constitué d'une résine de silicone, d'une résine de (méta)acrylate et d'une résine d'uréthane.

8. Composition de bioélectrode selon la revendication 6, dans laquelle la résine en tant que constituant (B) est adhésive.

9. Composition de bioélectrode selon la revendication 7, comprenant en outre, en tant que constituant (B), une résine de silicone ayant une unité RₓSiO_{(4-x)/2} et une unité SiO₂, dans laquelle R est un groupe hydrocarbure monovalent, substitué ou non, ayant de 1 à 10 atomes de carbone et x est compris entre 2,5 et 3,5.

10. Composition de bioélectrode selon la revendication 6, comprenant en outre de la poudre de carbone et/ou de la poudre de métal en tant que constituant (C).

11. Composition de bioélectrode selon la revendication 10, dans laquelle la poudre de carbone est constituée de noir de carbone ou de nanotube de carbone, ou des deux.

12. Composition de bioélectrode selon la revendication 10, dans laquelle la poudre métallique est choisie dans le groupe constitué par l'or, l'argent, le platine, le cuivre, l'étain, le titane, le nickel, l'aluminium, le tungstène, le molybdène, le ruthénium, le chrome et l'indium, de préférence,
dans laquelle la poudre de métal est de la poudre d'argent.

13. Composition de bioélectrode selon la revendication 6, comprenant en outre un solvant organique en tant que constituant (D).

14. Bioélectrode comprenant un substrat conducteur et une couche en contact avec le corps vivant formée sur le substrat conducteur, la couche en contact avec le corps vivant étant un produit durci de la composition de bioélectrode selon l'une quelconque des revendications 1 à 13, de préférence,
dans laquelle le substrat conducteur comprend une ou plusieurs sortes choisies dans le groupe constitué par l'or, l'argent, le chlorure d'argent, le platine, l'aluminium, le magnésium, l'étain, le tungstène, le fer, le cuivre, le nickel, l'acier inoxydable, le chrome, le titane, le carbone et les polymères conducteurs.

15. Procédé de fabrication d'une bioélectrode comportant un substrat conducteur et une couche en contact avec le corps vivant formée sur le substrat conducteur, comprenant:
l'application de la composition de bioélectrode selon l'une des revendications 1 à 13 sur le substrat conducteur; et
le durcissement de la composition de bioélectrode pour former la couche en contact avec le corps vivant, de préférence,
dans lequel le substrat conducteur comprend une ou plusieurs sortes choisies dans le groupe constitué par l'or, l'argent, le chlorure d'argent, le platine, l'aluminium, le magnésium, l'étain, le tungstène, le fer, le cuivre, le nickel, l'acier inoxydable, le chrome, le titane, le carbone et les polymères conducteurs.
